# EUROPEAN PATENT SPECIFICATION

(11) **EP 2 310 532 B1**
(45) Date of publication and mention of the grant of the patent: **07.05.2014**
(21) Application number: 09774341.3
(22) Date of filing: 30.06.2009
(51) Int. Cl.: C12Q 1/68, G01N 33/68

(54) **METHODS OF PROMOTING WEIGHT LOSS AND ASSOCIATED ARRAYS**
VERFAHREN ZUR FÖRDERUNG VON GEWICHTSVERLUST UND DAMIT ASSOZIIERTE ARRAYS
PROCÉDÉ POUR FAVORISER LA PERTE DE POIDS, ET JEUX ORDONNÉS D'ÉCHANTILLONS ASSOCIÉS

(30) Priority: 30.06.2008 US 76887 P; 29.09.2008 US 101011 P
(43) Date of publication of application: 20.04.2011
(73) Proprietor: The Washington University, St. Louis, MO 63130 (US)
(72) Inventor: GORDON, Jeffrey I., St. Louis, MO 63130 (US); TURNBAUGH, Peter, St. Louis, MO 63130 (US)
(74) Representative: Turner, Rhiannon Rosalind
(86) International application number: PCT/US2009/049253
(87) International publication number: WO 2010/002890

(56) References cited:
- TURNBAUGH PETER J ET AL: "An obesity-associated gut microbiome with increased capacity for energy harvest", NATURE: INTERNATIONAL WEEKLY JOURNAL OF SCIENCE, NATURE PUBLISHING GROUP, UNITED KINGDOM, vol. 444, no. 7122, 1 December 2006 (2006-12-01), pages 1027-1031, XP002492885, ISSN: 0028-0836, DOI: 10.1038/NATURE05414
- LEY RUTH E ET AL: "Microbial ecology: human gut microbes associated with obesity", NATURE: INTERNATIONAL WEEKLY JOURNAL OF SCIENCE, NATURE PUBLISHING GROUP, UNITED KINGDOM, vol. 444, no. 7122, 21 December 2006 (2006-12-21), pages 1022-1023, XP002510853, ISSN: 0028-0836, DOI: 10.1038/4441022A
- BJERKETORP ET AL: "Rapid lab-on-a-chip profiling of human gut bacteria", JOURNAL OF MICROBIOLOGICAL METHODS, ELSEVIER, AMSTERDAM, NL, vol. 72, no. 1, 4 November 2007 (2007-11-04), pages 82-90, XP022404762, ISSN: 0167-7012, DOI: 10.1016/J.MIMET.2007.10.011
- TURNBAUGH ET AL.: 'An obesity-associated gut microbiome with increased capacity for energy harvest.' NAURE vol. 7122, 21 December 2006, pages 1027 - 1031, XP002510854
- HUYGHE ET AL.: 'Novel microarray design strategy to study complex bacterial communities.' APPL ENVIRON MICROBIOL vol. 74, no. 6, March 2008, pages 1876 - 1885, XP008141709
- HALL ET AL.: 'Protein Microarray Technology.' MECH AGEING DEV. vol. 128, no. 1, January 2007, pages 161 - 167, XP005827809
- DIBAISE ET AL.: 'Gut microbiota and its possible relationship with obesity.' MAYO CLIN PROC vol. 83, no. 4, April 2008, pages 460 - 469, XP008141737
- ZABAROVSKY ET AL.: 'Restriction site tagged (RST) microarrays: a novel technique to study the species composition of complex microbial systems.' NUCL. ACIDS RES vol. 31, no. 16, 15 August 2003, page E95, XP008141714

## Description

### FIELD OF THE INVENTION

The present invention encompasses methods and arrays associated with body fat and/or weight loss.

### BACKGROUND OF THE INVENTION

According to the Centers for Disease Control (CDC), over sixty percent of the United States population is overweight, and greater than thirty percent are obese. This translates into more than 50 million adults in the United States with a Body Mass Index (BMI) of 30 or above. Obesity is also a worldwide health problem with an estimated 500 million overweight adult humans [body mass index (BMI) of 25.0-29.9 kg/m²] and 250 million obese adults (Bouchard, C (2000) N Engl J Med. 343, 1888-9). This epidemic of obesity is leading to worldwide increases in the prevalence of obesity-related disorders, such as diabetes, hypertension, cardiac pathology, and nonalcoholic fatty liver disease (NAFLD; Wanless, and Lentz (1990) Hepatology 12, 1106-1110. Silverman, et al, (1990). Am. J. Gastroenterol. 85, 1349-1355; Neuschwander-Tetri and, Caldwell (2003) Hepatology 37, 1202-1219). According to the National Institute of Diabetes, Digestive and Kidney Diseases (NIDDK) approximately 280,000 deaths annually are directly related to obesity. The NIDDK further estimated that the direct cost of healthcare in the U.S. associated with obesity is $51 billion. In addition, Americans spend $33 billion per year on weight loss products. In spite of this economic cost and consumer commitment, the prevalence of obesity continues to rise at alarming rates. From 1991 to 2000, obesity in the U.S. grew by 61%.

Although the physiologic mechanisms that support development of obesity are complex, the medical consensus is that the root cause relates to an excess intake of calories compared to caloric expenditure. While the treatment seems quite intuitive, dieting is not an adequate long-term solution for most people; about 90 to 95 percent of persons who lose weight subsequently regain it. Although surgical intervention has had some measured success, the various types of surgeries have relatively high rates of morbidity and mortality.

Pharmacotherapeutic principles are limited. In addition, because of undesirable side effects, the FDA has had to recall several obesity drugs from the market. Those that are approved also have side effects. Currently, two FDA-approved anti-obesity drugs are orlistat, a lipase inhibitor, and sibutramine, a serotonin reuptake inhibitor. Orlistat acts by blocking the absorption of fat into the body. An unpleasant side effect with orlistat, however, is the passage of undigested oily fat from the body. Sibutramine is an appetite suppressant that acts by altering brain levels of serotonin. In the process, it also causes elevation of blood pressure and an increase in heart rate. Other appetite suppressants, such as amphetamine derivatives, are highly addictive and have the potential for abuse. Moreover, different subjects respond differently and unpredictably to weight-loss medications.

Because surgical and pharmacotherapy treatments are problematic, new non-cognitive strategies are needed to prevent and treat obesity and obesity-related disorders.

Turnbaugh et al. (Nature (2006) vol. 444 p 1027-1031) discloses an obesity-associated gut microbiome. Ley et al. (Nature (2006) vol. 444 p 1022-1023) discloses that the ratio of *Bacteroidetes* and *Firmicutes* is related to the obese or lean status of the host. Bjerketorp et al. (J. Microbiol. Meth. (2007) vol. 72 p 82-90) describes rapid profiling of human gut bacteria.

### SUMMARY OF THE INVENTION

One aspect of the present invention encompasses an array comprising a substrate. The substrate has disposed thereon at least one of the nucleic acid sequences listed in Table 13 or 14, or nucleic acids capable of specifically hybridizing to each of the nucleic acid sequences listed in Table 13 or 14.

### BRIEF DESCRIPTION OF THE FIGURES

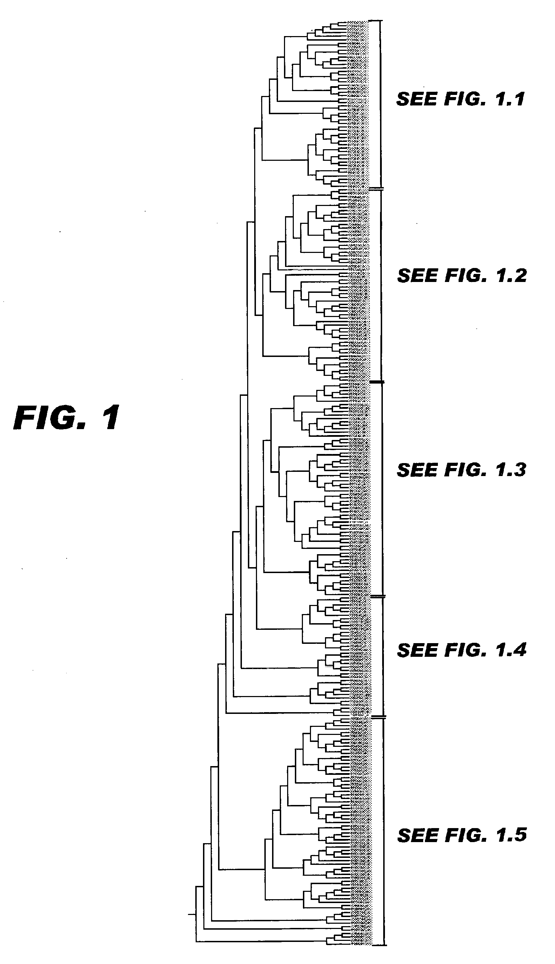

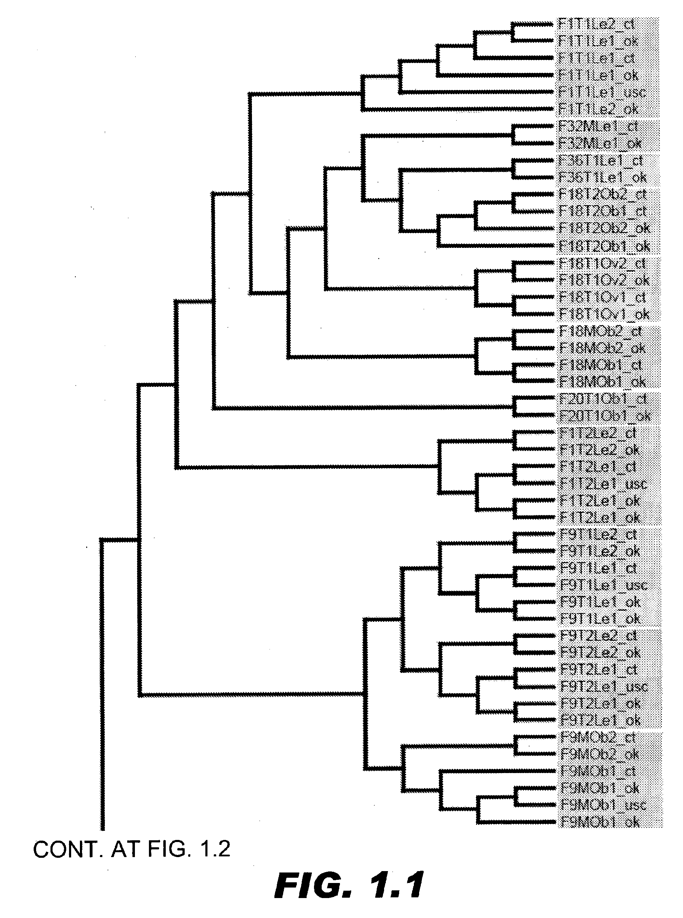

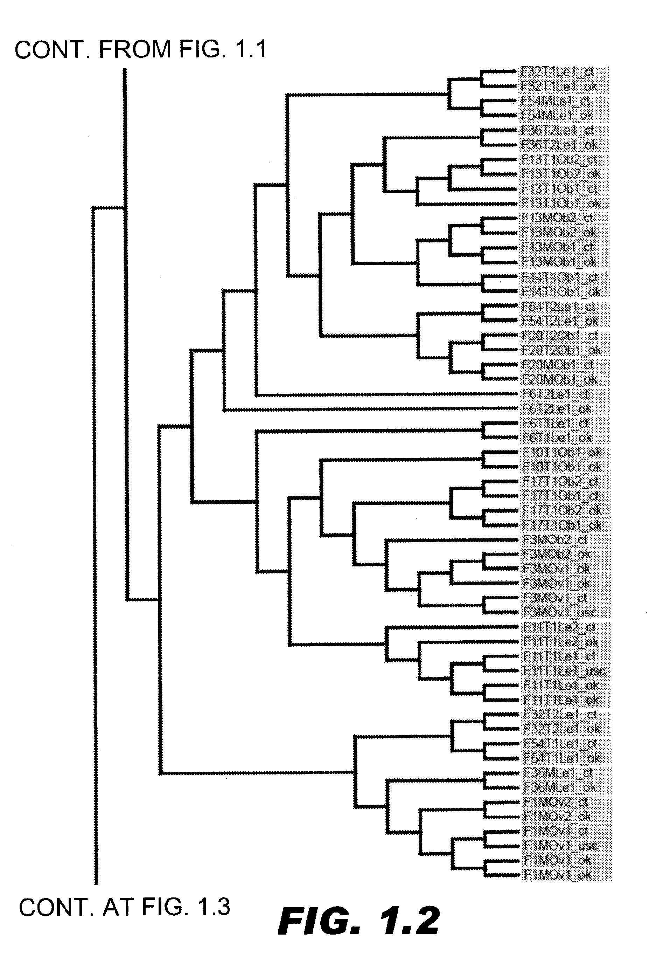

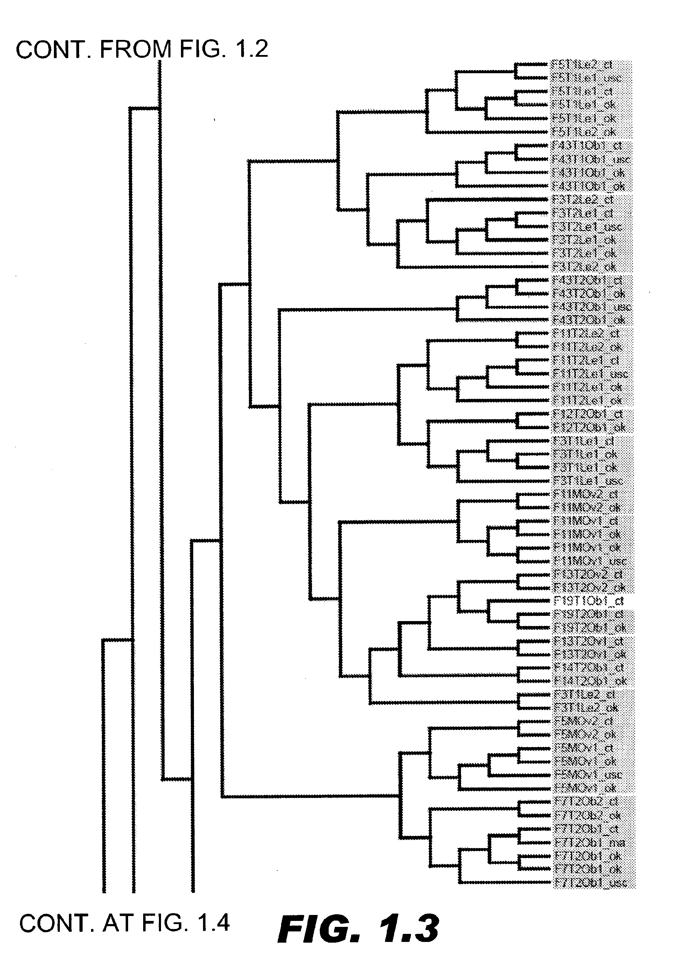

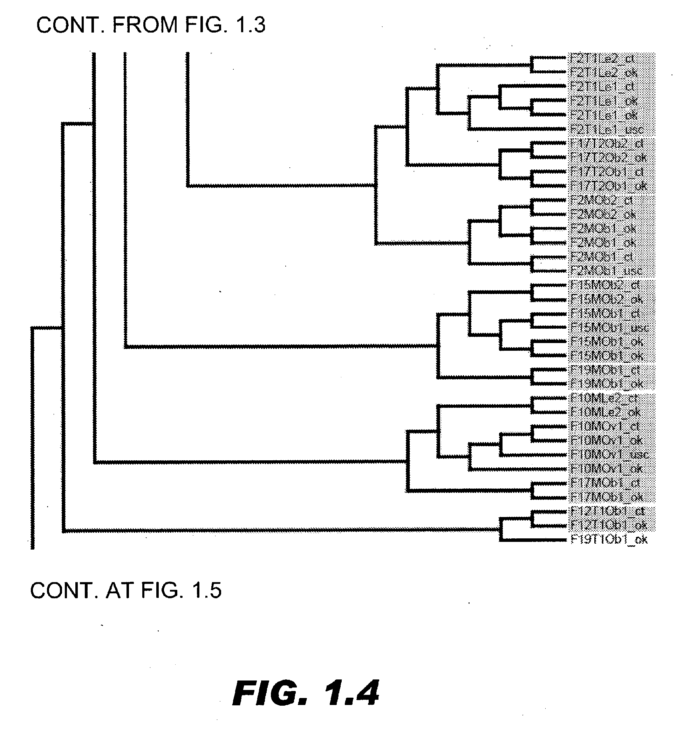

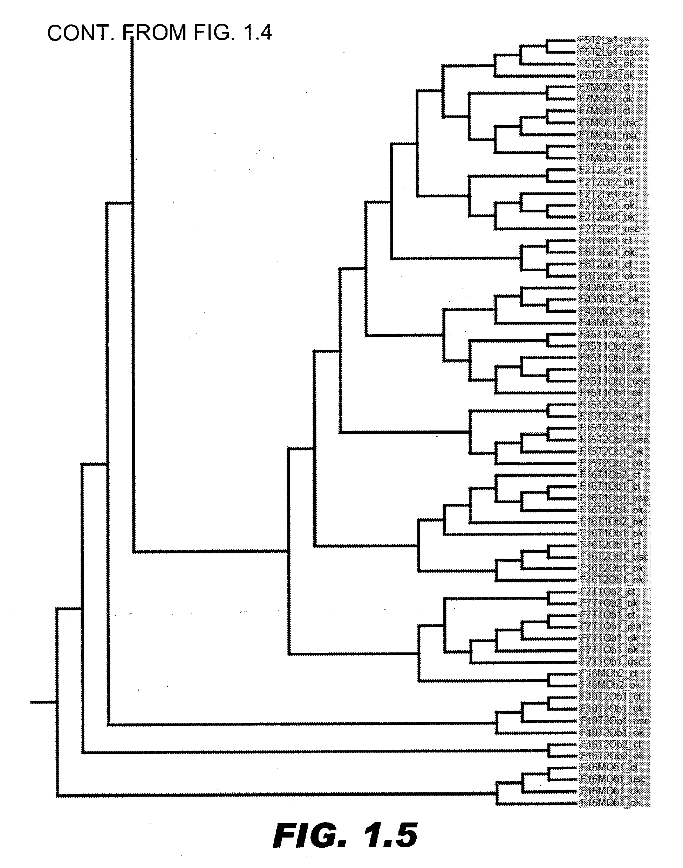

**FIG. 1** depicts the technical replicates (analyzed at four different sequencing centers) cluster. Fecal DNA samples were split and sequenced separately at four different sequencing centers. Abbreviations: usc, Environmental Genomics Core Facility, University of South Carolina; ok, Advanced Center for Genome Technology, University of Oklahoma, ct; 454 Life Sciences Branford, CT; and ma, Josephine Bay Paul Center, Marine Biological Laboratory, Woods Hole Massachusetts. Unweighted UniFrac-based clustering was performed on the combined dataset. Colored boxes enclose samples from the same individual (also indicated by identical IDs followed by the number 1 or 2. The location of the sequencing facility follows each sample ID.) Randomly selected sequences were analyzed (≤500 per replicate). **FIGS. 1.1, 1****.****2****,** **1****.****3****,** **1****.****4**, and **1.5** show details from **FIG. 1****.**

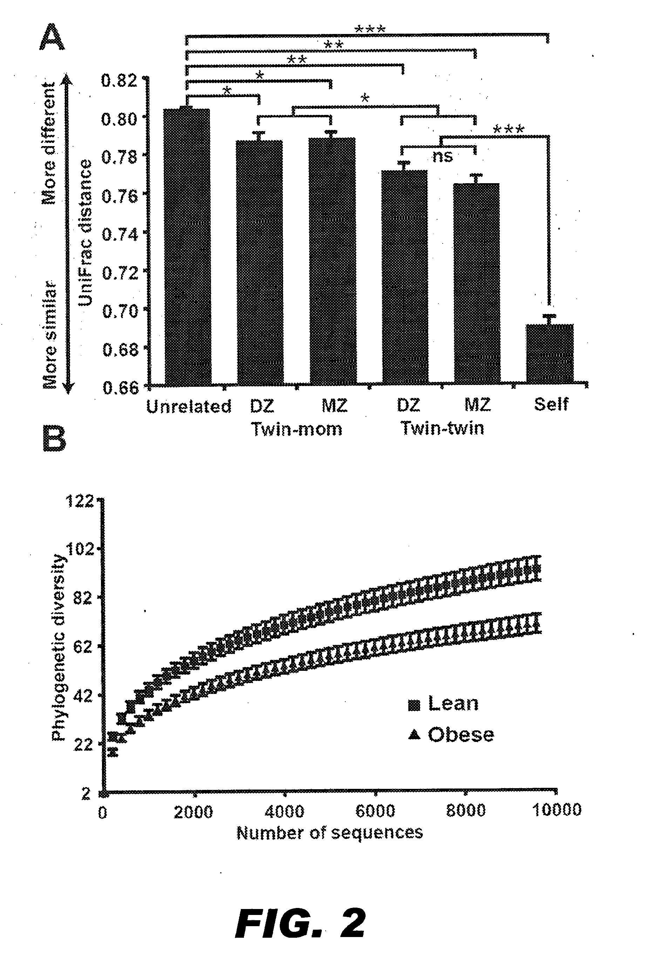

**FIG. 2** depicts 16S rRNA gene surveys revealing familial similarity and reduced diversity of the gut microbiota in obese individuals. (A) Comparison of the average UniFrac distance (a measure of differences in bacterial community structure) between individuals over time (self), twin-pairs, twins and their mother, and unrelated individuals. Briefly, 1,000 sequences were randomly sampled from each V2/3 dataset, OTUs were chosen, a UniFrac tree was built from representative sequences, and random permutations were done on the resulting UniFrac distance matrix. Asterisks indicate significant differences between the indicated categories [Student's t-test with Monte Carlo (1,000 permutations); *p<10-5; **p<10-14; ***p<10-41]. (B) Evidence of reduced diversity in the fecal microbiota of obese individuals. Phylogenetic diversity curves were generated by randomly sampling 1 to 10,000 sequences from each V6 16S rRNA dataset, and then calculating the total branch length leading to the sampled sequences (mean±95%Cl shown).

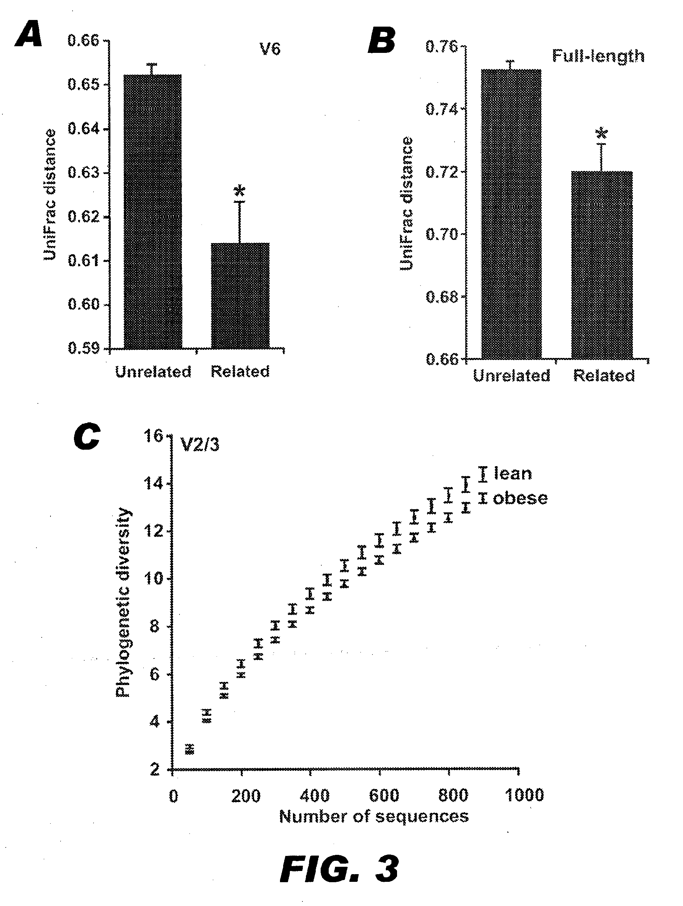

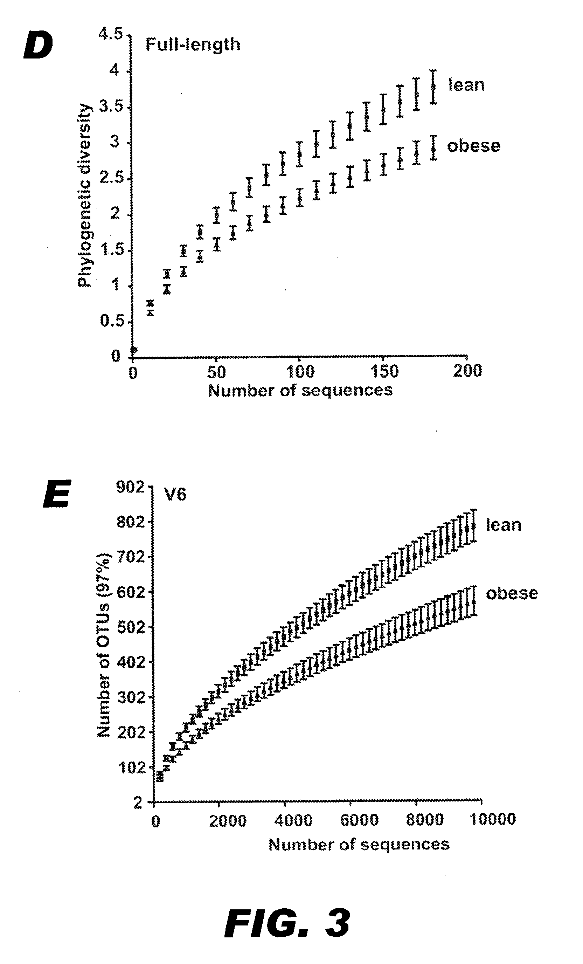

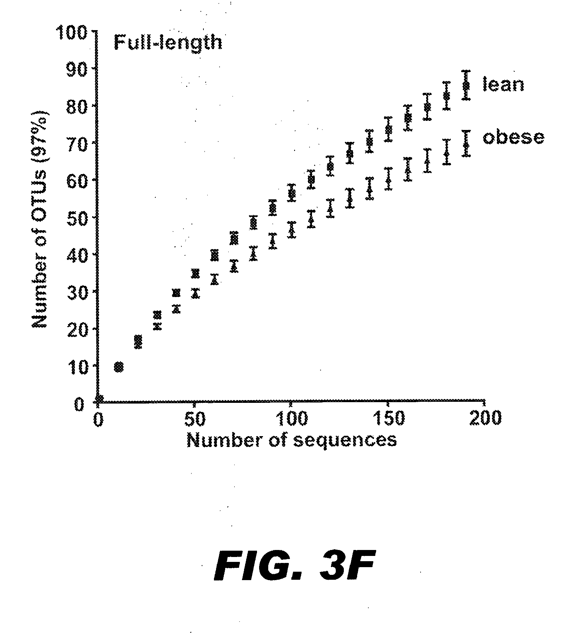

**FIG. 3** depicts 16S rRNA gene surveys revealing evidence for familial aggregation and reduced diversity in the obese gut microbiome. (A,B) Comparison of the average UniFrac distance (a measure of differences in bacterial community structure) between related and unrelated individuals. Briefly, 10,000 sequences were randomly sampled from each V6 dataset (Panel A) and 200 sequences were randomly sampled from each full-length dataset (Panel B), OTUs were chosen, a UniFrac tree was built from representative sequences, and random permutations were done on the resulting UniFrac distance matrix. Asterisks indicate significant differences between related and unrelated individuals [Student's t-test with Monte Carlo (1,000 permutations); *p<0.001]. (C,D) Phylogenetic diversity curves for the obese and lean gut microbiome. Briefly, 1 to 1,000 sequences were randomly sampled from each V2/3 dataset (Panel C), and 1 to 200 sequences were randomly sampled from each fulllength dataset (Panel D), and the average branch length leading to the sampled sequences was calculated. (E,F) Rarefaction curves for the obese and lean fecal microbiota. Briefly, 1 to 10,000 sequences were randomly sampled from each V6 dataset (Panel E), and 1 to 200 sequences were randomly sampled from each fulllength dataset (Panel F). The average number of OTUs in each sample was then calculated (mean±95%Cl shown).

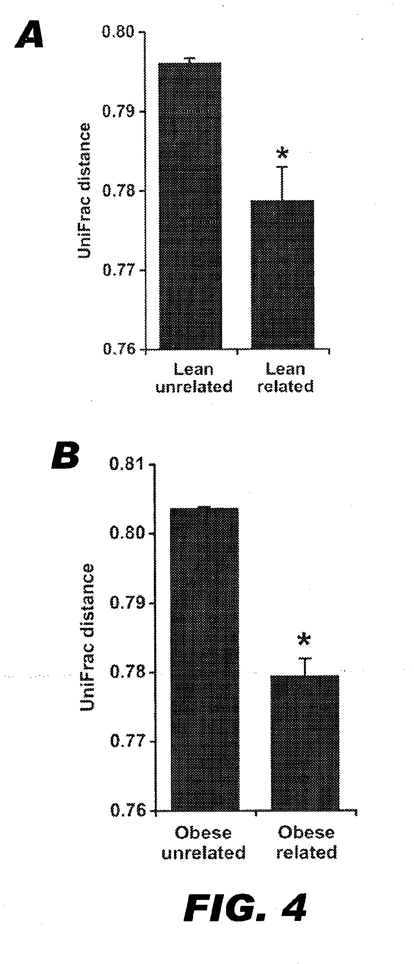

**FIG. 4** depicts a graph illustrating the stratification of related and unrelated individuals concordant for physiological states of obesity versus leanness confirms familial similarity. (A,B) Comparison of the average UniFrac distance (a measure of differences in bacterial community structure) between related and unrelated individuals concordant for leanness (Panel A) or obesity (Panel B). Briefly, 1,000 sequences were randomly sampled from each V2/3 dataset, OTUs were chosen, a UniFrac tree was built from representative sequences, and random permutations were done on the resulting UniFrac distance matrix. Asterisks indicate significant differences between related and unrelated individuals [Student's t-test with Monte Carlo (1,000 permutations); *p<10⁻⁵].

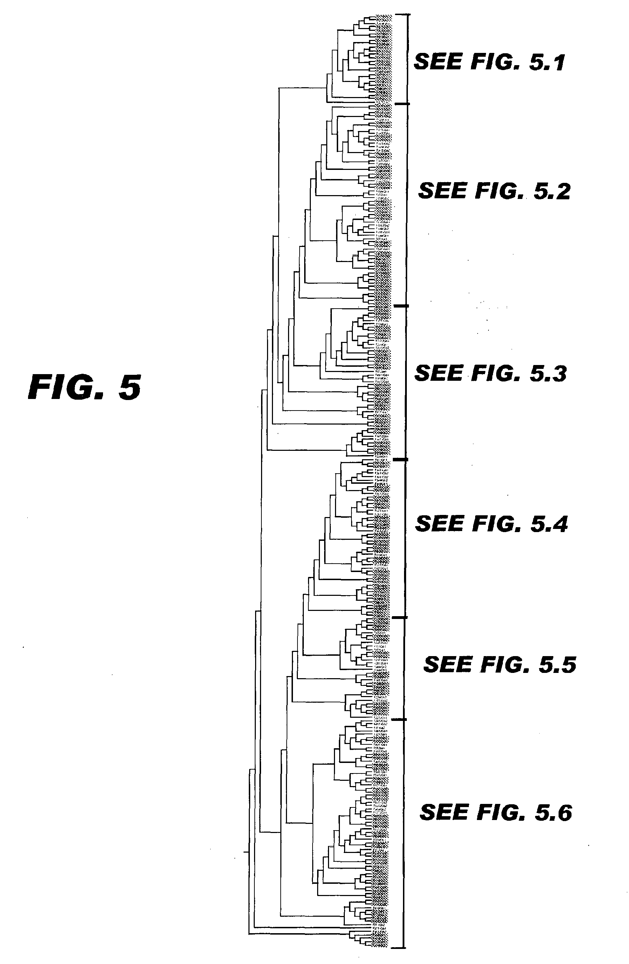

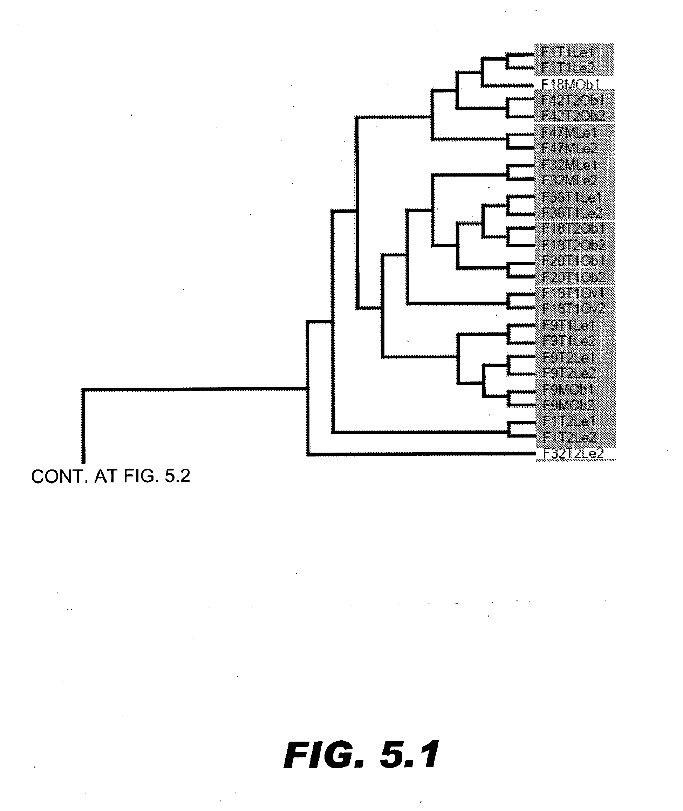

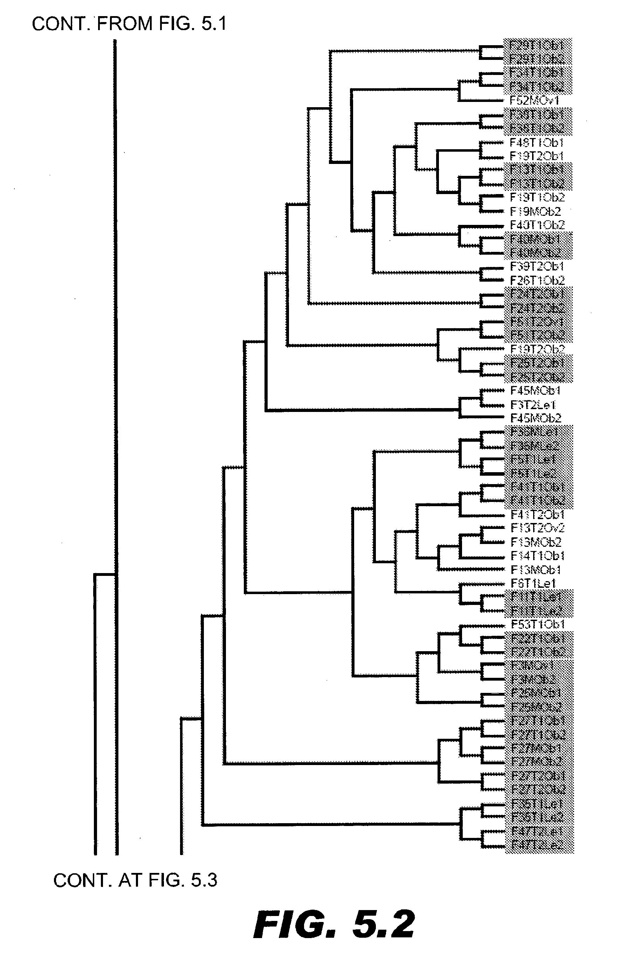

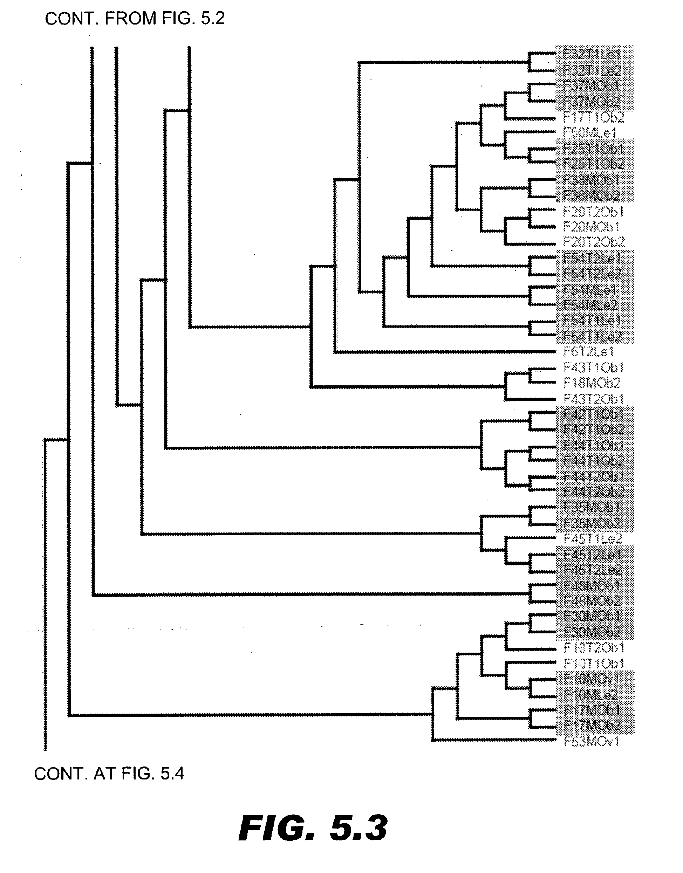

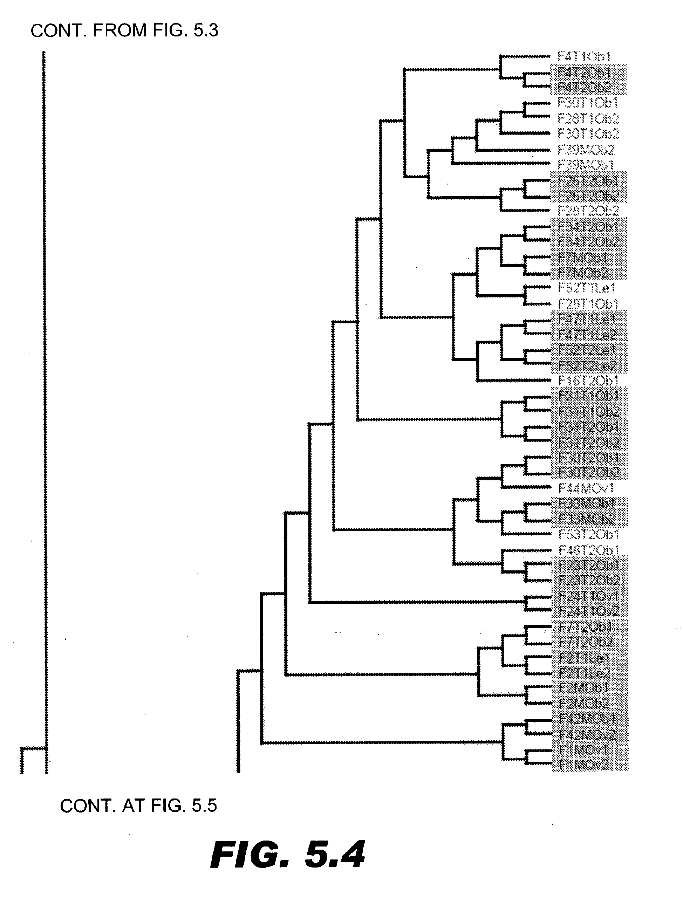

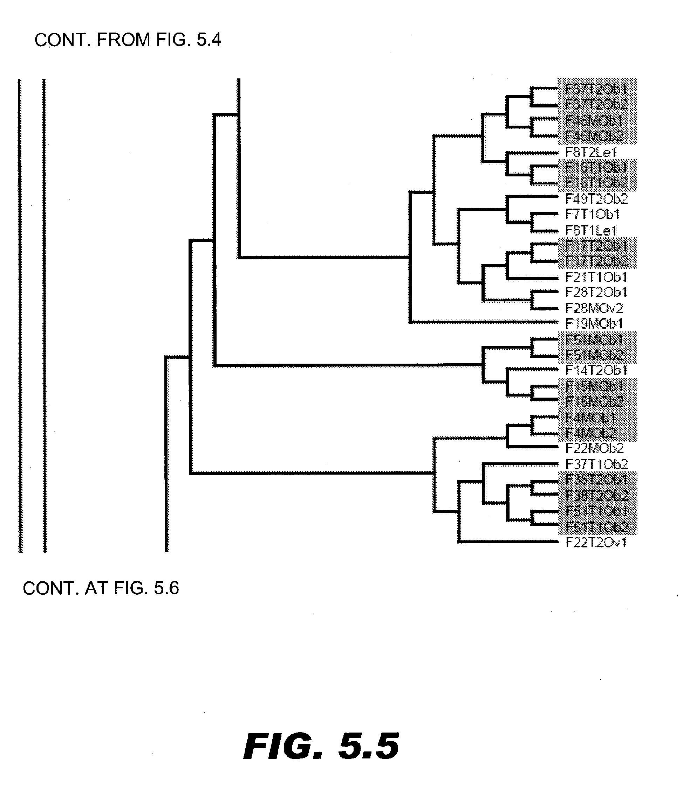

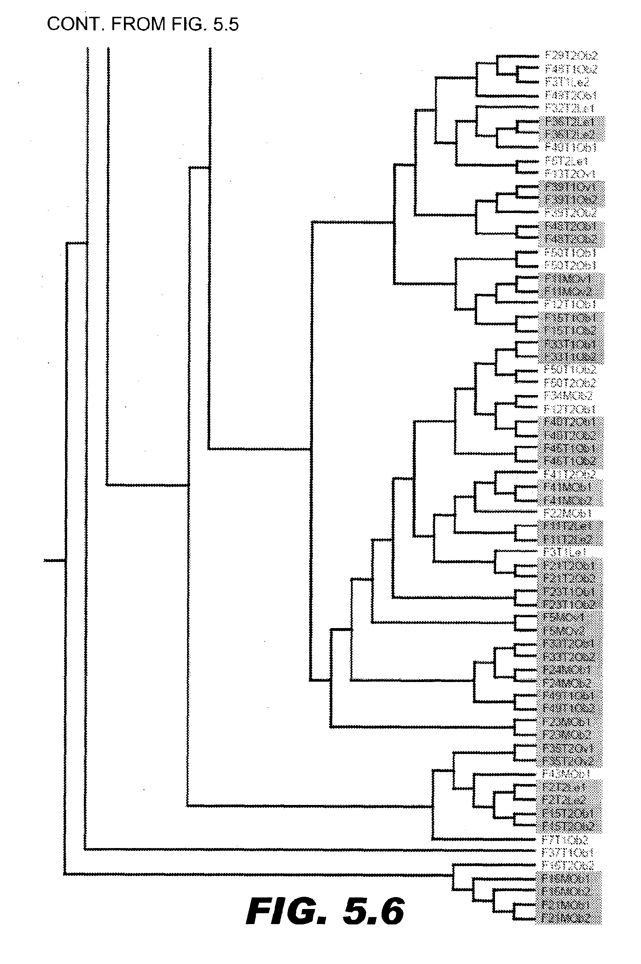

**FIG. 5** depicts clustering of the fecal microbiotas of monozygotic (MZ) and dizygotic (DZ) twins and their mothers sampled at the beginning of the study and two months later. Unweighted UniFrac-based clustering. Colored boxes link samples from the same individual (also indicated by identical IDs followed by the number 1 or 2). 34 of the individuals were only sampled once. 1,000 randomly V2/3 16S rRNA gene sequences were analyzed per sample. **FIGS. 5****.****1****,** **5****.****2****,** **5****.****3****,** **5****.****4****,** **5.5****,** and **5.6** show details from **FIG. 5****.**

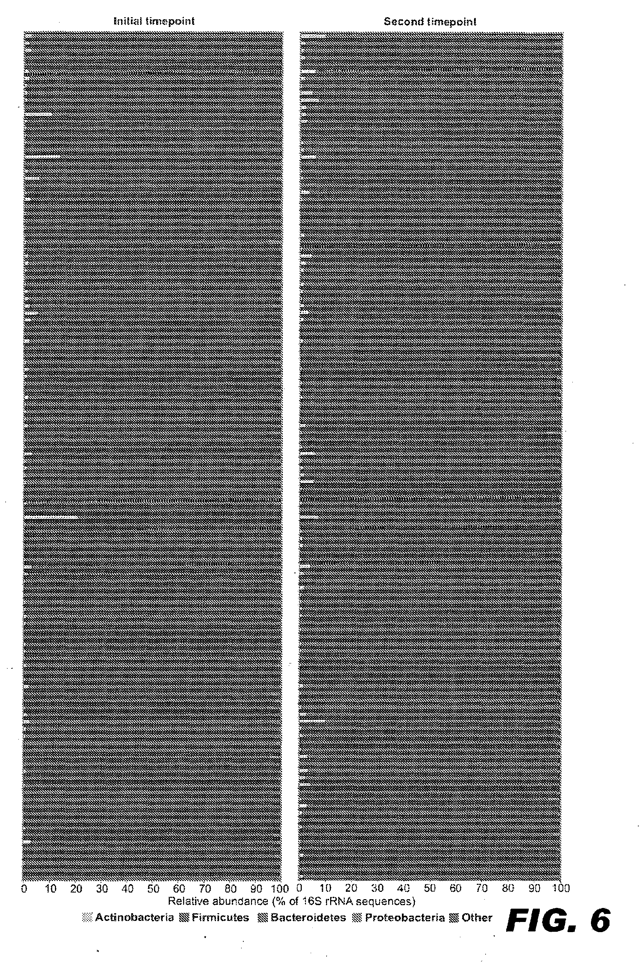

**FIG. 6** depicts the relative abundance of the major gut bacterial phyla across 120 gut samples obtained at two different timepoints. Fecal samples were collected at the initial and second timepoints (average interval between sample collection: 57±4 days). The relative abundance of the major gut bacterial phyla is based on analysis of V2/3 16S rRNA gene sequences. Samples are organized based on the rank order abundance of Firmicutes in the initial timepoint.

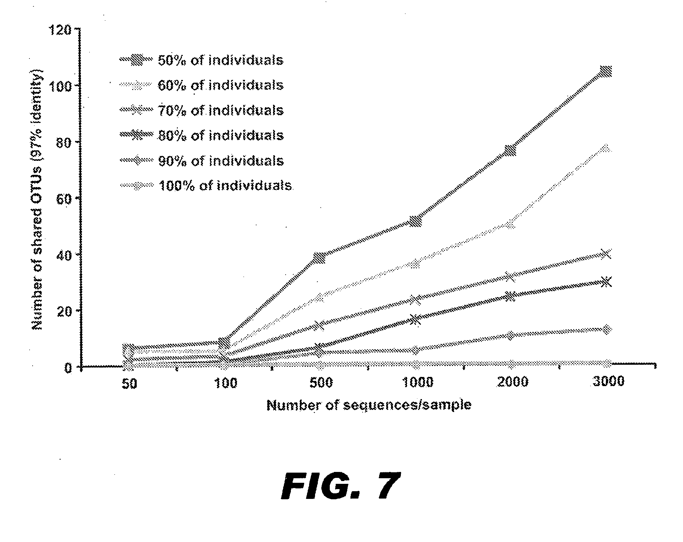

**FIG. 7** depicts the number of shared phylotypes (OTUs) as a function of the number of sequences per sample. 50-3,000 sequences were randomly selected from each sample, obtained from 93 different individuals. All sequences were binned into 'species'-level phylotypes using a 97% identity threshold. Less stringent parameters were used for OTU binning at all levels of coverage to allow for analysis of 3,000 sequences per sample (density cutoff=0.65, maximum of 3000 nodes).

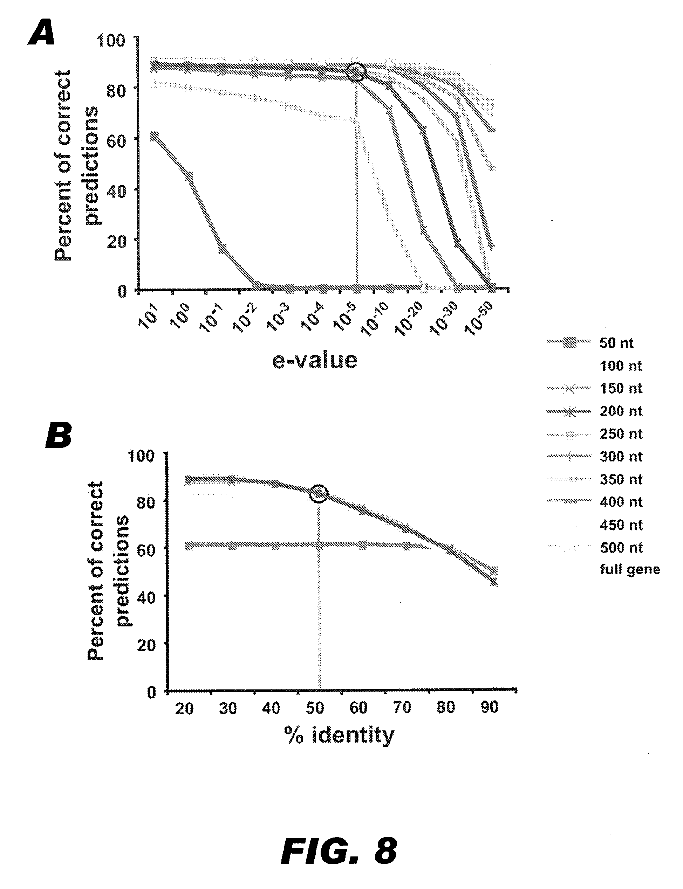

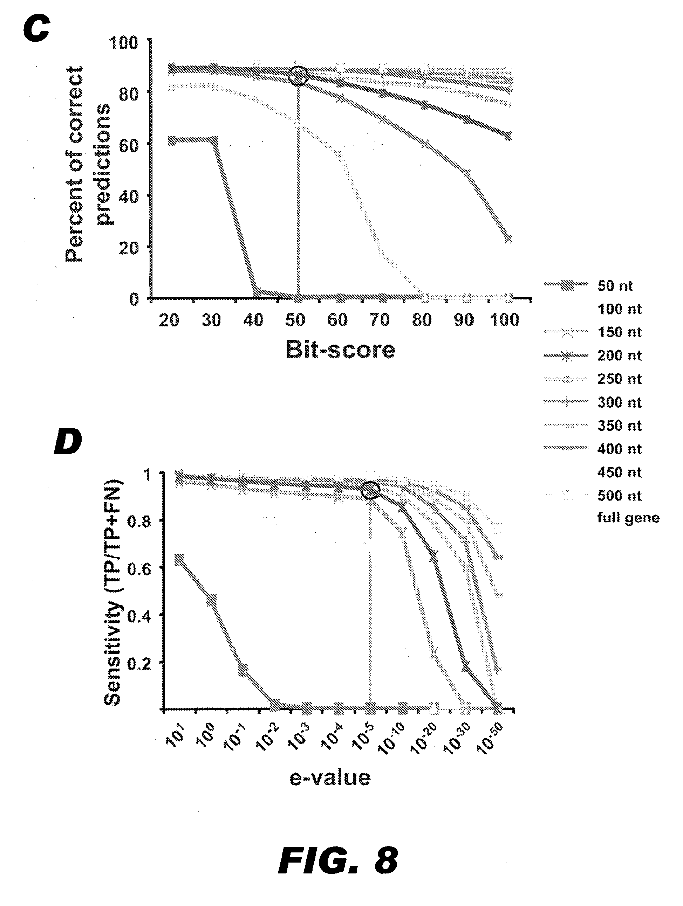

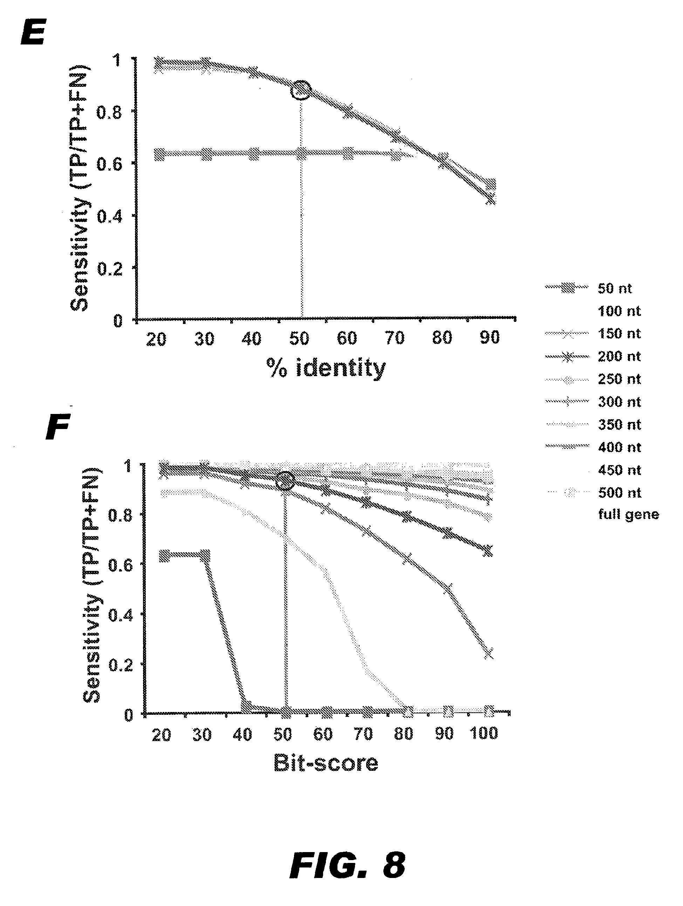

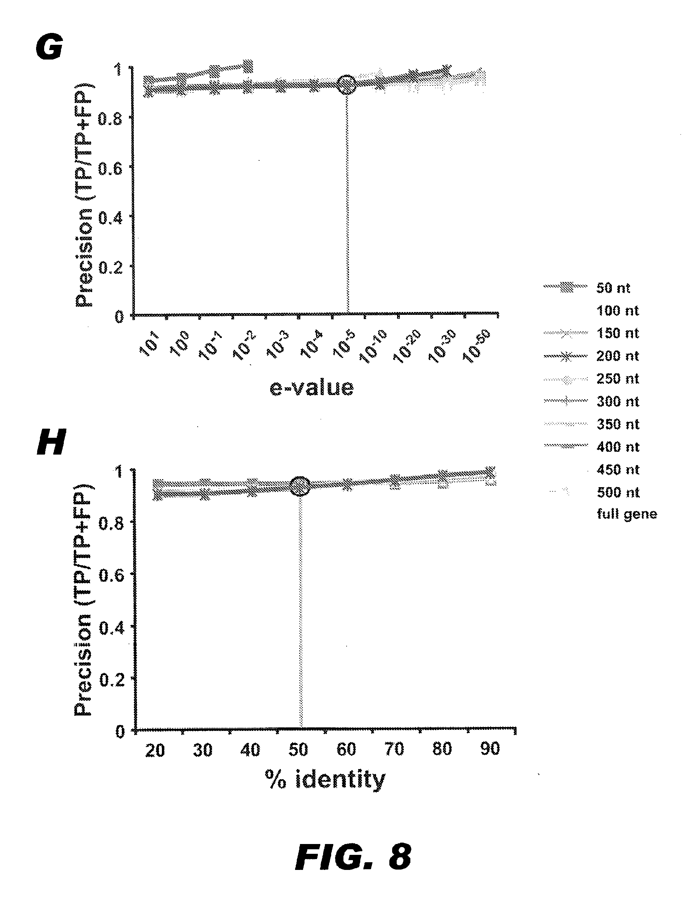

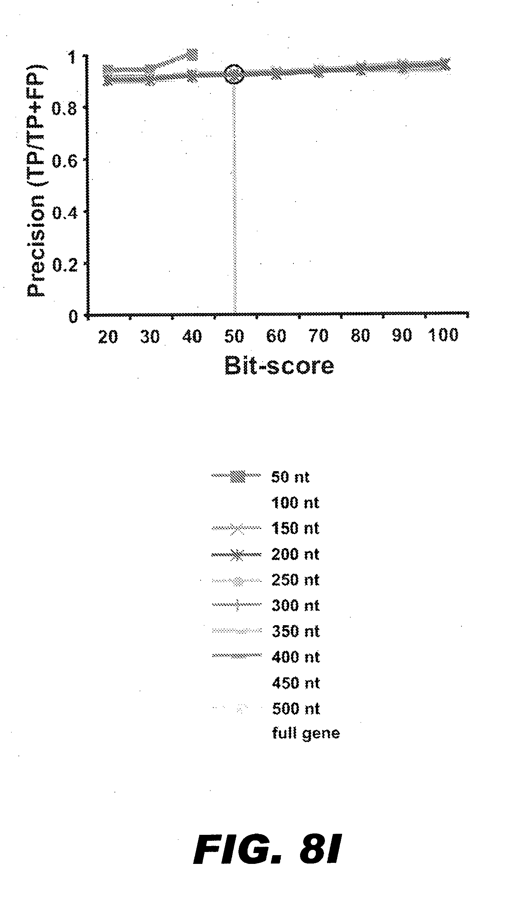

**FIG. 8** depicts the validation of annotation parameters using control datasets. (A-C) Percent of randomly fragmented annotated genes (KEGG v44) assigned to the correct KEGG orthologous group as a function of the (A) e-value, (B) % identity, or (C) bit-score cutoff used. (D-F) Sensitivity [true positives (TP) divided by true positives plus false negatives (FN)] as a function of the (D) e-value, (E) % identity, or (F) bit-score cutoff used. (G-I) Precision [true positives divided by true positives plus false positives (FP)] as a function of the (G) e-value, (H) % identity, or (I) bit-score cutoff used. The vertical gray line and circle indicates the cutoff values used in this analysis.

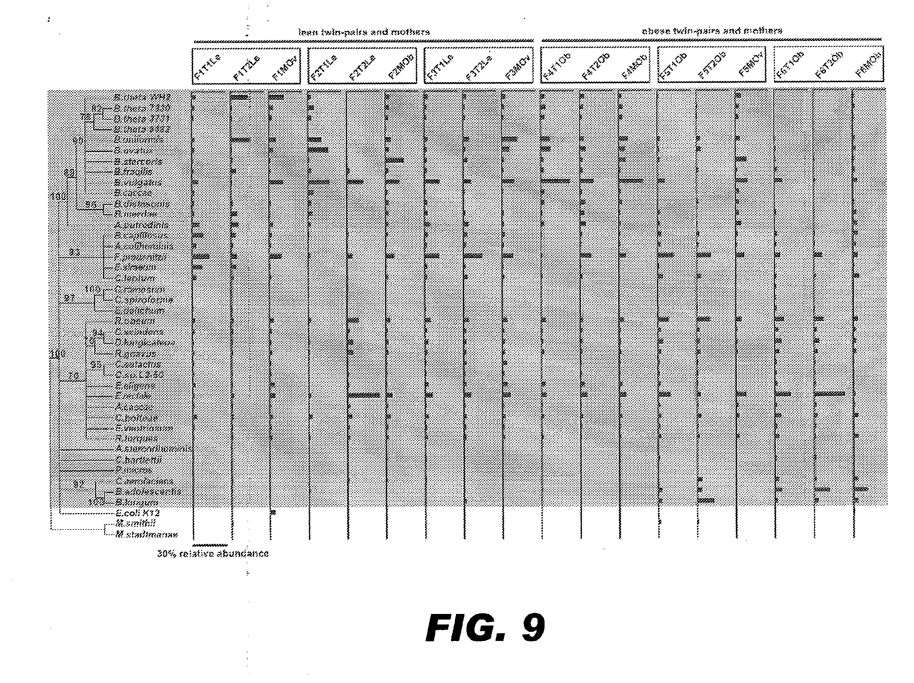

**FIG. 9** depicts the taxonomic profiles of microbial gene content in the human gut (fecal) microbiome. Full-length 16S sequences were obtained for each reference genome, likelihood parameters were determined using Modeltest, and a maximum-likelihood tree was generated using PAUP. Bootstrap values represent nodes found in >70 of 100 repetitions. Branches and distributions are colored by phylum: Bacteroidetes (orange), Firmicutes (blue), and Actinobacteria (green). Proteobacteria (E. coli) and Archaea (M. smithii and M. stadtmanae) are uncolored. The relative abundance of sequences homologous to each genome is depicted on a scale of 0 to 30% (BLASTX comparisons of microbiome datasets to reference genomes). Sample ID nomenclature: Family number, Twin number or mom, and BMI category (Le=lean, Ov=overweight, Ob=obese; e.g. F1T1Le stands for family 1, twin 1, lean).

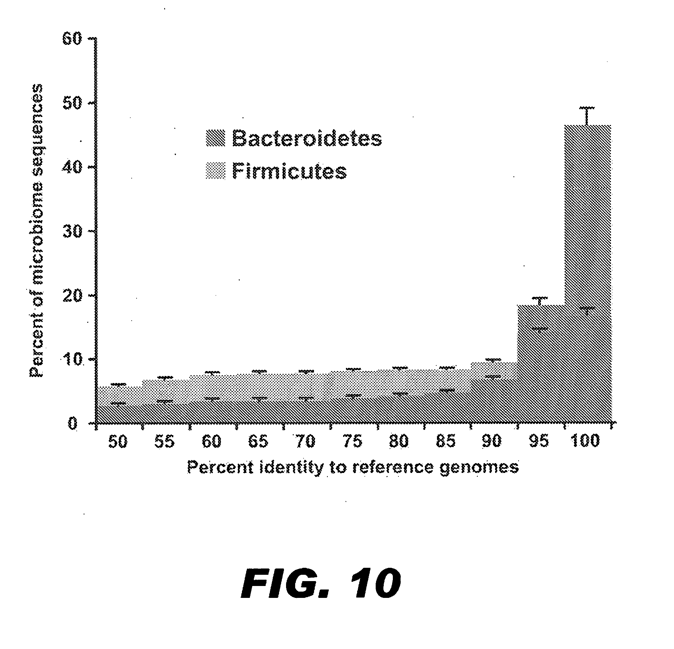

**FIG. 10** depicts the assignment of fecal microbiome reads to sequenced reference human gut-derived Bacteroidetes and Firmicutes genomes. Histogram of the percent identity (mean±SEM) obtained from sequence alignments between gut microbiome reads (n=18 datasets) and Firmicutes or Bacteroidetes reference genomes.

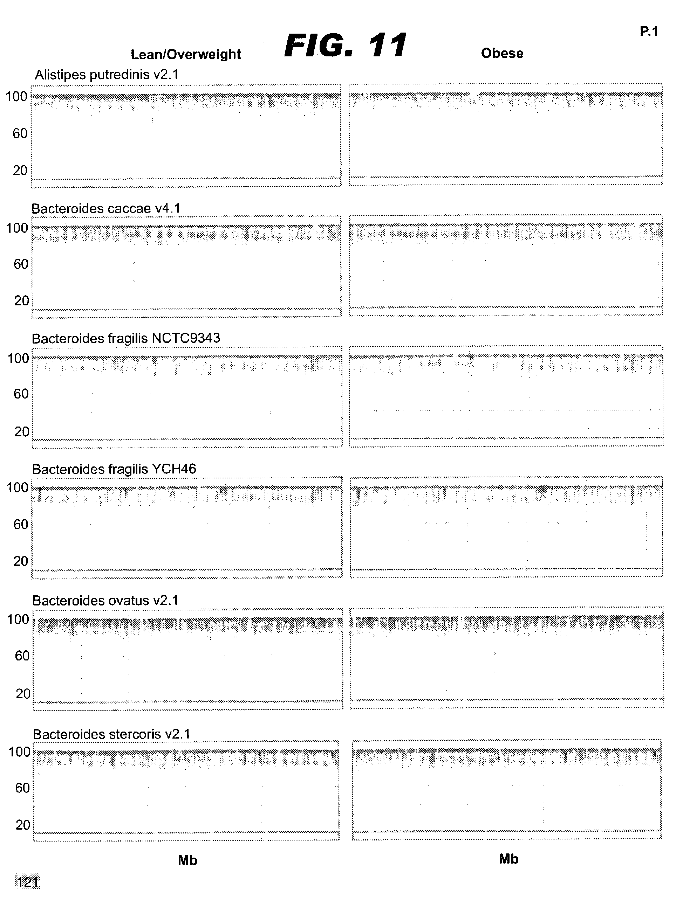

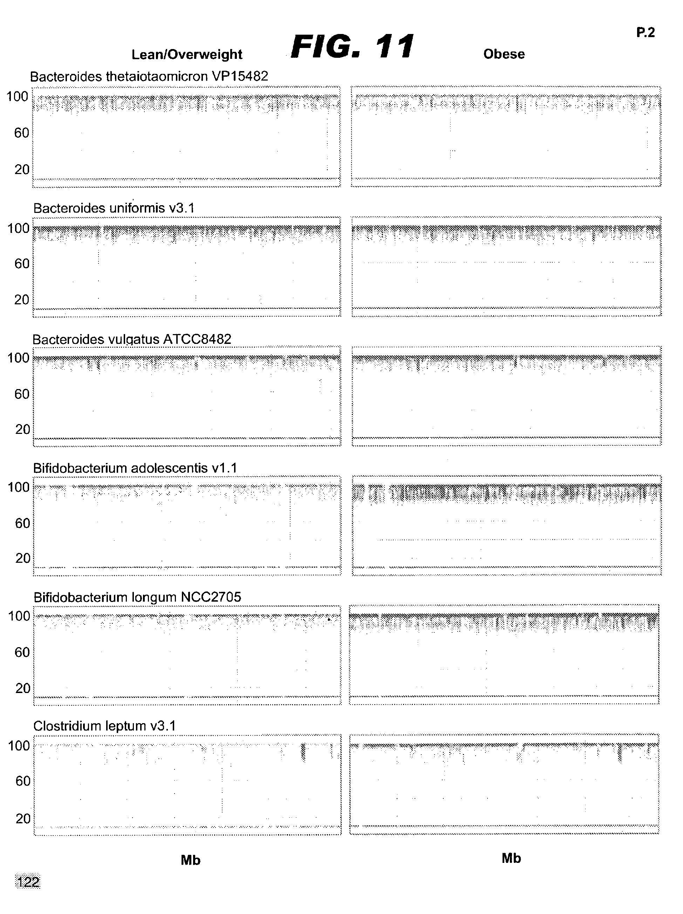

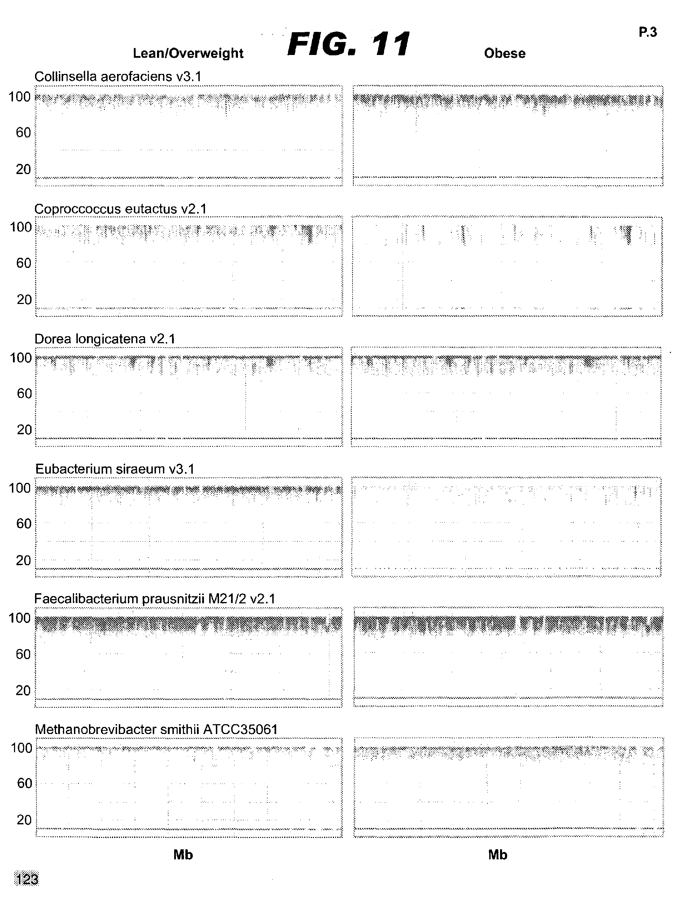

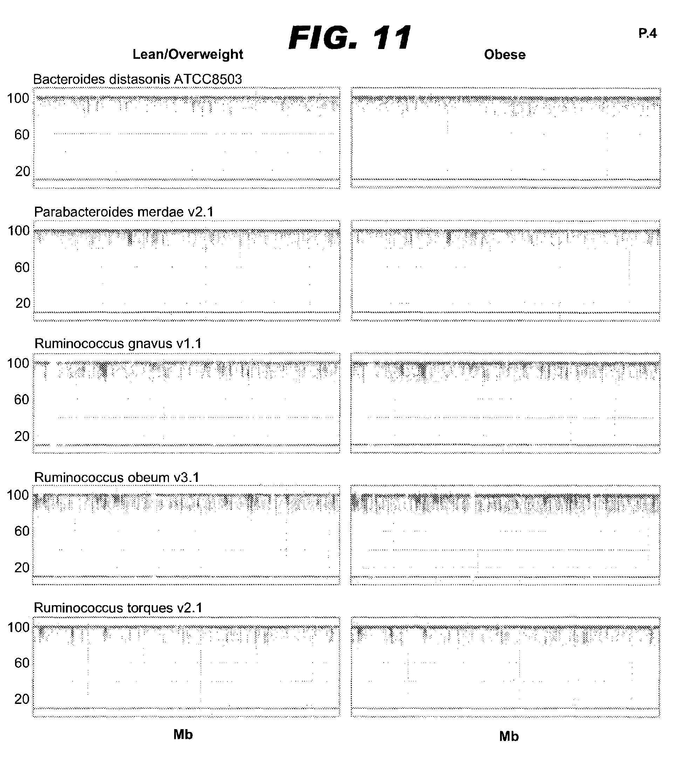

**FIG. 11** depicts the percent identity plots of the fecal microbiomes versus reference genomes. Each row (x-axis) represents a different genome. The y-axis shows the percent identity to microbiome sequences (red dots). The combined data from lean/overweight individuals are in the left column while the combined data from obese individuals are displayed in the right column. Supercontigs were used for draft genomes; the assembly version (v) can be found after the strain name. The lines found at 10% identity on each plot depict the sum of all sequences mapped across each genome.

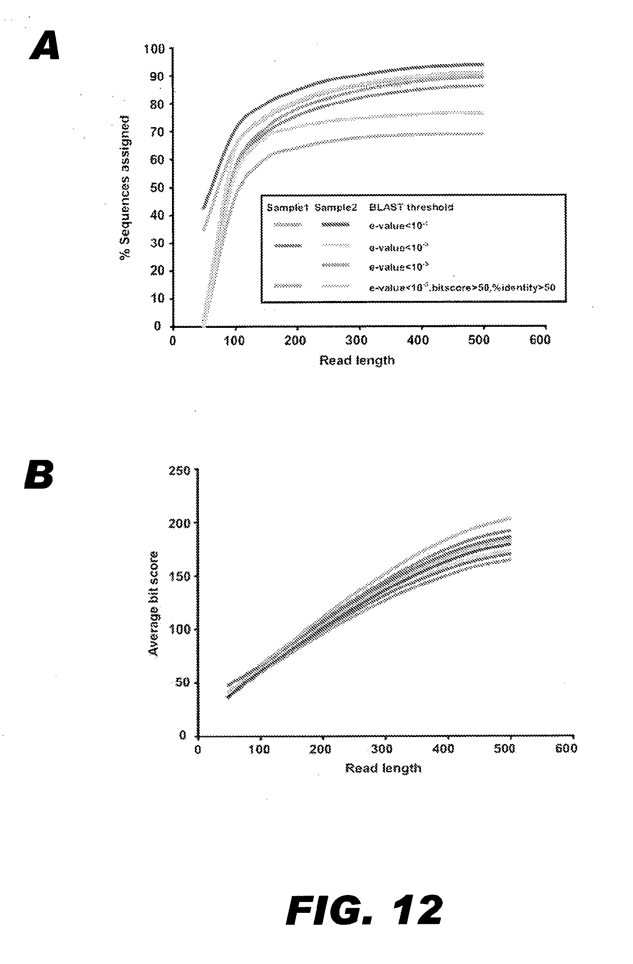

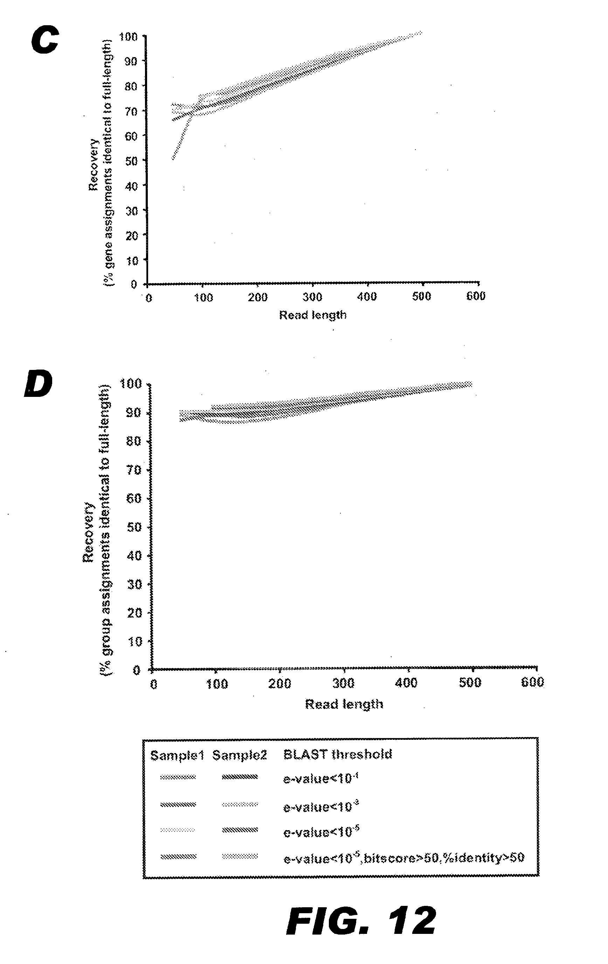

**FIG. 12** depicts the dependence of percentage (A), quality (B), and accuracy (C-D) of sequence assignments on read-length. Two fecal samples were processed using extra-long read pyrosequencing (454 FLX Titanium kit; samples TS28 and TS29). 10,000 sequences from the maximum of each read-length distribution (between 490 and 505 nt) were randomly selected from each sample. Simulated reads were created by sampling the first 50-500 nt of each of these 10,000 sequences, and each simulated read was compared using NCBI-BLASTX against our custom gut genome database. Multiple BLAST thresholds were used (see key in panel A). (A) Percent of sequences assigned to the reference genomes as a function of read-length. (B) Average BLAST bit score as a function of read-length. (C) Percent of gene assignments (from the gut genome database) identical to full-length sequence as a function of read-length. (D) Percent of group assignments (same assigned COG as the full-length sequence) as a function of read-length.

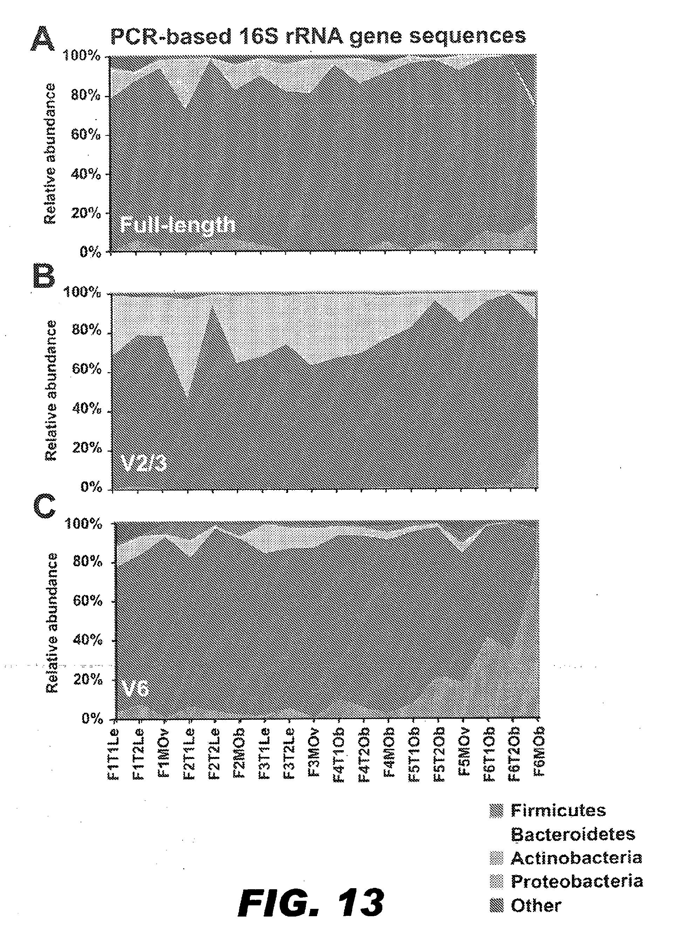

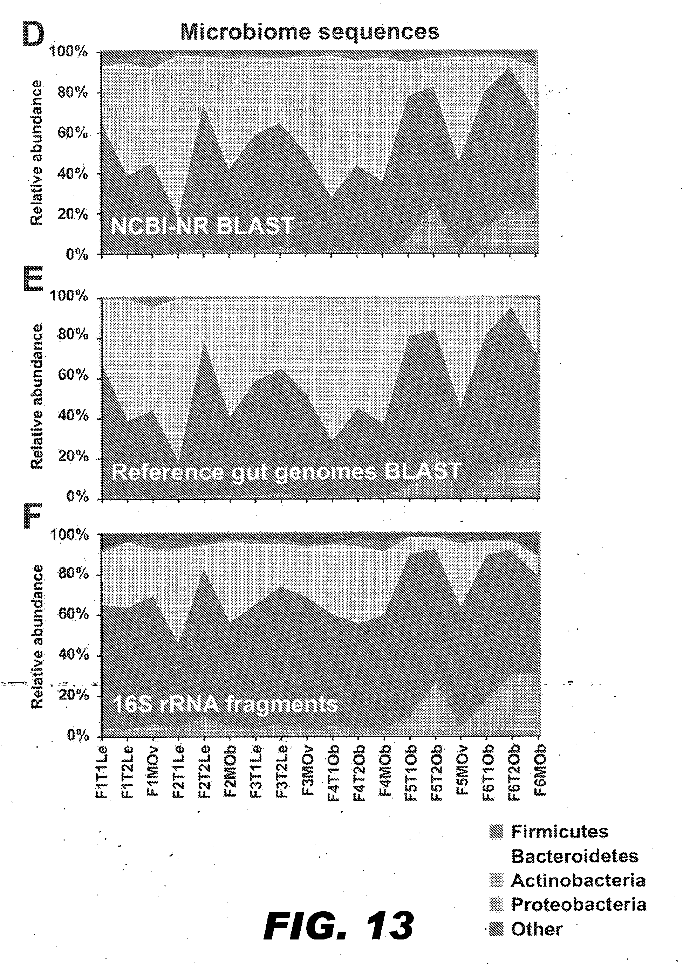

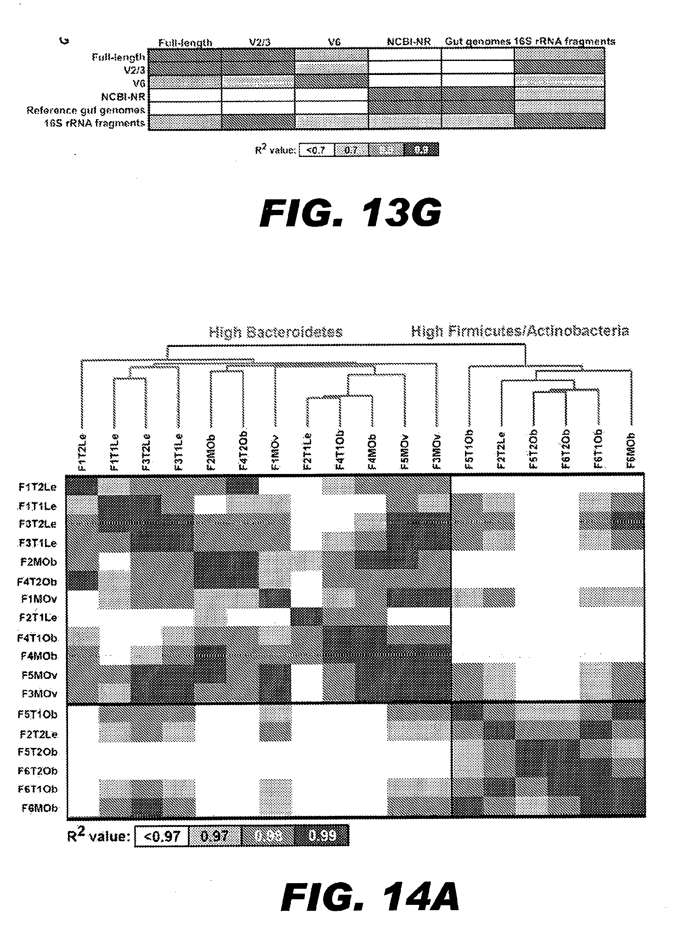

**FIG. 13** depicts the relative abundance of bacterial phyla in 18 human gut microbiomes. (A-C) PCR-based 16S rRNA gene sequences [(A) full-length, (B) V2/3 region, and (C) V6]. (D-E) Microbiome data analyzed by BLAST comparisons [(D) NCBI non-redundant database and (E) a custom 42 gut genome database]. (F) Analysis of 16S rRNA gene fragments identified in each microbiome. (G) Correlation matrix based on all pairwise comparisons (R²) of the relative abundance of the four major phyla (Actinobacteria, Firmicutes, Bacteroidetes, and Proteobacteria) across all six methods.

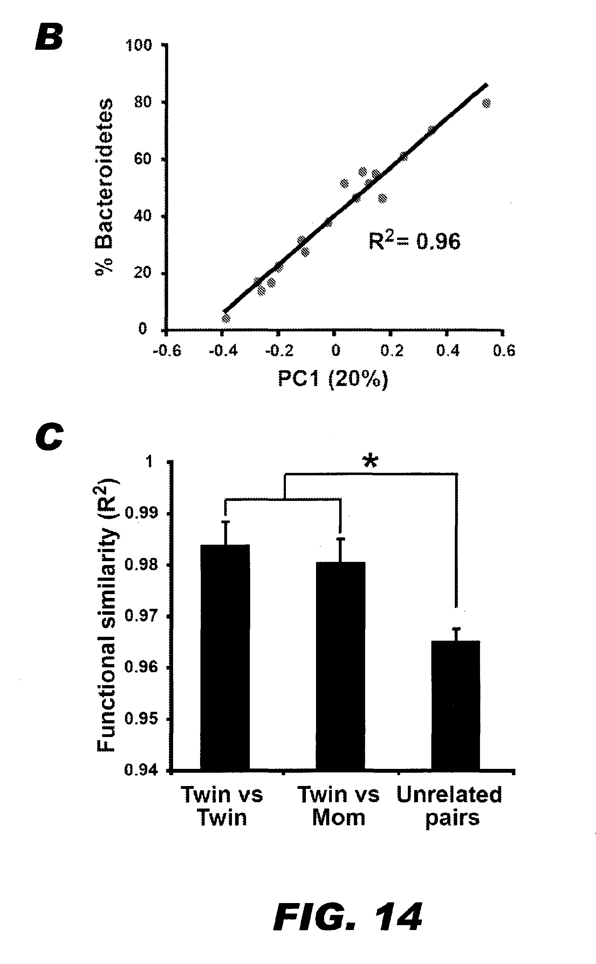

**FIG. 14** depicts the metabolic pathway-based clustering and analysis of the human gut microbiome of MZ twins. (A) Metabolic pathways were tallied using the KEGG database and annotation scheme. Functional profiles were clustered using a single-linkage hierarchical clustering with a Pearson's distance metric. All pairwise comparisons were made of the profiles by calculating each R² value. (B) A linear regression of the relative abundance of Bacteroidetes versus the first principal component derived from a PCA analysis of KEGG metabolic profiles. (C) Comparisons of functional similarity between twin pairs, between twins and their mother, and between unrelated individuals. Asterisks indicate significant differences (Student's t-test with Monte Carlo; p<0.01) and bars represent mean±SEM.

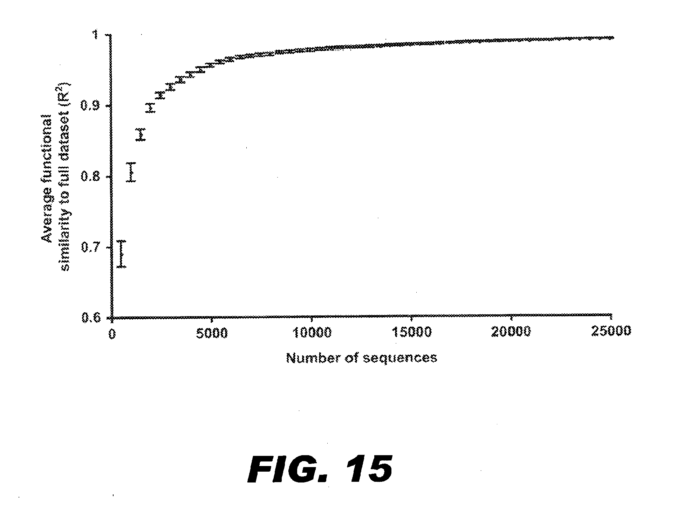

**FIG. 15** depicts the functional profiles of MZ fecal microbiomes, based on the relative abundance of KEGG pathways, which stabilize after ~20,000 sequences are collected for a given sample. Datasets were randomly subsampled between 500 and 25,000 sequences. The average functional similarity (R²) between the subsampled dataset and the full dataset is shown as a function of sequencing effort.

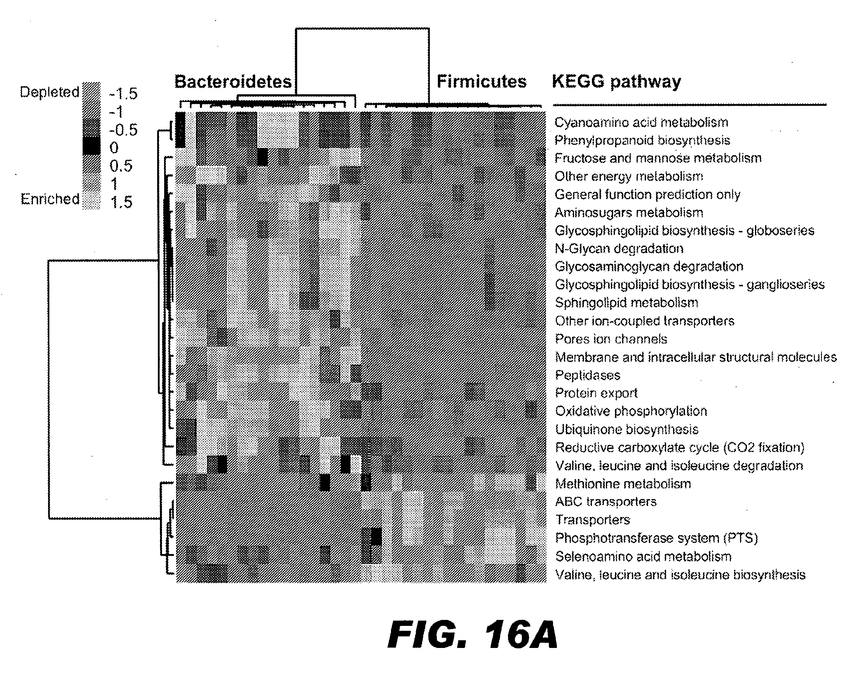

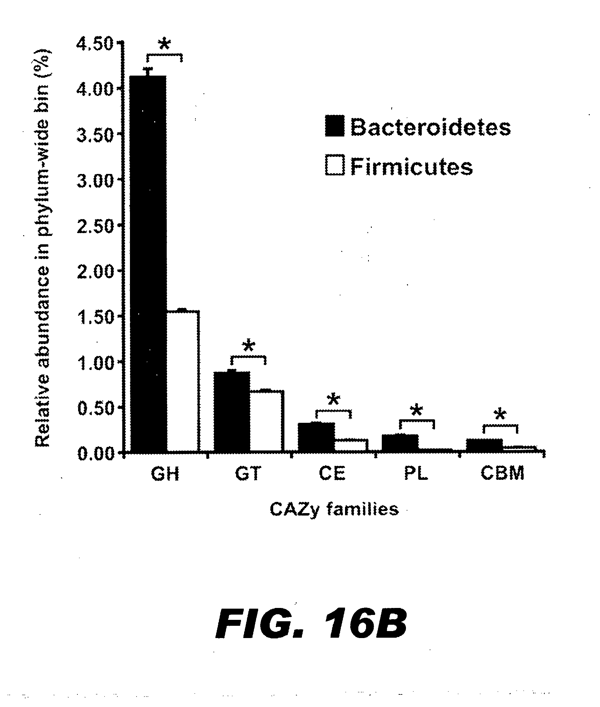

**FIG. 16** depicts the KEGG pathways and Carbohydrate Active Enzymes (CAZy) families whose representation is significantly different between Firmicutes and Bacteroidetes bins. Sequences from each of the 18 fecal microbiomes were binned based on sequence homology to the custom 42-member reference human gut genome database. (A) The frequency of each KEGG pathway was tallied for each bin and significantly different pathways were identified using a bootstrap re-sampling analysis (Xipe v2.4). Significantly different pathways reaching at least 0.6% relative abundance in at least two microbiomes were clustered using single-linkage hierarchical clustering and the Pearson's correlation distance metric. (B) The relative abundance of CAZy families in the Bacteroidetes and Firmicutes sequence bins. Asterisks indicate significant differences (Mann-Whitney test, p<0.0001).

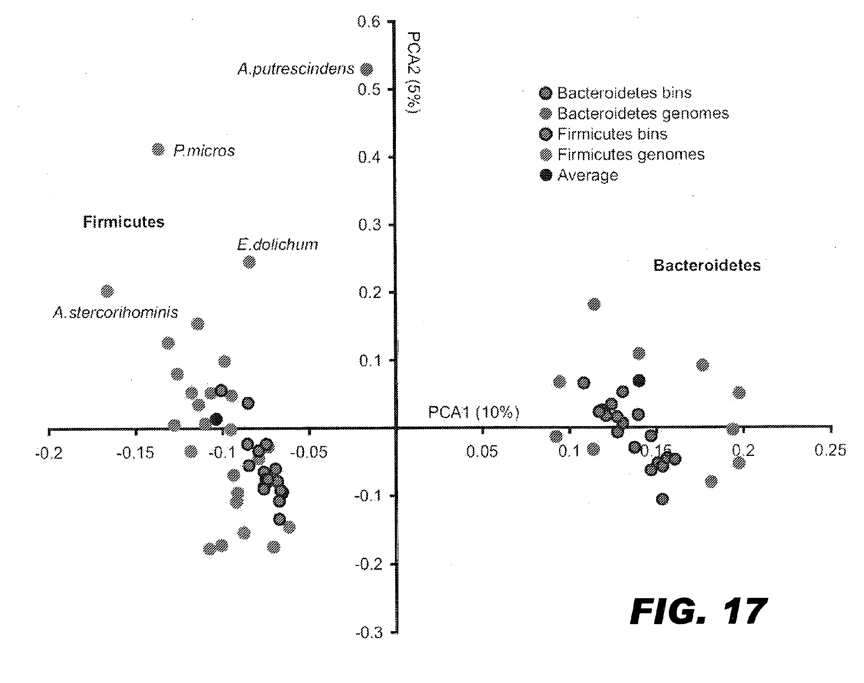

**FIG. 17** depicts the functional clustering of phylum-wide sequence bins and reference genomes from 36 human gut-derived Bacteroidetes and Firmicutes. The frequency of each KEGG pathway in phylum-wide sequence bins, and in 10,000 'simulated reads' generated from each of the reference genomes (Readsim v0.10; ref. 56), was tallied and pathways reaching at least 0.6% relative abundance in at least two fecal microbiomes were clustered using principal components analysis (PCA). An 'average' Firmicutes and Bacteroidetes genome was generated by pooling all reads generated from genomes within each phylum.

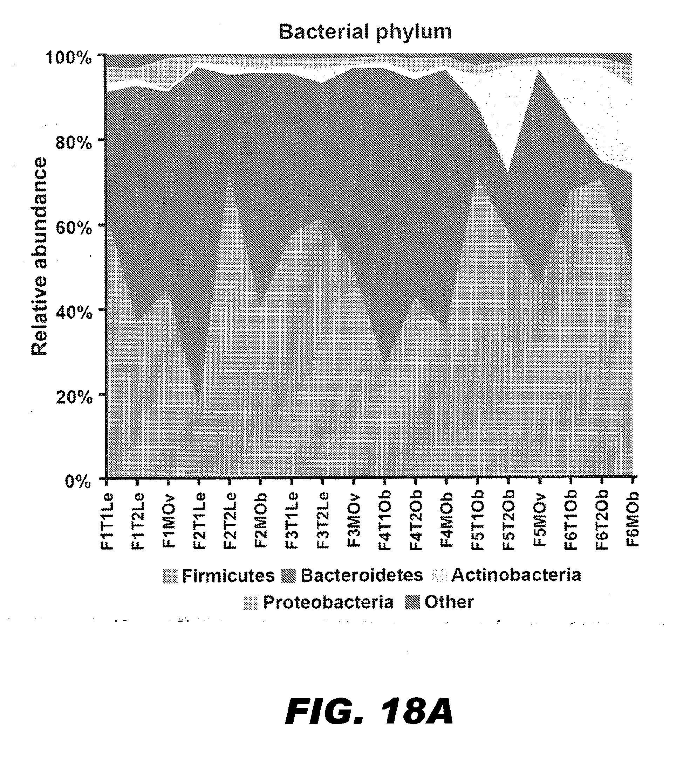

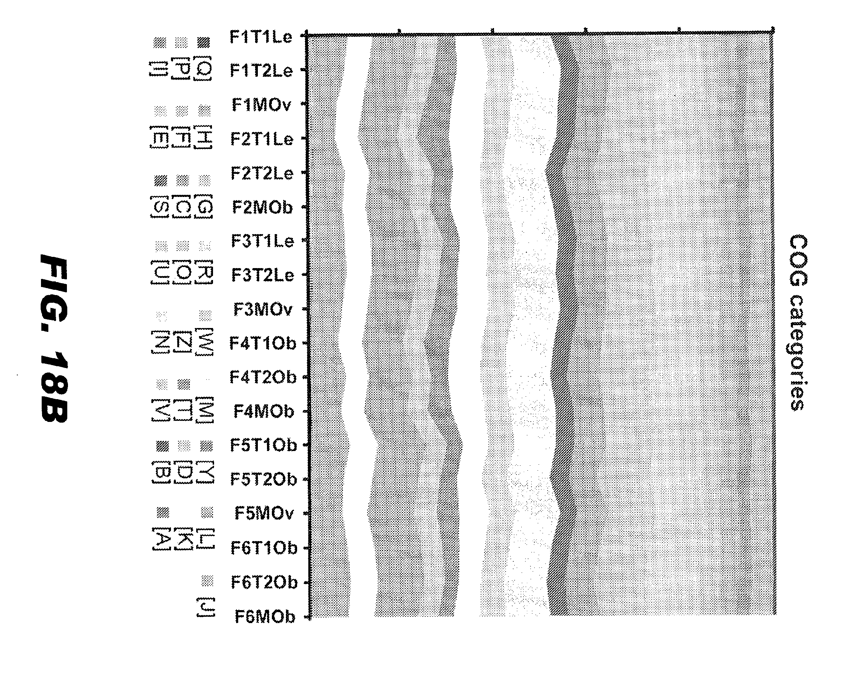

**FIG. 18** depicts the comparison of taxonomic and functional variations in the human gut microbiome. (A) Relative abundance of major phyla across 18 fecal microbiomes from MZ twins and their mothers, based on BLASTX comparisons of microbiomes and the NCBI non-redundant database. (B) Relative abundance of COG categories across each sampled gut microbiome.

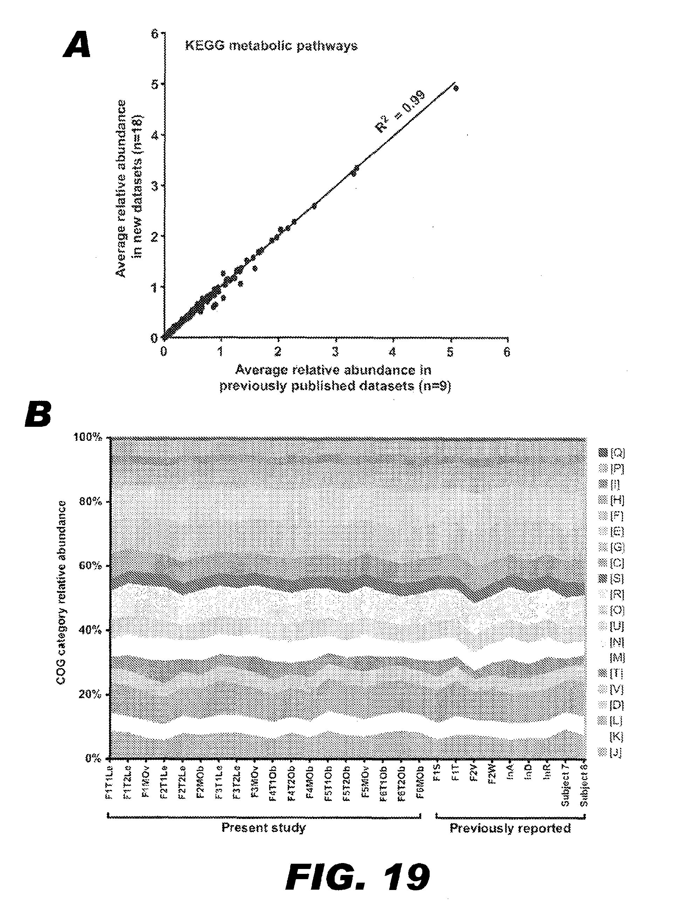

**FIG. 19** depicts the relative abundance of KEGG pathways and COG categories in the gut microbiomes of 18 individuals (6 MZ twin pairs and their mothers), plus 9 previously published adult microbiomes. 'Simulated reads' were generated from each of the 9 previously published microbiomes datasets obtained by capillary sequencing to mimic pyrosequencing reads, then re-annotated using the KEGG and STRING-extended COG databases. (A) The average relative abundance of KEGG pathways in MZ twin pairs and their mothers graphed as a function of the average relative abundance of KEGG pathways in the 9 previously published adult gut microbiome datasets. (B) The distribution of COG categories across all 27 datasets.

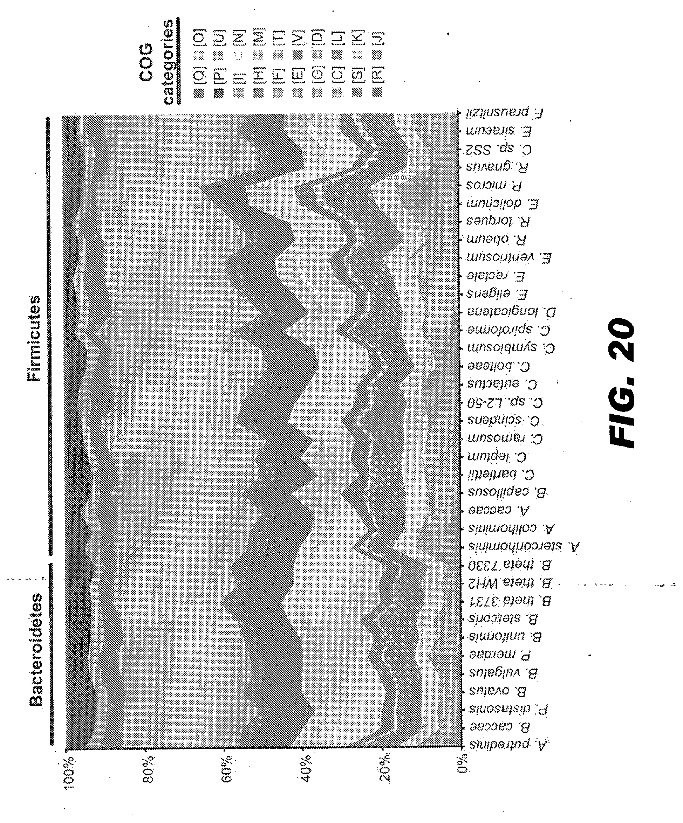

**FIG. 20** depicts the relative abundance of COG categories in 36 sequenced reference human gut-derived Firmicutes and Bacteroidetes genomes. 10,000 'simulated reads', generated from each of the reference genomes (Readsim v0.10), were annotated using the STRING-extended COG database.

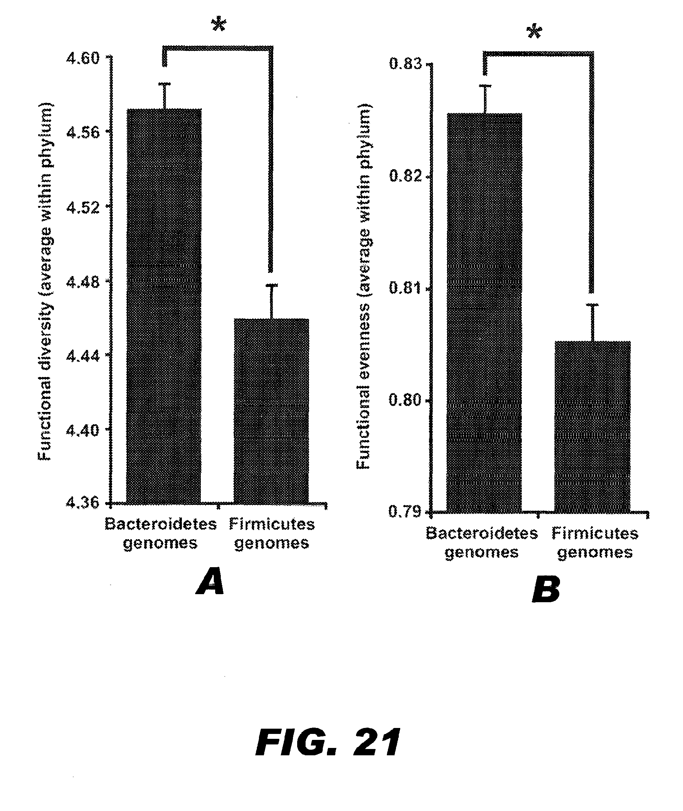

**FIG. 21** depicts the average functional diversity and evenness of 'simulated reads' generated from reference genomes from gut Firmicutes or Bacteroidetes. (A) Functional diversity was calculated in Estimates (v8.0), based on the abundance of each metabolic pathway across 10,000 'simulated reads' generated from each of the 36 reference genomes (Readsim v0.10). (B) Shannon evenness. Asterisks indicate significant differences (Mann-Whitney test, p<0.01).

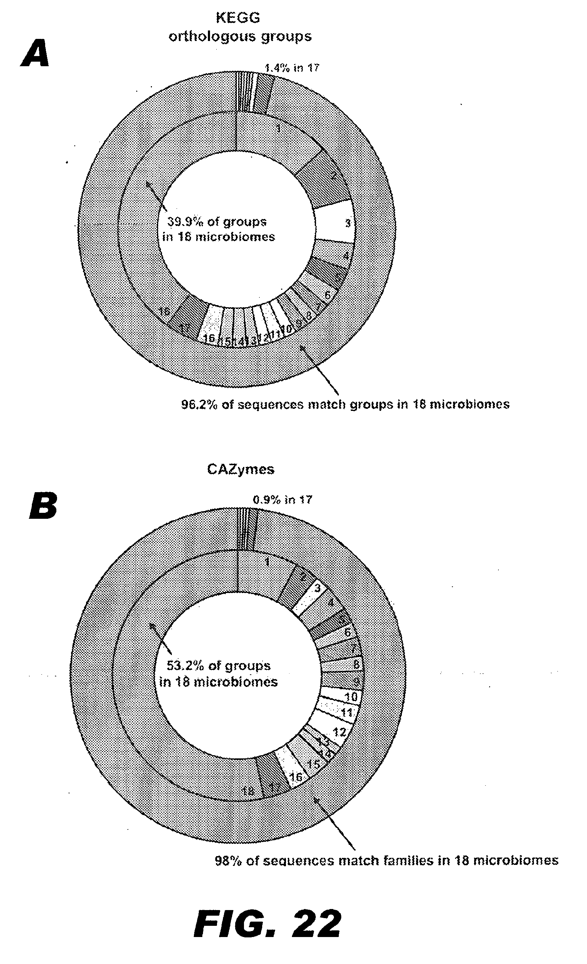

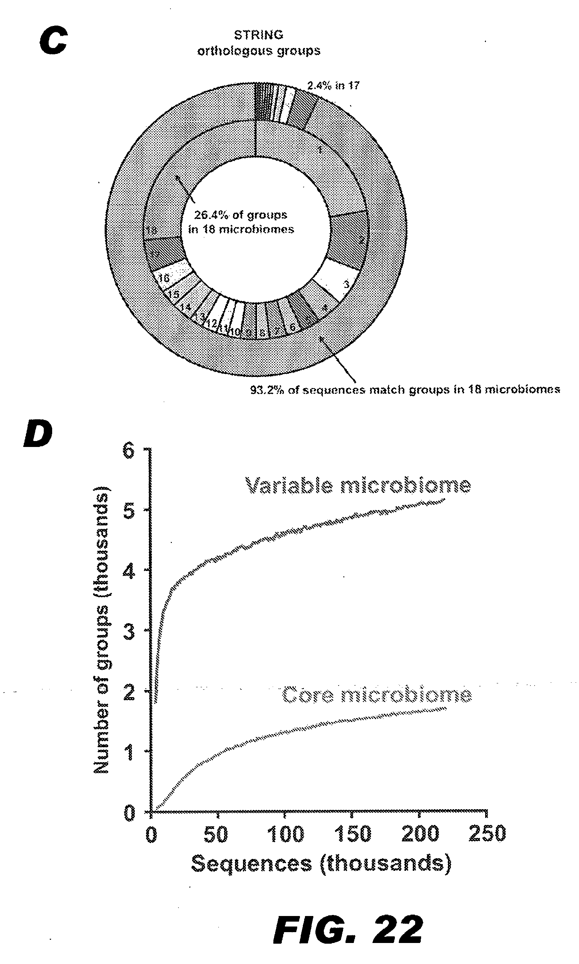

**FIG. 22** depicts the 'enzyme'-level functional groups shared between all or a subset of the sampled gut microbiomes. Sequences from each of the 18 microbiomes characterized in this study were assigned to (A) KEGG groups, (B) CAZy families, and (C) STRING annotations. Functional groups (inner circle), and the sequences assigned to each group (outer circle) were then tallied based on their co-occurrence in any combination of 1 to 18 microbiomes. For example, the outer aquacolored segment in Panel A demonstrates that 96.2% of the total sequences generated from all 18 samples were assigned to functional grouips that were common to all 18 microbiomes. (D) KEGG categories enriched or depleted in the core versus variable components of the gut microbiome. Sequences from each of the 18 fecal microbiomes were binned into the 'core' or 'variable' microbiome-based on the co-occurrence of KEGG orthologous groups (core groups were found in all 18 microbiomes while variable groups were present in fewer (<18) microbiomes; see **FIG. 20A**). General categories are shown. Asterisks indicate significant differences (Student's t-test, *p<0.05, **p<0.001, ***p<10-5).

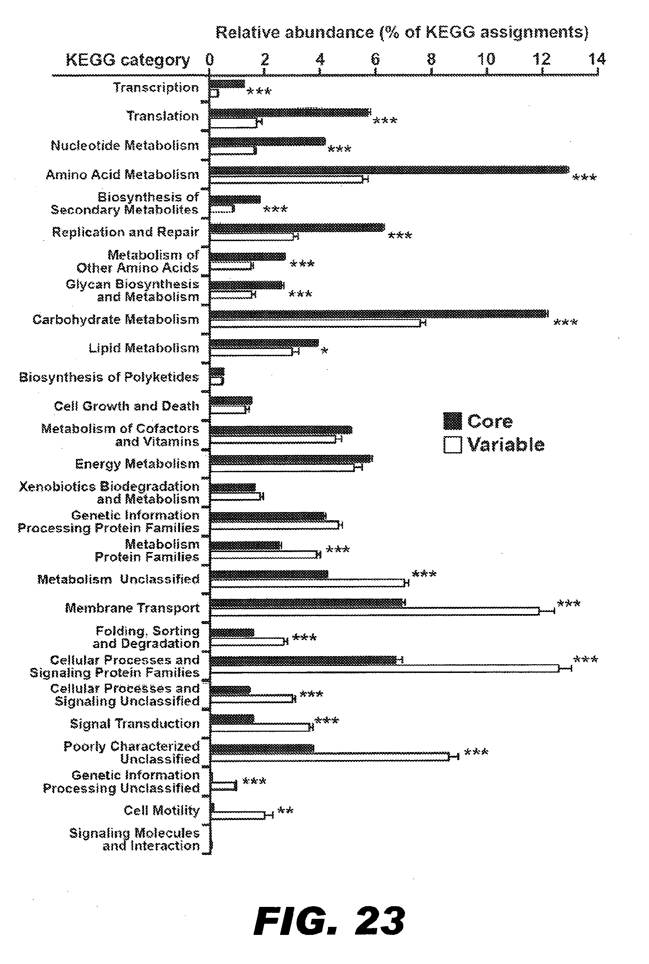

**FIG. 23** depicts the KEGG categories enriched or depleted in the core versus variable components of the gut microbiome. Sequences from each of the 18 fecal microbiomes were binned into the 'core' or 'variable' microbiome based on the co-occurrence of KEGG orthologous groups (core groups were found in all 18 microbiomes while variable groups were present in fewer (<18) microbiomes; see **FIG. 20A**). General categories are shown. Asterisks indicate significant differences (Student's t-test, *p<0.05, **p<0.001, ***p<10-5).

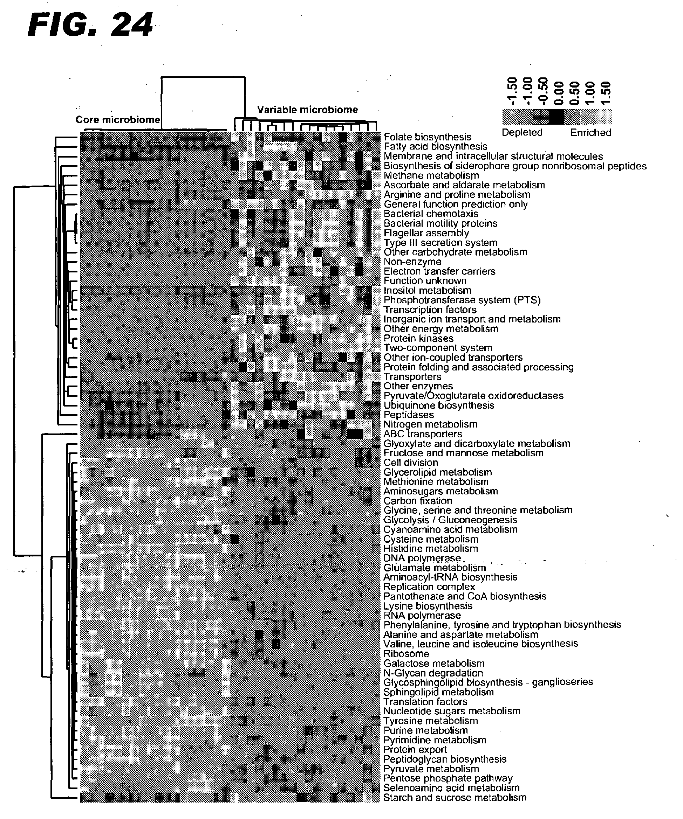

**FIG. 24** depicts the clustering of pathways enriched or depleted in the core microbiome. Sequences from each of the 18 distal gut microbiomes were binned into the 'core' or 'variable' microbiome based on the co-occurrence of KEGG orthologous groups [core groups were found in all 18 microbiomes while variable groups were present in fewer (<18) microbiomes; see **FIG. 20A**]. The frequency of each KEGG pathway was tallied for each bin and significantly different pathways were identified using a bootstrap re-sampling analysis (Xipe v2.4). Pathways significantly enriched (yellow) or depleted (blue), reaching at least 0.6% relative abundance in at least two microbiomes, were clustered using single-linkage hierarchical clustering and the Pearson's correlation distance metric.

### DETAILED DESCRIPTION OF THE INVENTION

It has been discovered, as demonstrated in the Examples, that there is a relationship between the human gut microbiota and obesity. In particular, an obese human subject typically has fewer Bacteroidetes and more Actinobacteria compared to a lean subject. An obese human subject may have proportionately fewer Bacteroidetes and more Actinobacteria and Firmicutes compared to a lean subject. The invention provides nucleic acid sequences that are associated with obesity in humans. These sequences may be used as diagnostic or prognostic biomarkers for obesity risk, biomarkers for drug discovery, biomarkers for the discovery of therapeutic targets involved in the regulation of energy balance, and biomarkers for the efficacy of a weight loss program.

### I. Biomarkers Comprising the Gut Microbiome

The gut microbiome may be used as a biomarker for obesity. The biomarker may be utilized to construct arrays that may be used for several applications including as a diagnostic or prognostic tool to determine obesity risk, judge the efficacy of existing weight loss regimes, aid in drug discovery, identify additional biomarkers involved in obesity or an obesity related disorder, and aid in the discovery of therapeutic targets involved in the regulation of energy balance, including but not limited to those that may directly affect the composition of the gut microbiome. Generally speaking, the array may comprise nucleic acid sequences modulated in an obese host microbiome or a lean host microbiome.

### (a) Array

The array may be comprised of a substrate having disposed thereon biomolecules that are modulated in an obese host microbiome compared to a lean host microbiome, the biomolecules being at least each of the nucleic acid sequences listed in Table 13 or 14, or nucleic acid sequences capable of specifically hybridizing to each of the nucleic acid sequences listed in Table 13 or 14. Several substrates suitable for the construction of arrays are known in the art, and one skilled in the art will appreciate that other substrates may become available as the art progresses. The substrate may be a material that may be modified to contain discrete individual sites appropriate for the attachment or association of the biomolecules and is amenable to at least one detection method. Non-limiting examples of substrate materials include glass, modified or functionalized glass, plastics (including acrylics, polystyrene and copolymers of styrene and other materials, polypropylene, polyethylene, polybutylene, polyurethanes, TeflonJ, etc.), nylon or nitrocellulose, polysaccharides, nylon, resins, silica or silica-based materials including silicon and modified silicon, carbon, metals, inorganic glasses and plastics. In an exemplary embodiment, the substrates may allow optical detection without appreciably fluorescing.

A substrate may be planar, a substrate may be a well, i.e. a 364 well plate, or alternatively, a substrate may be a bead. Additionally, the substrate may be the inner surface of a tube for flow-through sample analysis to minimize sample volume. Similarly, the substrate may be flexible, such as a flexible foam, including closed cell foams made of particular plastics.

The biomolecules may be attached to the substrate in a wide variety of ways, as will be appreciated by those in the art. The biomolecules may either be synthesized first, with subsequent attachment to the substrate, or may be directly synthesized on the substrate. The substrate and the biomolecules may be derivatized with chemical functional groups for subsequent attachment of the two. For example, the substrate may be derivatized with a chemical functional group including, but not limited to, amino groups, carboxyl groups, oxo groups or thiol groups. Using these functional groups, the biomolecule may be attached using functional groups on the biomolecule either directly or indirectly using linkers.

The biomolecules may also be attached to the substrate non-covalently. For example, biotinylated biomolecules can be prepared, which may bind to surfaces covalently coated with streptavidin, resulting in attachment. Alternatively, biomolecules may be synthesized on the surface using techniques such as photopolymerization and photolithography. Additional methods of attaching biomolecules to arrays and methods of synthesizing biomolecules on substrates are well known in the art, i.e. VLSIPS technology from Affymetrix (e.g., see U.S. Patent 6,566,495, and Rockett and Dix, "DNA arrays: technology, options and toxicological applications," Xenobiotica 30(2):155-177, all of which are hereby incorporated by reference in their entirety).

In one embodiment, the biomolecules attached to the substrate are located at a spatially defined address of the array. Arrays may comprise from about 1 to about several hundred thousand addresses or more. In one embodiment, the array may be comprised of less than 10,000 addresses. In another alternative embodiment, the array may be comprised of at least 10,000 addresses. In yet another alternative embodiment, the array may be comprised of less than 5,000 addresses. In still another alternative embodiment, the array may be comprised of at least 5,000 addresses. In a further embodiment, the array may be comprised of less than 500 addresses. In yet a further embodiment, the array may be comprised of at least 500 addresses.

A biomolecule may be represented more than once on a given array. In other words, more than one address of an array may be comprised of the same biomolecule. In some embodiments, two, three, or more than three addresses of the array may be comprised of the same biomolecule. In certain embodiments, the array may comprise control biomolecules and/or control addresses. The controls may be internal controls, positive controls, negative controls, or background controls.

The array may be comprised of biomolecules indicative of an obese host microbiome (e.g. the nucleic acid sequences listed in Table 13). Alternatively, the array may be comprised of biomolecules indicative of a lean host microbiome (e.g. the nucleic acid sequences listed in Table 14). A biomolecule is "indicative" of an obese or lean microbiome if it tends to appear more often in one type of microbiome compared to the other. Additionally, the array may be comprised of biomolecules that are modulated in the obese host microbiome compared to the lean host microbiome. As used herein, "modulated" may refer to a biomolecule whose representation or activity is different in an obese host microbiome compared to a lean host microbiome. For instance, modulated may refer to a biomolecule that is enriched, depleted, up-regulated, down-regulated, degraded, or stabilized in the obese host microbiome compared to a lean host microbiome. In one embodiment, the array may be comprised of biomolecules enriched in the obese host microbiome compared to the lean host microbiome. In another embodiment, the array may be comprised of biomolecules depleted in the obese host microbiome compared to the lean host microbiome. In yet another embodiment, the array may be comprised of biomolecules up-regulated in the obese host microbiome compared to the lean host microbiome. In still another embodiment, the array may be comprised of biomolecules down-regulated in the obese host microbiome compared to the lean host microbiome. In still yet another embodiment, the array may be comprised of biomolecules degraded in the obese host microbiome compared to the lean host microbiome. In an alternative embodiment, the array may be comprised of biomolecules stabilized in the obese host microbiome compared to the lean host microbiome.

As used herein, "biomolecule" refers to a nucleic acid. Nucleic acids may include RNA, DNA, and naturally occurring or synthetically created derivatives. A biomolecule may be present in, produced by, or modified by a microorganism within the gut.

In one embodiment, the biomolecules of the array may be selected from the biomolecules listed in Table 13. For instance, the biomolecules of the array may be selected from the group comprising nucleic acids corresponding to SEQ ID NO:1 through SEQ ID NO:273. In another embodiment, the biomolecules of the array may be selected from the biomolecules listed in Table 14. For instance, the biomolecules of the array may be selected from the group comprising nucleic acids corresponding to SEQ ID NO:274 through SEQ ID NO:383. In yet another embodiment, the biomolecules of the array may be selected from the biomolecules listed in Table 13 and Table 14, for instance, the nucleic acids corresponding to SEQ ID NO:1 through SEQ ID NO:383.

For each of the above embodiments, methods of determining biomolecules that are indicative of, or modulated in, an obese host microbiome compared to a lean host microbiome may be determined using methods detailed in the Examples.

The arrays may be utilized in several suitable applications. For example, the arrays may be used in methods for detecting association between two or more biomolecules. This method typically comprises incubating a sample with the array under conditions such that the biomolecules comprising the sample may associate with the biomolecules attached to the array. The association is then detected, using means commonly known in the art, such as fluorescence. "Association," as used in this context, may refer to hybridization, covalent binding, or ionic binding. A skilled artisan will appreciate that conditions under which association may occur will vary depending on the biomolecules, the substrate, and the detection method utilized. As such, suitable conditions may have to be optimized for each individual array created.

In yet another embodiment, the array may be used as a tool in a method to determine whether a compound has efficacy for treatment of obesity or an obesity-related disorder in a host. Alternatively, the array may be used as a tool in a method to determine whether a compound increases or decreases the relative abundance of Bacteriodes, Actinobacteria, or Firmicutes in a subject. Typically, such methods comprise comparing a plurality of biomolecules of the host's microbiome before and after administration of a compound, such that if the abundance of biomolecules associated with obesity decreased after treatment, or the abundance of biomolecules indicative of Bacteroides increases, or the abundance of biomolecules indicative of Firmicutes and/or Actinobacteria decreases, the compound may be efficacious in treating obesity in a host.

The array may also be used to quantitate the plurality of biomolecules of the host microbiome before and after administration of a compound. The abundance of each biomolecule in the plurality may then be compared to determine if there is a decrease in the abundance of biomolecules associated with obesity after treatment.

In some embodiments, the array may be used as a diagnostic or prognostic tool to identify subjects that are susceptible to more efficient energy harvesting, and therefore, more susceptible to weight gain and/or obesity. Such a method may generally comprise incubating the array with biomolecules derived from the subject's gut microbiome to determine the relative abundance of nucleic acids or nucleic acid products associated with Bacteroidetes, Actinobacteria, or Firmictues. In some embodiments, the array may be used to determine the relative abundance of Mollicutes, Mollicute-associated nucleic acids, or Mollicute-associated nucleic acid products in a subject's gut microbiome. Methods to collect, isolate, and/or purify biomolecules from the gut microbiome of a subject to be used in the above methods are known in the art, and are detailed in the examples.

### II. Kits

Described herein is a kit for evaluating a compound, therapeutic, or drug. Typically, the kit comprises an array as described above and a computer-readable medium. The computer-readable medium may have a plurality of digitally-encoded profiles wherein each profile of the plurality has a plurality of values, each value representing the abundance of a biomolecule in a host microbiome detected by the array. The array may be used to determine a profile for a particular host under particular conditions, and then the computer-readable medium may be used to determine if the profile is similar to known profile stored on the computer-readable medium. Non-limiting examples of possible known profiles include obese and lean profiles for several different hosts, for example, rodents, humans, livestock animals, companion animals, or zoological animals.

### DEFINITIONS

The term "abundance" refers to the representation of a given taxonomic group (e.g. phylum, order, family, genera, or species) of microorganism present in the gastrointestinal tract of a subject.

The term "activity of the microbiota population" refers to the microbiome's ability to harvest energy and nutrients.

The term "antagonist" refers to a molecule that inhibits or attenuates the biological activity of a Fiaf polypeptide and in particular, the ability of Fiaf to inhibit LPL, and/or the ability of the microbiota to regulate Fiaf. Antagonists may include proteins such as antibodies, nucleic acids, carbohydrates, small molecules, or other compounds or compositions that modulate the activity of a Fiaf polypeptide either by directly interacting with the polypeptide or by acting on components of the biological pathway in which Fiaf participates.

The term "agonist" refers to a molecule that enhances or increases the biological activity of a Fiaf polypeptide and in particular, the ability of Fiaf to inhibit LPL. Agonists may include proteins, peptides, nucleic acids, carbohydrates, small molecules (e.g., such as metabolites), or other compounds or compositions that modulate the activity of a Fiaf polypeptide either by directly interacting with the polypeptide or by acting on components of the biological pathway in which Fiaf participates.

The term "altering" as used in the phrase "altering the microbiota population" is to be construed in its broadest interpretation to mean a change in the representation of microbes or the functions/activities of microbial communities in the gastrointestinal tract of a subject. The change may be a decrease or an increase in the presence of a particular microbial species, genus, family, order, or class, or change in the expression of microbial community associated nucleic acids or a change in the protein and metabolic products produced by members of the community.

"BMI" as used herein is defined as a human subject's weight (in kilograms) divided by height (in meters) squared.

An "effective amount" is a therapeutically-effective amount that is intended to qualify the amount of agent that will achieve the goal of a decrease in body fat, or in promoting weight loss.

Fas stands for fatty acid synthase.

Fiaf stands for fasting-induced adipocyte factor, also known as angiopoietin like protein 4 (Angpltl4).

LPL stands for lipoprotein lipase.

The term "obesity-related disorder" includes disorders resulting from, at least in part, obesity. Representative disorders include metabolic syndrome, type II diabetes, hypertension, cardiovascular disease, and nonalcoholic fatty liver disease.

The term "metagenomics" refers to the application of modern genomic techniques to the study of the composition and operations of communities of microbial organisms sampled directly in their natural environments, by passing the need for isolation and lab cultivation of individual species.

PPAR stands for peroxisome proliferator-activator receptor.

A "subject in need of treatment for obesity" generally will have at least one of three criteria: (i) BMI over 30; (ii) 100 pounds overweight; or (iii) 100% above an "ideal" body weight as determined by generally recognized weight charts.

### EXAMPLES

The following examples illustrate various iterations of the invention.

### Example 1: The gut microbiota is linked to family and BMI

The bacterial lineages of the human gut microbiota are largely unexplored. In this study, the lineages of gut microbiota of 31 monozygotic (MZ) twin pairs, 23 dizygotic (DZ) twin pairs, and where available their mothers (n=46), were characterized. (**Tables 1-5**). MZ and DZ co-twins and parent-offspring pairs provide an attractive paradigm for assessing the impact of genotype and shared early environment exposures on the gut microbiome. Moreover, genetically 'identical' MZ twin pairs gain weight in response to overfeeding in a more reproducible way than do unrelated individuals and are more concordant for body mass index (BMI) than dizygotic twin pairs, suggesting shared features of their energy balance influenced by host genotype.

| **Table 1: V2/31 165 rRNA gene sequencing statistics** | | | | | | | | |
|---|---|---|---|---|---|---|---|---|
| Subject ID | Data ID time-point | Family number | Twin /Mom | Ancestry | Zygosity | BMI category | Months without Antibiotics | Total sequences |
| F1T1Le1 | TS1 | 1 | Twin | EA | MZ | Lean | >6 | 6415 |
| F1T1Le2 | TS1.2 | 1 | Twin | EA | MZ | Lean | >6 | 1627 |
| F1T2Le1 | TS2 | 1 | Twin | EA | MZ | Lean | NA | 15495 |
| F1T2Le2 | TS2.2 | 1 | Twin | EA | MZ | Lean | >6 | 1957 |
| F1MOv1 | TS3 | 1 | Mom | EA | NA | Overweight | >6 | 7870 |
| F1MOv2 | TS3.2 | 1 | Mom | EA | NA | Overweight | >6 | 1799 |
| F2T1Le1 | TS4 | 2 | Twin | EA | MZ | Lean | >6 | 9343 |
| F2T1Le2 | TS4.2 | 2 | Twin | EA | MZ | Lean | >6 | 2886 |
| F2T2Le1 | TS5 | 2 | Twin | EA | MZ | Lean | >6 | 13991 |
| F2T2Le2 | TS5.2 | 2 | Twin | EA | MZ | Lean | >6 | 3606 |
| F2MOb1 | TS6 | 2 | Mom | EA | NA | Obese | >6 | 7717 |
| F2MOb2 | TS6.2 | 2 | Mom | EA | NA | Obese | >6 | 4325 |
| F3T1Le1 | TS7 | 3 | Twin | EA | MZ | Lean | >6 | 11808 |
| F3T1Le2 | TS7.2 | 3 | Twin | EA | MZ | Lean | >6 | 2962 |
| F3T2Le1 | TS8 | 3 | Twin | EA | MZ | Lean | >6 | 16793 |
| F3T2Le2 | TS8.2 | 3 | Twin | EA | MZ | Lean | >6 | 632 |
| F3Mov1 | TS9 | 3 | Mom | EA | NA | Overweight | >6 | 11291 |
| F3MOb2 | TS9.2 | 3 | Mom | EA | NA | Obese | >6 | 2965 |
| F4T1Ob1 | TS10 | 4 | Twin | AA | MZ | Obese | >6 | 2280 |
| F4T1Ob2 | TS10.2 | 4 | Twin | AA | MZ | Obese | >6 | 979 |
| F4T2Ob1 | TS11 | 4 | Twin | AA | MZ | Obese | >6 | 2458 |
| F4T2Ob2 | TS11.2 | 4 | Twin | AA | MZ | Obese | >6 | 2437 |
| F4MOb1 | TS12 | 4 | Mom | AA | NA | Obese | >1 | 2086 |
| F4MOb2 | TS12.2 | 4 | Mom | AA | NA | Obese | >2 | 1692 |
| F5T1Le1 | TS13 | 5 | Twin | EA | MZ | Lean | >6 | 8509 |
| F5T1Le2 | TS13.2 | 5 | Twin | EA | MZ | Lean | >6 | 1689 |
| F5T2Le1 | TS14 | 5 | Twin | EA | MZ | Lean | >6 | 15903 |
| F5MOv1 | TS15 | 5 | Mom | EA | NA | Overweight | >6 | 15690 |
| F5MOv2 | TS15.2 | 5 | Mom | EA | NA | Overweight | >6 | 3967 |
| F5T1Le1 | TS16 | 6 | Twin | EA | MZ | Lean | NA | 5975 |
| F5T2Le1 | TS17 | 6 | Twin | EA | MZ | Lean | >6 | 1182 |
| F7T1Ob1 | TS19 | 7 | Twin | EA | MZ | Obese | >6 | 21459 |
| F7T1Ob2 | TS19.2 | 7 | Twin | EA | MZ | Obese | >6 | 3953 |
| F7T2Ob1 | TS20 | 7 | Twin | EA | MZ | Obese | >6 | 32871 |
| F7T2Ob2 | TS20.2 | 7 | Twin | EA | MZ | Obese | >6 | 5045 |
| F7MOb1 | TS21 | 7 | Mom | EA | NA | Obese | >6 | 26781 |
| F7MOb2 | TS21.2 | 7 | Mom | EA | NA | Obese | >6 | 4752 |
| F8T1Le1 | TS22 | 8 | Twin | EA | MZ | Lean | >6 | 5110 |
| F8T2Le1 | TS23 | 8 | Twin | EA | MZ | Lean | >6 | 1978 |
| F9T1Le1 | TS25 | 9 | Twin | EA | MZ | Lean | >6 | 10017 |
| F9T1Le2 | TS25.2 | 9 | Twin | EA | MZ | Lean | >6 | 4626 |
| F9T2Le1 | TS26 | 9 | Twin | EA | MZ | Lean | >6 | 16757 |
| F9T2Le2 | TS26.2 | 9 | Twin | EA | MZ | Lean | >6 | 5111 |
| F9MOb1 | TS27 | 9 | Mom | EA | NA | Obese | >6 | 11885 |
| F9MOb2 | TS27.2 | 9 | Mom | EA | NA | Obese | >6 | 2068 |
| F10T1Ob1 | TS28 | 10 | Twin | EA | MZ | Obese | >6 | 6694 |
| F10T2Ob1 | TS29 | 10 | Twin | EA | MZ | Obese | >6 | 2411 |
| F10MOv1 | TS30 | 10 | Mom | EA | NA | Overweight | >6 | 8273 |
| F10MLe2 | TS30.2 | 10 | Mom | EA | NA | Lean | >6 | 3280 |
| F11T1Le1 | TS31 | 11 | Twin | EA | MZ | Lean | >6 | 18941 |
| F11T1Le2 | TS31.2 | 11 | Twin | EA | MZ | Lean | >6 | 5842 |
| F11T2Le1 | TS32 | 11 | Twin | EA | MZ | Lean | >6 | 9773 |
| F11T2Le2 | TS32.2 | 11 | Twin | EA | MZ | Lean | >6 | 6178 |
| F11MOv1 | TS33 | 11 | Mom | EA | NA | Overweight | >6 | 18037 |
| F11MOv2 | TS33.2 | 11 | Mom | EA | NA | Overweight | >6 | 1593 |
| F12T1Ob1 | TS34 | 12 | Twin | EA | MZ | Obese | >6 | 1730 |
| F12T2Ob1 | TS35 | 12 | Twin | EA | MZ | Obese | >6 | 3887 |
| F13T1Ob1 | TS37 | 13 | Twin | EA | MZ | Obese | >6 | 3534 |
| F13T1Ob2 | TS37.2 | 13 | Twin | EA | MZ | Obese | >6 | 4458 |
| F13T2Ov1 | TS38 | 13 | Twin | EA | MZ | Overweight | >6 | 3043 |
| F13T2Ov2 | TS38.2 | 13 | Twin | EA | MZ | Overweight | >6 | 2566 |
| F13MOb1 | TS39 | 13 | Mom | EA | NA | Obese | >6 | 5848 |
| F13MOb2 | TS39.2 | 13 | Mom | EA | NA | Obese | >6 | 2146 |
| F14T1Ob1 | TS43 | 14 | Twin | EA | MZ | Obese | >6 | 2905 |
| F14T2Ob1 | TS44 | 14 | Twin | EA | MZ | Obese | >6 | 1621 |
| F15T1Ob1 | TS49 | 15 | Twin | EA | MZ | Obese | >6 | 11936 |
| F15T1Ob2 | TS49.2 | 15 | Twin | EA | MZ | Obese | >6 | 4220 |
| F15T2Ob1 | TS50 | 15 | Twin | EA | MZ | Obese | >6 | 12672 |
| F15T2Ob2 | TS50.2 | 15 | Twin | EA | MZ | Obese | >6 | 4603 |
| F15MOb1 | TS51 | 15 | Mom | EA | NA | Obese | >6 | 13789 |
| F15MOb2 | TS51.2 | 95 | Mom | EA | NA | Obese | >6 | 3284 |
| F16T1Ob1 | TS55 | 16 | Twin | EA | DZ | Obese | >6 | 3817 |
| F16T1Ob2 | TS55.2 | 16 | Twin | EA | DZ | Obese | >6 | 5210 |
| F16TZOb1 | TS56 | 16 | Twin | EA | DZ | Obese | >6 | 5147 |
| F16T2Ob2 | TS56.2 | 16 | Twin | EA | DZ | Obese | >6 | 4490 |
| F16MOb1 | TS57 | 16 | Mom | EA | NA | Obese | >0 | 8440 |
| F16MOb2 | TS57.2 | 16 | Mom | EA | NA | Obese | >1 | 2365 |
| F17T1Ob1 | TS61 | 17 | Twin | EA | DZ | Obese | >6 | 672 |
| F17T1Ob2 | TS61.2 | 17 | Twin | EA | DZ | Obese | >6 | 3738 |
| F17T2Ob1 | TS62 | 17 | Twin | EA | DZ | Obese | >6 | 2311 |
| F17T2Ob2 | TS62.2 | 17 | Twin | EA | DZ | Obese | >6 | 3821 |
| F17MOb1 | TS63 | 17 | Mom | EA | NA | Obese | >6 | 2132 |
| F17MOb2 | TS63.2 | 17 | Mom | EA | NA | Obese | >6 | 1853 |
| F18T1Ov1 | TS64 | 18 | Twin | EA | MZ | Overweight | >6 | 4571 |
| F18T1Ov2 | TS64.2 | 18 | Twin | EA | MZ | Overweight | >6 | 4523 |
| F18T2Ob1 | TS65 | 18 | Twin | EA | MZ | Obese | >6 | 2502 |
| F18T2Ob2 | TS65.2 | 18 | Twin | EA | MZ | Obese | >6 | 3943 |
| F18MOb1 | TS66 | 18 | Mom | EA | NA | Obese | >6 | 3491 |
| F18MOb2 | TS66.2 | 18 | Mom | EA | NA | Obese | >6 | 6187 |
| F19T1Ob1 | TS67 | 19 | Twin | EA | DZ | Obese | NA | 988 |
| F19T1Ob2 | TS67.2 | 19 | Twin | EA | DZ | Obese | NA | 1861 |
| F19T2Ob1 | TS68 | 19 | Twin | EA | DZ | Obese | >6 | 3870 |
| F19T2Ob2 | TS68.2 | 19 | Twin | EA | DZ | Obese | >6 | 2242 |
| F19MOb1 | TS69 | 19 | Mom | EA | NA | Obese | >6 | 5290 |
| F19MOb2 | TS69.2 | 19 | Mom | EA | NA | Obese | >0 | 2305 |
| F20T1Obt | TS70 | 20 | Twin | EA | DZ | Obese | >6 | 2139 |
| F20T1Ob2 | TS70.2 | 20 | Twin | EA | DZ | Obese | >6 | 2166 |
| F20T2Ob1 | TS71 | 20 | Twin | EA | DZ | Obese | >6 | 3130 |
| F20T2Ob2 | TS71.2 | 20 | Twin | EA | DZ | Obese | >6 | 2293 |
| F20MOb1 | TS72 | 20 | Mom | EA | NA | Obese | >6 | 1674 |
| F20MOb2 | TS72.2 | 20 | Mom | EA | NA | Obese | >6 | 376 |
| F21T1Ob1 | TS73 | 21 | Twin | EA | DZ | Obese | >6 | 2963 |
| F21T2Ob1 | TS74 | 21 | Twin | EA | DZ | Obese | >6 | 2177 |
| F21T2Ob2 | TS74.2 | 21 | Twin | EA | DZ | Obese | >6 | 1791 |
| F21MOb1 | TS75 | 21 | Mom | EA | NA | Obese | >6 | 1434 |
| F21MOb2 | TS75.2 | 21 | Mom | EA | NA | Obese | >6 | 1887 |
| F22T1Ob1 | TS76 | 22 | Twin | AA | MZ | Obese | >6 | 2977 |
| F22T1Ob2 | TS76.2 | 22 | Twin | AA | MZ | Obese | >6 | 1962 |
| F22T2Ov1 | TS77 | 22 | Twin | AA | MZ | Overweight | >6 | 2168 |
| F22MOb1 | TS78 | 22 | Mom | AA | NA | Obese | >6 | 1460 |
| F22MOb2 | TS78.2 | 22 | Mom | AA | NA | Obese | >6 | 2482 |
| F23T1Ob1 | TS82 | 23 | Twin | AA | MZ | Obese | >6 | 1628 |
| F23T1Ob2 | TS82.2 | 23 | Twin | AA | MZ | Obese | >6 | 1673 |
| F23T2Ob1 | TS83 | 23 | Twin | AA | MZ | Obese | >6 | 1572 |
| F23T2Ob2 | TS83.2 | 23 | Twin | AA | MZ | Obese | >6 | 3349 |
| F23MOb1 | TS84 | 23 | Mom | AA | NA | Obese | >6 | 2215 |
| F23MOb2 | TS84.2 | 23 | Mom | AA | NA | Obese | >6 | 2033 |
| F24T1Ob1 | TS85 | 24 | Twin | EA | DZ | Overweight | >3 | 2385 |
| F24T1Ov2 | TS85.2 | 24 | Twin | EA | DZ | Overweight | >6 | 2122 |
| F24T1Ob1 | TS86 | 24 | Twin | EA | DZ | Obese | >1 | 4107 |
| F24T2Ob2 | TS86.2 | 24 | Twin | EA | DZ | Obese | >3 | 1704 |
| F24MOb1 | TS87 | 24 | Mom | EA | NA | Obese | >6 | 2605 |
| F24MOb1 | TS87.2 | 24 | Mom | EA | NA | Obese | >6 | 1587 |
| F25T1Ob1 | TS88 | 25 | Twin | EA | DZ | Obese | >4 | 2497 |
| F25T1Ob2 | TS88.2 | 25 | Twin | EA | DZ | Obese | >6 | 2129 |
| F25T2Ob1 | TS89 | 25 | Twin | EA | DZ | Obese | >6 | 2108 |
| F25T2Ob2 | TS89.2 | 25 | Twin | EA | DZ | Obese | >6 | 3549 |
| F25MOb1 | TS90 | 25 | Mom | EA | NA | Obese | >6 | 2615 |
| F25MOb2 | TS90.2 | 25 | Mom | EA | NA | Obese | >6 | 2725 |
| F26TtOb1 | TS91 | 26 | Twin | AA | MZ | Obese | >5 | 675 |
| F26TtOb2 | TS91.2 | 26 | Twin | AA | MZ | Obese | >6 | 2307 |
| F26T2Ob1 | TS92 | 26 | Twin | AA | MZ | Obese | >6 | 2036 |
| F26T2Ob2 | TS92.2 | 26 | Twin | AA | MZ | Obese | >6 | 2335 |
| F27T1Ob1 | TS94 | 27 | Twin | AA | MZ | Obese | >6 | 1861 |
| F27T1Ob2 | TS94.2 | 27 | Twin | AA | MZ | Obese | >6 | 2511 |
| F27T2Ob1 | TS95 | 27 | Twin | AA | MZ | Obese | >6 | 2842 |
| F27T2Ob2 | TS95.2 | 27 | Twin | AA | MZ | Obese | >6 | 2550 |
| F27MOb1 | TS96 | 27 | Mom | AA | NA | Obese | >6 | 1516 |
| F27MOb2 | TS96.2 | 27 | Mom | AA | NA | Obese | >6 | 2909 |
| F28T1Ob1 | TS97 | 28 | Twin | AA | DZ | Obese | >6 | 2326 |
| F28T1Ob2 | TS97.2 | 28 | Twin | AA | DZ | Obese | >6 | 2944 |
| F28T2Ob1 | TS98 | 28 | Twin | AA | DZ | Obese | >6 | 2970 |
| F28T2Ob2 | TS98.2 | 28 | Twin | AA | DZ | Obese | >6 | 2851 |
| F28MOv2 | TS99.2 | 28 | Mom | AA | NA | Overweight | >6 | 3136 |
| F29T1Ob1 | TS100 | 29 | Twin | AA | MZ | Obese | >6 | 3504 |
| F29T1Ob2 | TS100.2 | 29 | Twin | AA | MZ | Obese | >6 | 2616 |
| F29T20b2 | TS101.2 | 29 | Twin | AA | MZ | Obese | >6 | 2387 |
| F30T1Ob1 | TS103 | 30 | Twin | AA | MZ | Obese | >6 | 1473 |
| F30T1Ob2 | TS103.2 | 30 | Twin | AA | MZ | Obese | >6 | 3012 |
| F30T2Ob1 | TS104 | 30 | Twin | AA | MZ | Obese | >6 | 1970 |
| F30T2Ob2 | TS104.2 | 30 | Twin | AA | MZ | Obese | >6 | 2895 |
| F30MOb1 | TS105 | 30 | Mom | AA | NA | Obese | >6 | 1864 |
| F30MOb2 | TS105.2 | 30 | Mom | AA | NA | Obese | >6 | 2096 |
| F31T1Ob1 | TS106 | 31 | Twin | AA | MZ | Obese | >6 | 2698 |
| F31T1Ob2 | TS106.2 | 31 | Twin | AA | MZ | Obese | >6 | 2250 |
| F31T2Ob1 | TS107 | 31 | Twin | AA | MZ | Obese | >6 | 3132 |
| F31T2Ob2 | TS107.2 | 31 | Twin | AA | MZ | Obese | >6 | 4521 |
| F32T1Le1 | TS109 | 32 | Twin | EA | DZ | Lean | >6 | 2583 |
| F32T1Le2 | TS109.2 | 32 | Twin | EA | DZ | Lean | >6 | 1682 |
| F32T2Le1 | TS110 | 32 | Twin | EA | DZ | Lean | >6 | 2286 |
| F32T2Le2 | TS110.2 | 32 | Twin | EA | DZ | Lean | >6 | 4408 |
| F32MLe1 | TS111 | 32 | Mom | EA | NA | Lean | >6 | 3822 |
| F32MLe2 | TS111.2 | 32 | Mom | EA | NA | Lean | >6 | 2597 |
| F33T1Ob1 | TS115 | 33 | Twin | AA | MZ | Obese | >6 | 2619 |
| F33T1Ob2 | TS115.2 | 33 | Twin | AA | MZ | Obese | >6 | 2017 |
| F33T2Ob1 | TS116 | 33 | Twin | AA | MZ | Obese | >6 | 5558 |
| F33T2Ob2 | TS116.2 | 33 | Twin | AA | MZ | Obese | >6 | 2440 |
| F33MOb1 | TS117 | 33 | Mom | AA | NA | Obese | >6 | 3430 |
| F33MOb2 | TS117.2 | 33 | Mom | AA | NA | Obese | >6 | 2932 |
| F34T1Ob1 | TS118 | 34 | Twin | AA | DZ | Obese | >0 | 2209 |
| F34T1Ob2 | TS118.2 | 34 | Twin | AA | DZ | Obese | >6 | 3030 |
| F34T2Ob1 | TS119 | 34 | Twin | AA | DZ | Obese | >6 | 2791 |
| F34T2Ob2 | TS119.2 | 34 | Twin | AA | DZ | Obese | >0 | 3828 |
| F34MOb1 | TS120 | 34 | Mom | AA | NA | Obese | >6 | 97 |
| F34MOb2 | TS120.2 | 34 | Mom | AA | NA | Obese | >6 | 3015 |
| F35T1Le1 | TS124 | 35 | Twin | EA | DZ | Lean | >6 | 2336 |
| F35T1Le2 | TS124.2 | 35 | Twin | EA | DZ | Lean | >6 | 2102 |
| F35T2Ov1 | TS125 | 35 | Twin | EA | DZ | Overweight | >6 | 2381 |
| F35T2Ov2 | TS125.2 | 35 | Twin | EA | DZ | Overweight | >6 | 1889 |
| F35MOb1 | TS126 | 35 | Mom | EA | NA | Obese | >6 | 1733 |
| F35MOb2 | TS126.2 | 35 | Mom | EA | NA | Obese | >6 | 2676 |
| F36T1Le1 | TS127 | 36 | Twin | EA | DZ | Lean | >6 | 4119 |
| F36T1Le2 | TS127.2 | 36 | Twin | EA | DZ | Lean | >6 | 1929 |
| F36T2Le1 | TS128 | 36 | Twin | EA | DZ | Lean | >6 | 4698 |
| F36T2Le2 | TS128.2 | 36 | Twin | EA | DZ | Lean | >6 | 2857 |
| F36MLe1 | TS129 | 36 | Mom | EA | NA | Lean | >6 | 2628 |
| F36MLe2 | TS129.2 | 36 | Mom | EA | NA | Lean | >6 | 2247 |
| F37T1Ob1 | TS130 | 37 | Twin | AA | MZ | Obese | >6 | 3121 |
| F37T1Ob2 | TS130.2 | 37 | Twin | AA | MZ | Obese | >1 | 3391 |
| F37T2Ob1 | TS131 | 37 | Twin | AA | MZ | Obese | >6 | 3338 |
| F37T2Ob2 | TS131.2 | 37 | Twin | AA | MZ | Obese | NA | 3168 |
| F37MOb1 | TS132 | 37 | Mom | AA | NA | Obese | >1 | 2586 |
| F37MOb2 | TS132.2 | 37 | Mom | AA | NA | Obese | NA | 4130 |
| F38T1Ob1 | TS133 | 38 | Twin | AA | MZ | Obese | >6 | 2355 |
| F38T1Ob2 | TS133.2 | 38 | Twin | AA | MZ | Obese | >6 | 3902 |
| F38T2Ob1 | TS134 | 38 | Twin | AA | MZ | Obese | >3 | 1378 |
| F38T2Ob2 | TS134.2 | 38 | Twin | AA | MZ | Obese | >5 | 2656 |
| F38MOb1 | TS135 | 38 | Mom | AA | NA | Obese | >6 | 3068 |
| F38MOb2 | TS135.2 | 38 | Mom | AA | NA | Obese | >6 | 2436 |
| F39T1Ov1 | TS136 | 39 | Twin | AA | DZ | Overweight | >6 | 2962 |
| F39T1Ob2 | TS136.2 | 39 | Twin | AA | DZ | Obese | >6 | 4164 |
| F39T2Ob1 | TS137 | 39 | Twin | AA | DZ | Obese | >6 | 3748 |
| F39T2Ob2 | TS137.2 | 39 | Twin | AA | DZ | Obese | >0 | 2902 |
| F39MOb1 | TS138 | 39 | Mom | AA | NA | Obese | >6 | 3289 |
| F39MOb2 | TS138.2 | 39 | Mom | AA | NA | Obese | >6 | 1369 |
| F40T1Ob1 | TS139 | 40 | Twin | AA | DZ | Obese | >6 | 2756 |
| F40T1Ob2 | TS139.2 | 40 | Twin | AA | DZ | Obese | >6 | 3195 |
| F40T2Ob1 | TS140 | 40 | Twin | AA | DZ | Obese | >6 | 2698 |
| F40T2Ob2 | TS140.2 | 40 | Twin | AA | DZ | Obese | >6 | 2851 |
| F40MOb1 | TS141 | 40 | Mom | AA | NA | Obese | >6 | 2083 |
| F40MOb2 | TS141.2 | 40 | Mom | AA | NA | Obese | >6 | 3125 |
| F41T1Ob1 | TS142 | 41 | Twin | AA | DZ | Obese | >6 | 2432 |
| F41T1Ob2 | TS142.2 | 41 | Twin | AA | DZ | Obese | >0 | 3466 |
| F41T2Ob1 | TS143 | 41 | Twin | AA | DZ | Obese | >6 | 3944 |
| F41T2Ob2 | TS143.2 | 41 | Twin | AA | DZ | Obese | >6 | 3721 |
| F41MOb1 | TS144 | 41 | Mom | AA | NA | Obese | >6 | 2804 |
| F41MOb2 | TS144.2 | 41 | Mom | AA | NA | Obese | >6 | 4354 |
| F42T1Ob1 | TS145 | 42 | Twin | AA | DZ | Obese | >0 | 2738 |
| F42T1Ob2 | TS145.2 | 42 | Twin | AA | DZ | Obese | >1 | 3633 |
| F42T2Ob1 | TS146 | 42 | Twin | AA | DZ | Obese | >0 | 3214 |
| F42T20b2 | TS146.2 | 42 | Twin | AA | DZ | Obese | >1 | 3380 |
| F42Mob1 | TS147 | 42 | Mom | AA | NA | Obese | >2 | 3513 |
| F42Mov2 | TS147.2 | 42 | Mom | AA | NA | Overweight | >4 | 4957 |
| F43T1Ob1 | TS148 | 43 | Twin | EA | MZ | Obese | >6 | 6128 |
| F43T2Ob1 | TS149 | 43 | Twin | EA | MZ | Obese | >5 | 11555 |
| F43MOb1 | TS150 | 43 | Mom | EA | NA | Obese | >6 | 8045 |
| F44T1Ob1 | TS151 | 44 | Twin | AA | DZ | Obese | >6 | 3800 |
| F44T1Ob2 | TS151.2 | 44 | Twin | AA | DZ | Obese | >6 | 3210 |
| F44T2Ob1 | TS152 | 44 | Twin | AA | DZ | Obese | >6 | 3326 |
| F44T2Ob2 | TS152.2 | 44 | Twin | AA | DZ | Obese | >6 | 2742 |
| F44Mov1 | TS153 | 44 | Mom | AA | NA | Overweight | >6 | 4118 |
| F45T1Le2 | TS154.2 | 45 | Twin | AA | MZ | Lean | >6 | 1466 |
| F45T2Le1 | TS155 | 45 | Twin | AA | MZ | Lean | >6 | 2267 |
| F45T2Le2 | TS155.2 | 45 | Twin | AA | MZ | Lean | >6 | 2361 |
| F45MOb1 | TS156 | 45 | Mom | AA | NA | Obese | >2 | 1694 |
| F45MOb2 | TS156.2 | 45 | Mom | AA | NA | Obese | >6 | 1906 |
| F46T1Ob1 | TS160 | 46 | Twin | AA | DZ | Obese | >6 | 2367 |
| F46T1Ob2 | TS160.2 | 46 | Twin | AA | DZ | Obese | >6 | 2049 |
| F46T2Ob1 | TS161 | 46 | Twin | AA | DZ | Obese | >6 | 2185 |
| F46MOb1 | TS162 | 46 | Mom | AA | NA | Obese | >6 | 3564 |
| F46MOb2 | TS162.2 | 46 | Mom | AA | NA | Obese | >6 | 4041 |
| F47T1Le1 | TS163 | 47 | Twin | AA | MZ | Lean | >2 | 1624 |
| F47T1Le2 | TS163.2 | 47 | Twin | AA | MZ | Lean | >3 | 2495 |
| F47T2Le1 | TS164 | 47 | Twin | AA | MZ | Lean | >6 | 2651 |
| F47T2Le2 | TS164.2 | 47 | Twin | AA | MZ | Lean | >6 | 3018 |
| F47MLe1 | TS165 | 47 | Mom | AA | NA | Lean | >6 | 2767 |
| F47MLe2 | TS165.2 | 47 | Mom | AA | NA | Lean | >6 | 2839 |
| F48T1Ob1 | TS166 | 48 | Twin | AA | DZ | Obese | >2 | 3628 |
| F48T1Ob2 | TS166.2 | 48 | Twin | AA | DZ | Obese | >6 | 3252 |
| F48T2Ob1 | TS167 | 48 | Twin | AA | DZ | Obese | >6 | 2822 |
| F48T2Ob2 | TS167.2 | 48 | Twin | AA | DZ | Obese | >6 | 4538 |
| F48MOb1 | TS168 | 48 | Mom | AA | NA | Obese | >6 | 2882 |
| F48MOb2 | TS168.2 | 48 | Mom | AA | NA | Obese | >6 | 4569 |
| F49T1Ob1 | TS169 | 49 | Twin | AA | DZ | Obese | >6 | 4217 |
| F49T1Ob2 | TS169.2 | 49 | Twin | AA | DZ | Obese | >6 | 3644 |
| F49T2Ob1 | TS170 | 49 | Twin | AA | DZ | Obese | >3 | 2117 |
| F49T2Ob2 | TS170.2 | 49 | Twin | AA | DZ | Obese | >6 | 2785 |
| F50T1Ob1 | TS178 | 50 | Twin | AA | DZ | Obese | >6 | 2378 |
| F50T1Ob2 | TS178.2 | 50 | Twin | AA | DZ | Obese | >6 | 2894 |
| F50T2Ob1 | TS179 | 50 | Twin | AA | DZ | Obese | >6 | 2122 |
| F50T2Ob2 | TS179.2 | 50 | Twin | AA | DZ | Obese | >6 | 3189 |
| F50MLe1 | TS180 | 50 | Mom | AA | NA | Lean | >6 | 2132 |
| F51T1Ob1 | TS181 | 51 | Twin | AA | DZ | Obese | >3 | 3455 |
| F51T1Ob2 | TS181.2 | 51 | Twin | AA | DZ | Obese | >6 | 2812 |
| F51T2Ov1 | TS182 | 51 | Twin | AA | DZ | Overweight | >6 | 7014 |
| F51T2Ob2 | TS182.2 | 51 | Twin | AA | DZ | Obese | >6 | 6903 |
| F51MOb1 | TS183 | 51 | Mom | AA | NA | Obese | >2 | 3243 |
| F51 MOb2 | TS183.2 | 51 | Mom | AA | NA | Obese | >6 | 2884 |
| F52T1Le1 | TS184 | 52 | Twin | AA | MZ | Lean | >6 | 1925 |
| F52T2Le1 | TS185 | 52 | Twin | AA | MZ | Lean | >6 | 2545 |
| F52T2Le2 | TS185.2 | 52 | Twin | AA | MZ | Lean | >2 | 2538 |
| F52MOv1 | TS186 | 52 | Mom | AA | NA | Overweight | >6 | 1735 |
| F53T1Ob1 | TS190 | 53 | Twin | AA | MZ | Obese | NA | 3165 |
| F53T2Ob1 | TS191 | 53 | Twin | AA | MZ | Obese | >6 | 2720 |
| F53MOv1 | TS192 | 53 | Mom | AA | NA | Overweight | >6 | 5067 |
| F54T1Le1 | TS193 | 54 | Twin | EA | DZ | Lean | >6 | 1799 |
| F54T1Le2 | TS193.2 | 54 | Twin | EA | DZ | Lean | >6 | 1739 |
| F54T2Le1 | TS194 | 54 | Twin | EA | DZ | Lean | >6 | 2291 |
| F54T2Le2 | TS194.2 | 54 | Twin | EA | DZ | Lean | >6 | 1612 |
| F54MLe1 | TS195 | 54 | Mom | EA | NA | Lean | >6 | 2782 |
| F54MLe2 | TS195.2 | 54 | Mom | EA | NA | Lean | >6 | 2462 |
| | | | | | | **TOTAL** | | **119519** |

| **Table 2: V6 16S rRNA gene sequencing statistics** | | | | | |
|---|---|---|---|---|---|
| **Subject ID^{a}** | **Data ID** | **Twin/Mom** | **Family** | **BMI** | **Sequences** |
| F1T1Le1 | TS1 | Twin | 1 | Lean | 25,140 |
| F1T2Le1 | TS2 | Twin | 1 | Lean | 42,186 |
| F1MOv1 | TS3 | Mom | 1 | Overweight | 17,726 |
| F2T1Le1 | TS4 | Twin | 2 | Lean | 25,705 |
| F2T2Le1 | TS5 | Twin | 2 | Lean | 26,608 |
| F2MOb1 | TS6 | Mom | 2 | Obese | 27,007 |
| F3T1Le1 | TS7 | Twin | 3 | Lean | 17,469 |
| F3T2Le1 | TS8 | Twin | 3 | Lean | 17,170 |
| F3MOv1 | TS9 | Mom | 3 | Overweight | 14,787 |
| F5T1Le1 | TS13 | Twin | 5 | Lean | 15,296 |
| F5T2Le1 | TS14 | Twin | 5 | Lean | 14,220 |
| F5MOv1 | TS15 | Mom | 5 | Overweight | 14,244 |
| F7T1Ob1 | TS19 | Twin | 7 | Obese | 43,635 |
| F7T2Ob1 | TS20 | Twin | 7 | Obese | 13,476 |
| F7MOb1 | TS21 | Mom | 7 | Obese | 23,714 |
| F9T1Le1 | TS25 | Twin | 9 | Lean | 20,491 |
| F9T2Le1 | TS26 | Twin | 9 | Lean | 27,626 |
| F9MOb1 | TS27 | Mom | 9 | Obese | 25.494 |
| F10T1Ob1 | TS28 | Twin | 10 | Obese | 20,905 |
| F10T2Ob1 | TS29 | Twin | 10 | Obese | 15,698 |
| F10MOv1 | TS30 | Mom | 10 | Overweight | 32,083 |
| F11T1Le1 | TS31 | Twin | 11 | Lean | 16,530 |
| F11T2Le1 | TS32 | Twin | 11 | Lean | 31,690 |
| F11MOv1 | TS33 | Mom | 11 | Overweight | 28,962 |
| F15T1Ob1 | TS49 | Twin | 15 | Obese | 22,201 |
| F15T2Ob1 | TS50 | Twin | 15 | Obese | 30,498 |
| F15MOb1 | TS51 | Mom | 15 | Obese | 22,691 |
| F16T1Ob1 | TS55 | Twin | 16 | Obese | 37,027 |
| F16T2Ob1 | TS56 | Twin | 16 | Obese | 31,512 |
| F16MOb1 | TS57 | Mom | 16 | Obese | 30,392 |
| F43T1Ob1 | TS148 | Twin | 43 | Obese | 26,458 |
| F43T2Ob1 | TS149 | Twin | 43 | Obese | 35,838 |
| F43MOb1 | TS150 | Mom | 43 | Obese | 23,463 |
| | | | | ***TOTAL*** | 817,942 |

| | | | | | |
|---|---|---|---|---|---|
| ^{a}ID nomenclature: Family number, Twin number or mother, and BMI category (Le=lean; Ov=overweight, Ob=obese; e.g. F1T1Le stands for family 1, twin 1, lean) | | | | | |

| **Table 3: Full-length 16S rRNA gene sequencing statistics** | | | | | |
|---|---|---|---|---|---|
| **Subject ID^{a}** | **Data ID** | **Twin/Mom** | **Family** | **BMI** | **Sequences** |
| F1T1Le1 | TS1 | Twin | 1 | Lean | 349 |
| F1T2Le1 | TS2 | Twin | 1 | Lean | 351 |
| F1MOv1 | TS3 | Mom | 1 | Overweight | 331 |
| F2T1Le1 | TS4 | Twin | 2 | Lean | 351 |
| F2T2Le1 | TS5 | Twin | 2 | Lean | 345 |
| F2MOb1 | TS6 | Mom | 2 | Obese | 348 |
| F3T1Le1 | TS7 | Twin | 3 | Lean | 237 |
| F3T2Le1 | TS8 | Twin | 3 | Lean | 354 |
| F3MOv1 | TS9 | Mom | 3 | Overweight | 357 |
| F5T1Le1 | TS13 | Twin | 5 | Lean | 337 |
| F5T2Le1 | TS14 | Twin | 5 | Lean | 350 |
| F5MOv1 | TS15 | Mom | 5 | Overweight | 338 |
| F7T1Ob1 | TS19 | Twin | 7 | Obese | 333 |
| F7T2Ob1 | TS20 | Twin | 7 | Obese | 340 |
| F7MOb1 | TS21 | Mom | 7 | Obese | 332 |
| F9T1Le1 | TS25 | Twin | 9 | Lean | 351 |
| F9T2Le1 | TS26 | Twin | 9 | Lean | 252 |
| F9MOb1 | TS27 | Mom | 9 | Obese | 343 |
| F10T1Ob1 | TS28 | Twin | 10 | Obese | 344 |
| F10T2Ob1 | TS29 | Twin | 10 | Obese | 337 |
| F10MOv1 | TS30 | Mom | 10 | Overweight | 261 |
| F15T1Ob1 | TS49 | Twin | 15 | Obese | 338 |
| F15T2Ob1 | TS50 | Twin | 15 | Obese | 319 |
| F15MOb1 | TS51 | Mom | 15 | Obese | 331 |
| F16T1Ob1 | TS55 | Twin | 16 | Obese | 353 |
| F16T2Ob1 | TS56 | Twin | 16 | Obese | 278 |
| F16MOb1 | TS57 | Mom | 16 | Obese | 348 |
| F43T1Ob1 | TS148 | Twin | 43 | Obese | 323 |
| F43T2Ob1 | TS149 | Twin | 43 | Obese | 340 |
| F43MOb1 | TS150 | Mom | 43 | Obese | 349 |
| | | | | ***TOTAL*** | 9,920 |

| | | | | | |
|---|---|---|---|---|---|
| ^{a}ID nomenclature: Family number, Twin number or mother, and BMI category (Le=lean; Ov=overweight, Ob=obese; e.g. F1T1LE stands for family 1, twin 1, lean) | | | | | |

| **Table 4: Phytotypes shared across ≥ 70% of all individuals (V2/3 dataset: 1,000 random sequences/individual)^{a}** | | | | | | | |
|---|---|---|---|---|---|---|---|
| Phylo -type ID | Individuals with phylotype | % of individuals with phylotype | Number of reads grouped into phylotype | Highest relative abundance across all individuals | Lowest relative abundance across all individuals | Mean ± sem% of 16S rRNA gene sequences across all individuals | Taxonomic classification^{b} |
| 1 | 151 | 98.1 | 7942 | 28.7 | 0 | 6.53 ± 0.41 | Bacteria; Fimircutes; Clostridia; Faecalibacterium |
| 2 | 151 | 98.1 | 5375 | 25.5 | 0 | 4.41 ± 0.34 | Bacteria; Firmicutes; Clostridia; Clostridiales; Ruminococcus |
| 3 | 144 | 93.5 | 2518 | 14.7 | 0 | 2.06 ± 0.16 | Bacteria; Firmicutes; Clostridia; Clostridiales |
| 4 | 143 | 92.9 | 5606 | 30.5 | 0 | 4.56 ± 0.41 | Bacteria; Firmicutes; Clostridia; Clostridiales; Eubacterium rectale |
| 5 | 140 | 90.9 | 1629 | 8.1 | 0 | 1.34 ± 0.11 | Bacteria; Firmicutes; Clostridia; Clostridiales; Clostridium Clostridioforme |
| 6 | 134 | 87.0 | 757 | 12.7 | 0 | 0.62 ± 0.09 | Bacteria; Firmicutes; Clostridia; Clostridiales; Ruminococcus; Rum inococcus schinkii |
| 7 | 133 | 86.4 | 1485 | 12.2 | 0 | 1.23 ± 0.14 | Bacteria; Firmicutes; Clostridia; Clostridiales; Coprococcus |
| 8 | 133 | 86.4 | 1392 | 6.5 | 0 | 1.14 ± 0.10 | Bacteria; Firmicutes; Clostridia; Clostridiales |
| 9 | 133 | 86.4 | 1201 | 10.5 | 0 | 0.99 ± 0.12 | Bacteria; Firmicutes; Clostridia; Clostridiales; Rum inococcus |
| 10 | 128 | 83.1 | 819 | 5.2 | 0 | 0.68 ± 0.06 | Bacteria; Firmicutes; Clostridia; Clostridiales |
| 11 | 127 | 82.5 | 747 | 3.7 | 0 | 0.62 ± 0.05 | Bacteria; Fimircutes; Clostridia; Faecalibacterium |
| 12 | 126 | 81.8 | 11598 | 51.6 | 0 | 9.39 ± 0.79 | Bacteria; Bacteroidetes; Bacteroidales; Bacteroidaceae |
| 13 | 125 | 81.2 | 2585 | 34.3 | 0 | 2.15 ± 0.31 | Bacteria; Fimircutes; Clostridia; Faecalibacterium |
| 14 | 123 | 79.9 | 3512 | 15.3 | 0 | 2.89 ± 0.25 | Bacteria; Fimircutes; Clostridia; Faecalibacterium |
| 15 | 120 | 77.9 | 792 | 8.4 | 0 | 0.66 ± 0.08 | Bacteria; Firmicutes; Clostridia; Clostridiales; Clostridium nexile |
| 16 | 118 | 76.6 | 632 | 2.7 | 0 | 0.52 ± 0.05 | Bacteria; Fimircutes; Clostridia; Faecalibacterium |
| 17 | 115 | 74.7 | 3422 | 43.3 | 0 | 2.79 ± 0.41 | Bacteria; Bacteroidetes; Bacteroidales; Bacteroidaceae |
| 18 | 113 | 73.4 | 441 | 2.3 | 0 | 0.37 ± 0.03 | Bacteria; Firmicutes; Clostridia; Clostridiales; Clostridium nexile |
| 19 | 112 | 72.7 | 1168 | 17.4 | 0 | 0.98 ± 0.16 | Bacteria; Firmicutes; Clostridia; Clostridiales; Rum inococcus |
| 20 | 111 | 72.1 | 749 | 5.2 | 0 | 0.61 ± 0.07 | Bacteria; Firmicutes; Clostridia; Clostridiales |
| 21 | 108 | 70.1 | 640 | 3.5 | 0 | 0.53 ± 0.06 | Bacteria; Firmicutes; Clostridia; Clostridiales; Ruminococcus |

| | | | | | | | |
|---|---|---|---|---|---|---|---|
| ^{a} 1,000 sequences were randomly sampled from a single timepoint for each individual ^{b} Based on the consensus taxonomy of ≥ 90% sequences within each phylotype (best-BLAST-hit against the Greengenes database) | | | | | | | |

| **Table 5: Phylotypes shared across >90% of all individuals (V6 dataset: 10,000 random sequences/individual)** | | | | | | | |
|---|---|---|---|---|---|---|---|
| Phylo -type ID | Individuals with phylotype | % of individuals with phylotype | Number of reads grouped into phylotype | Highest relative abundance across all individuals | Lowest relative abundance across all individuals | Mean ± sem% of 16S rRNA gene sequences across all individuals | Taxonomic classification^{a} |
| 1 | 33 | 100.0 | 10400 | 9.7 | 0.011 | 3.40 ± 0.45 | Bacteria; Firmicutes; Clostridia; Clostridiales; Clostridium nexile |
| 2 | 33 | 100.0 | 5161 | 5.9 | 0.011 | 1.67 ± 0.23 | Bacteria; Firmicutes; Clostridiales; Clostridium nexile; Clostridium fusiformis |
| 3 | 33 | 100.0 | 6077 | 6.7 | 0.021 | 1.97 ± 0.32 | Bacteria; Firmicutes; Clostridia; Clostridiales; Ruminococcus |
| 4 | 33 | 100.0 | 16600 | 26.8 | 0.011 | 5.36 ± 1.02 | Bacteria; Firmicutes; Clostridia; Clostridiales; Eubacterium rectale |
| 5 | 33 | 100.0 | 11654 | 12.5 | 0.011 | 3.78 ± 0.58 | Bacteria; Firmicutes; Clostridia; Clostridiales; Rum inococcus |
| 6 | 32 | 97.0 | 3113 | 5.8 | 0.000 | 1.01 ± 0.23 | Bacteria; Firmicutes; Clostridia; Clostridiales; Clostridium nexile |
| 7 | 32 | 97.0 | 2908 | 4.2 | 0.000 | 0.96 ± 0.21 | Bacteria; Bacteroidetes; Bacteroidales; Bacteroidaceae |
| 8 | 32 | 97.0 | 2382 | 3.7 | 0.000 | 0.78 ± 0.13 | Bacteria; Firmicutes; Clostridia; Clostridiales; Rum inococcus |
| 9 | 32 | 97.0 | 1712 | 4.4 | 0.000 | 0.56 ± 0.14 | Bacteria; Firmicutes; Clostridia; Clostridiales; Rum inococcus; Rum inococcus schinkii |
| 10 | 31 | 93.9 | 3940 | 6.6 | 0.000 | 1.29 ± 0.26 | Bacteria; Fimircutes; Clostridia: Faecalibacterium |
| 11 | 31 | 93.9 | 3729 | 4.9 | 0.000 | 1.21 ± 0.18 | Bacteria; Firmicutes; Clostridia; Clostridiales; Clostridium nexile |
| 12 | 30 | 90.9 | 454 | 0.7 | 0.000 | 0.15 ± 0.03 | Bacteria; Firmicutes; Clostridia; Clostridiales; Ruminococcus |
| 13 | 30 | 90.9 | 687 | 1.1 | 0.000 | 0.23 ± 0.04 | Bacteria; Firmicutes; Clostridia |
| 14 | 30 | 90.9 | 999 | 2.3 | 0.000 | 0.33 ± 0.08 | Bacteria; Firmicutes; Clostridia; Preptostreptococace ae; Peptostreptococcus anaerobius; Clostridium bifermentans |
| 15 | 30 | 90.9 | 1241 | 5.3 | 0.000 | 0.40 ± 0.16 | Bacteria; Firmicutes; Clostridia; Clostridiales; Clostridium bolteae |
| 16 | 30 | 90.9 | 160 | 0.2 | 0.000 | 0.05 ± 0.01 | Bacteria; Actinobacteria; Actinobacteridae; Actinomycineae |
| 17 | 30 | 90.9 | 1417 | 2.0 | 0.000 | 0.46 ± 0.09 | Bacteria; Firmicutes; Clostridia; Clostridiales; Rum inococcus |
| 18 | 30 | 90.9 | 1014 | 1.2 | 0.000 | 0.33 ± 0.06 | Bacteria; Firmicutes; Clostridia; Clostridiales |
| 19 | 30 | 90.9 | 1353 | 1.6 | 0.000 | 0.44 ± 0.08 | Bacteria; Firmicutes; Clostridia; Clostridiales; Ruminococcus; Ruminococcus luti |
| 20 | 30 | 90.9 | 2686 | 6.0 | 0.000 | 0.88 ± 0.22 | Bacteria; Firmicutes; Clostridia; Clostridiales; Clostridium clostridioforme |
| 21 | 30 | 90.9 | 7454 | 12.2 | 0.000 | 2.43 ± 0.63 | Bacteria; Fimircutes; Clostridia; Faecalibacterium |

| | | | | | | | |
|---|---|---|---|---|---|---|---|
| ^{a}Based on the consensus taxonomy of >90% sequences within each phylotype (best-BLAST-hit against the Greengenes database) | | | | | | | |

| **Table 6: Phylotypes shared across ≥70% of all individiuals (Full-length dataset; 200 random sequences/individual)** | | | | | | | |
|---|---|---|---|---|---|---|---|
| **Phylotype ID** | **Individuals with phylotype** | **% of individuals with phylotype** | **Number of reads grouped into phylotype** | **Highest relative abundance across all individuals** | **Lowest relative abundance across all individuals** | **Mean±sem % of 16S rRNA gene sequences across all individuals** | **Taxonomic Classifications^{a}** |
| 1 | 28 | 93.3 | 378 | 17.9 | 0.0 | 7.81 ± 1.04 | Bacteria; Firmicutes; Clostridia; Faecalibacterium |
| 2 | 27 | 90.0 | 347 | 25.0 | 0.0 | 6.90 ± 1.20 | Bacteria; Firmicutes; Clostridia; Clostridiales; Rum inococcus |
| 3 | 26 | 86.7 | 128 | 9.9 | 0.0 | 2.62 ± 0.47 | Bacteria; Firmicutes; Clostridia; Clostridiales; |
| 4 | 26 | 86.7 | 298 | 23.1 | 0.0 | 6.00 ± 1.14 | Bacteria; Firmicutes; Clostridia; Clostridiales; Eubacterium rectale |
| 5 | 26 | 86.7 | 127 | 12.0 | 0.0 | 2.64 ± 0.49 | Bacteria; Firmicutes; Clostridia; Clostridiales; Clostridium clostridioforme |
| 6 | 22 | 73.3 | 110 | 10.9 | 0.0 | 2.33 ± 0.55 | Bacteria; Bacteroidetes; Bacteroidales; Bacteroidaceae |
| 7 | 22 | 73.3 | 87 | 5.7 | 0.0 | 1.76 ± 0.29 | Bacteria; Firmicutes; Clostridia; Clostridiales; Clostridium nexile; Clostridium fusiformis |
| 8 | 21 | 70.0 | 112 | 11.9 | 0.0 | 2.32 ± 0.49 | Bacteria; Firmicutes; Clostridia; Clostridiales; Coprococcus |
| 9 | 21 | 70.0 | 75 | 6.9 | 0.0 | 1.53 ± 0.32 | Bacteria; Firmicutes; Clostridia; Clostridiales; Clostridium nexile |
| 10 | 21 | 70.0 | 54 | 5.7 | 0.0 | 1.14 ± 0.23 | Bacteria; Firmicutes; Clostridia; Clostridiales; Clostridium nexile |

| | | | | | | | |
|---|---|---|---|---|---|---|---|
| ^{a}Based on the consensus taxonomy of >90% sequences within each phylotype (best-BLAST-hit against the Greengenes database) | | | | | | | |

### Sample characteristics

Twin pairs who had been enrolled in the Missouri Adolescent Female Twin Study (MOAFTS) were recruited for this study (mean period of enrollment, 11.7±1.2 years; range, 4.4-13.0 years). The MOAFTS twin cohort, comprised of female like-sex twin pairs, was identified from Missouri birth records over the period 1994-1999, when the twins were median age 15. A total of 350 twins from the larger MOAFTS cohort completed screening interviews for the present study. Pairs most likely to meet study criteria were identified at the wave five interview of the MOAFTS twin cohort (which has 90% retention of wave four participants). Eligibility was then confirmed at screening interview. All twins were 25-32 years old, of European or African ancestry (EA and AA, respectively), were generally concordant for obesity (BMI>30 kg/m²) or leanness (BMI=18.5-24.9 kg/m²) [1 twin pair was lean/overweight (overweight defined as BMI ≥25 and <30) and 6 pairs were overweight/obese], and had not taken antibiotics for at least 5.49±0.09 months. Each participant completed a detailed medical, lifestyle, and dietary questionnaire. Participants were broadly representative of the overall Missouri population with respect to BMI, parity, education, and marital status. Although all were born in Missouri, they currently live throughout the USA: 29% live in the same house, but some live >800 km apart. Since fecal samples are readily attainable and representative of interpersonal differences in gut microbial ecology, they were collected from each individual and frozen immediately. The collection procedure was repeated again with an average interval between sample collections of 57±4 days.

### Community DNA Preparation

Frozen de-identified fecal samples were stored at -80°C before processing. In order to homogenize each sample, a 10-20 g aliquot of each sample was pulverized in liquid nitrogen with a mortar and pestle. An aliquot (~500mg) of each sample was then suspended, while frozen, in a solution containing 500 µl of extraction buffer [200 mM Tris (pH 8.0), 200 mM NaCl, 20 mM EDTA], 210 µl of 20% SDS, 500 µl of a mixture of phenol:chloroform:isoamyl alcohol (25:24:1, pH 7.9), and 500 µl of a slurry of 0.1 mm-diameter zirconia/silica beads (BioSpec Products, Bartlesville, OK). Microbial cells were subsequently lysed by mechanical disruption with a bead beater (BioSpec Products) set on high for 2 min at room temperature, followed by extraction with phenol:chloroform:isoamyl alcohol, and precipitation with isopropanol. DNA obtained from three separate 10 mg frozen aliquots of each fecal sample were pooled (≥200µg DNA) and used for pyrosequencing (see below).

### Full-length 16S rRNA sequence-based surveys

Five replicate PCR reactions were performed for each fecal DNA sample. To generate full length or near full length bacterial 16S rRNA amplicons, each 25 µl reaction contained 100 ng of gel purified DNA (Qiaquick, Qiagen), 10 mM Tris (pH 8.3), 50 mM KCI, 2 mM MgSO4, 0.16 µM dNTPs, 0.4 µM of the bacteria-specific primer 8F (5'-AGAGTTTGATCCTGGCTCAG-3'), 0.4 µM of the universal primer 1391 R (5'-GACGGGCGGTGWGTRCA-3'), 0.4 M betaine, and 3 units of Taq polymerase (Invitrogen). Cycling conditions were 94°C for 2 min, followed by 25 cycles of 94°C for 1 min, 55°C for 45 sec, and 72°C for 2 min. Replicate PCRs were pooled and concentrated (Millipore; Montage PCR filter columns). Full-length 16S rRNA gene amplicons (1.3kb) were then gel-purified using the Qiaquick kit (Qiagen), subcloned into TOPO TA pCR4.0 (Invitrogen), and the ligated DNA transformed into E. coli TOP10 (Invitrogen). For each sample, 384 colonies containing cloned 16S rRNA nucleic acid amplicons were processed for sequencing. Plasmid inserts were sequenced bidirectionally using vector-specific primers plus the internal primer 907R (5'-CCGTCAATTCCTTTRAGTTT-3').

16S rRNA gene sequences were edited and assembled into consensus sequences using the PHRED and PHRAP software packages within the Xplorseq program. Sequences that did not assemble were discarded and bases with PHRED quality scores <20 were trimmed. Sequences were checked for chimeras using Bellerophon program version 3 with the default parameters (final dataset n=8,941 near full-length 16S rRNA gene sequences; for sequence designations see **Table 1**). Alignments for reference genome 16S rRNA gene sequences were manually edited in ARB.

### V2/3 16S rRNA sequence-based surveys

Four replicate PCR reactions targeting the V2/3 region of bacterial 16S rRNA genes were performed on the same fecal DNA samples used above. Each 20 µl reaction contained 100 ng of gel purified DNA (Qiaquick, Qiagen), 8 µl 2.5X HotMaster PCR Mix (Eppendorf), 0.3 µM of the primer 8F [5'-GCCTTGCCAGCCCGCTCAG-**TC***AGAGTTTGATCCTGGCTCAG*-3'; composite of 454 primer B (underlined), linker nucleotides (TC), and the universal bacterial primer 8F (italics)], and 0.3 µM of the primer 338R [5'-GCCTCCCTCGCGCCATCAGNNNNNNNN**CA**-*TGCTGCCTCCCGTAGGAGT*-3'; 454 Life Sciences primer A (underlined), a unique 8 base barcode (Ns), linker nucleotides (CA), and the broad-range bacterial primer 338R (italics)]. Cycling conditions were 95°C for 2 min, followed by 30 cycles of 95°C for 20 sec, 52°C for 20 sec, and 65°C for 1 min. Replicate PCRs were pooled and purified with Ampure magnetic purification beads (Agencourt).

PCR products were quantified with the bisbenzimide H assay. An aliquot of each PCR product was incubated for 5 min at room temperature in TNE reagent [10 mM Trizma HCl pH 8.1, 100mM NaCl, 1 mM EDTA, and 50 ng/ml freshly prepared bisbenzimide H (Sigma)]. Samples were read on a flurometer or plate reader (excitation at 365nm, emission at 460nm) relative to a standard curve constructed using E. coli DNA (Sigma). Multiple pools, each containing approximately equimolar amounts of PCR products, were assembled for 454 FLX amplicon pyrosequencing (n=33-100 barcoded samples/pool). Technical replicates were analyzed from selected representatives of each pool across four different sequencing centers; results were highly reproducible, discriminating between individuals and between samples from the same individual over time (**FIG. 1**).

### V6 16S rRNA sequence-based surveys

PCR reactions targeting the V6 region of bacterial 16S rRNA genes were performed on the same fecal DNA samples used above. Each 32 µl reaction contained 100 ng of gel purified DNA (Qiaquick, Qiagen), PCR buffer (PurePeak DNA polymerization mix, Thermo-Fisher), 0.625 mM PurePeak dNTPs (Thermo-Scientific), 0.625 µM Fusion Primer A, 0.625 µM Fusion Primer B, and 5U Pfu polymerase (Stratagene). The primer set included 5 forward primers (Fusion A) and 4 reverse primers (Fusion B) fused to the 454 Life Sciences adaptors A and B respectively. Cycling conditions were 94°C for 3 min, followed by 30 cycles of 94°C for 30 sec, 57°C for 45 sec, and 72°C for 1 min, with a final extension period of 72°C for 2 min. PCR products were purified with MinElute columns (Qiagen), and DNA was quantified using a Bioanalyzer (Agilent) and the PicoGreen assay (Invitrogen). Two pools of PCR products were constructed for 454 FLX amplicon pyrosequencing, composed of 18 and 20 samples, respectively (the second run contained 3 samples from the V2/3 region and 3 technical replicates, one additional sample (TS30) was sequenced in a third run, bringing the total number of V6 samples processed to 33). Since technical replicates were highly reproducible (see above and **FIG. 5**), datasets for a given individual's biospecimen were pooled for all subsequent analyses. Any sequences that did not have an exact match to the proximal primer or that contained one or more ambiguous bases were removed as low quality. The proximal primer and any fuzzy matches (identified with BLAST and the fuzznuc program) to the distal primer were then trimmed from the sequences. Finally, any trimmed sequences shorter than 50 nucleotides were also removed as low quality.

### Picking Operational Taxonomic Units (OTUs)

Pyrosequencing data was pre-processed to remove sequences with low quality scores, sequences with ambiguous characters, or sequences outside of the length bounds (V6 < 50nt, V2/3 < 200nt) and binned according to sample based on the error-correcting barcodes. Similar sequences were identified using the Megablast software and the following parameters: E-value 1⁻¹⁰; minimum coverage, 99%; and minimum pairwise identity, 97%. Candidate OTUs were identified as sets of sequences connected to each other at this level using the top 4000 hits per sequence. Each candidate OTU was considered valid if the average density of connection was above threshold; otherwise it was broken up into smaller connected components.

### Tree building and UniFrac clustering for PCA analysis

A relaxed neighbor-joining tree was built from one representative sequence per OTU using Clearcut, employing the Kimura correction (the PH lanemask was applied to V2/3 data), but otherwise with default comparisons. Unweighted UniFrac was run using the resulting tree and the counts of each sequence in each sample. Priniciple component analysis (PCA) was performed on the resulting matrix of distances between each pair of samples. To determine if the UniFrac distances were on average significantly different for pairs of samples (i.e. between twin-pairs, between twins and their mother, or between unrelated individuals), a t-test was performed on the UniFrac distance matrix, and a p-value was generated for the t-statistic by permutation of the rows and columns as in the Mantel test, regenerating the t-statistic for 1000 random samples, and using the distribution to obtain an empirical p-value.

### Taxonomy assignment

Taxonomy was assigned using the best-BLAST-hit against Greengenes (E-value cutoff of 1e⁻¹⁰, minimum 88% coverage, 88% percent identity) and the Hugenholtz taxomony, downloaded May 12, 2008, excluding sequences annotated as chimeric (http://greengenes.lbl.gov/Download/Sequence_Data/Greengenes_format/).

### Rarefaction and phylogenetic diversity measurements

To determine which individuals had the most diverse communities of gut bacteria, rarefaction plots and Phylogenetic Diversity (PD) measurements, as described by Faith (Biological Conservation 1992), were made for each sample. PD is the total amount of branch length in a phylogenetic tree constructed from the combined 16S rRNA dataset, leading to the sequences in a given sample. To account for differences in sampling effort between individuals, and to estimate the thoroughness of sampling of each individual, the accumulation of PD (branch length) with sampling effort was plotted in a manner analogous to rarefaction curves. The PD rarefaction curve for each individual was generated by applying custom python code that can be downloaded from http://bayes.colorado.edu/unifrac, to the Arb parsimony insertion tree.

### Results

To characterize the bacterial lineages present in the fecal microbiotas of these 44 individuals, 16S rRNA sequencing was performed, targeting the full-length gene with an ABI 3730xl capillary sequencer. Additionally, multiplex sequencing with a 454 FLX pyrosequencer was used to survey the V2/3 variable region and the V6 hypervariable region (**Tables 1, 2** and **3**). Complementary phylogenetic and taxon-based methods were used to compare 16S rRNA sequences among fecal communities. Phylogenetic clustering with UniFrac is based on the principle that communities can be compared in terms of their shared evolutionary history, as measured by the degree to which they share branch length on a phylogenetic tree. This approach was complemented with taxon-based methods; these methods disregard some of the information contained in the phylogenetic tree of the taxa in question, but have the advantage that specific taxa unique to, or shared among, groups of samples can be identified (e.g., those from lean or obese individuals). Prior to both types of analyses, 16S rRNA gene sequences were grouped into Operational Taxonomic Units (OTUs/phylotypes) using the furthest-neighbor-like algorithm and a sequence identity threshold of 97%, which is commonly used to define 'species'-level phylotypes. Taxonomic assignments were made using BLAST and Hugenholtz taxonomy annotations in the Greengenes database.

No matter which region of the 16S rRNA gene was examined (V2/3 or V6 pyrosequencing reads, or the near-complete gene from Sanger reads), individuals from the same family (a twin and her co-twin, or twins and their mother) had a more similar bacterial community structure than unrelated individuals (**FIGS. 2A** and **3A****, B**) and shared significantly more phylotypes [G=55.2, p<10⁻¹² (V2/3); G=112.3, p<0.001 (V6); G=11.3, p<0.001 (full-length)]. No significant correlation was seen between the degree of physical separation of family members' current homes and the degree of similarity between their microbial communities (defined by UniFrac). The observed familial similarity was not due to an indirect effect of the physiologic states of obesity versus leanness; similar results were observed after stratifying twin-pairs and their mothers by BMI category (concordant lean or concordant obese individuals; **FIG. 4**). Surprisingly, there was no significant difference in the degree of similarity in the gut microbiotas of adult MZ versus DZ twin-pairs (**FIG. 2A**). However, in the present study it was not assessed whether MZ and DZ twin pairs had different degrees of similarities at earlier stages of their lives.

Multiplex pyrosequencing of V2/3 and V6 amplicons allowed higher levels of coverage of community diversity compared to what was feasible using Sanger sequencing, reaching on average 3,984±232 (V2/3) and 24,786±1,403 (V6) sequences per sample. To control for differences in coverage between samples, all analyses were performed on an equal number of randomly selected sequences [200 full-length, 1,000 V2/3, and 10,000 V6]. At this level of coverage, there was little overlap between the sampled fecal communities: only 2, 5, and 21 phylotypes were found in >90% of the individuals surveyed (full-length, V2/3, and V6 data respectively). Moreover, the number of 16S rRNA gene sequences belonging to these phylotypes varied greatly between fecal microbiotas (**Tables 4, 5** and **6**).

Samples taken from the same individual at the initial collection point and 57±4 days later were remarkably consistent with respect to the specific phylotypes found (**FIGS. 1** and **5**), but showed variations in the relative abundance of the major gut bacterial phyla (**FIG. 6**). There was no significant association between UniFrac distance and the time between sample collections. Overall, fecal samples from the same individual were much more similar to one another than samples from family members or unrelated individuals (**FIG. 2A**), demonstrating that short-term temporal changes in community structure within an individual are minor compared to inter-personal differences.

After assigning V2/3, V6 and full-length 16S rRNA gene sequences to bacterial taxa (see Example 3 below), it was found that obese individuals generally had a lower relative abundance of the Bacteroidetes and a higher relative abundance of the Firmicutes and Actinobacteria: the statistical significance of these observations varied depending upon the sequencing methods used (**Table 7**), likely due to differences in PCR conditions (for example, the 8F primer has a known bias against Actinobacteria).

In summary, across all methods, obesity was associated with a significant decrease in the level of diversity (**FIG. 2B** and **FIGS. 3C-F**). This reduced diversity suggests an analogy: the obese gut microbiota is not like a rainforest or reef, which are adapted to high energy flux and are highly diverse, but rather may be more like a fertilizer runoff where a reduced diversity microbial community blooms with abnormal energy input.

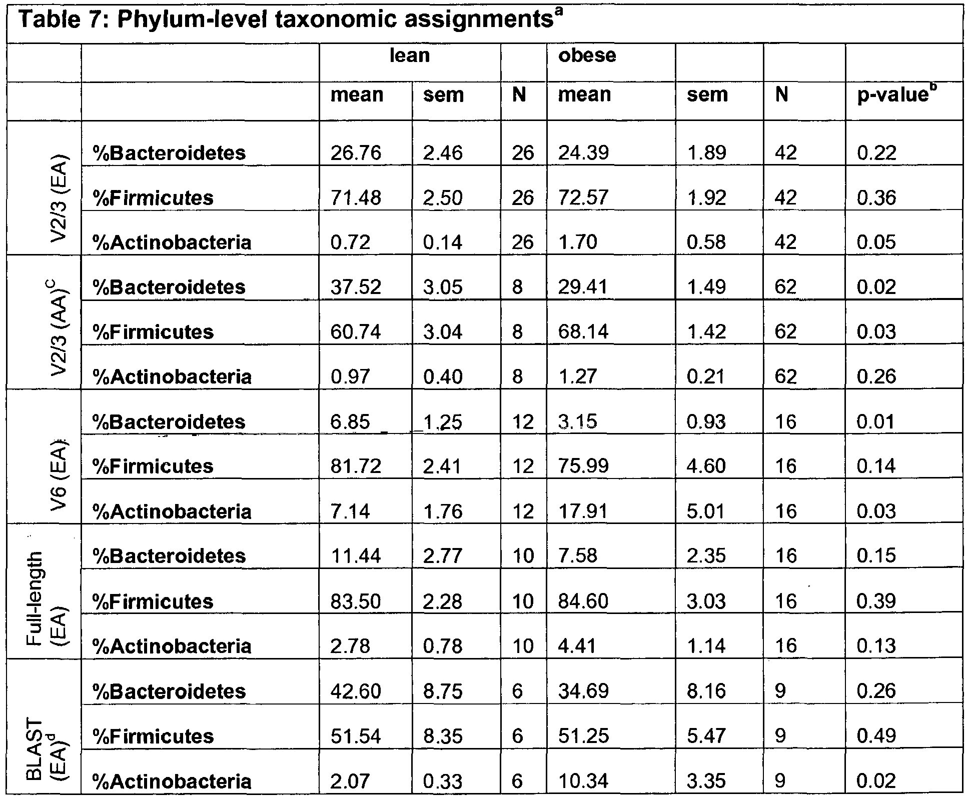

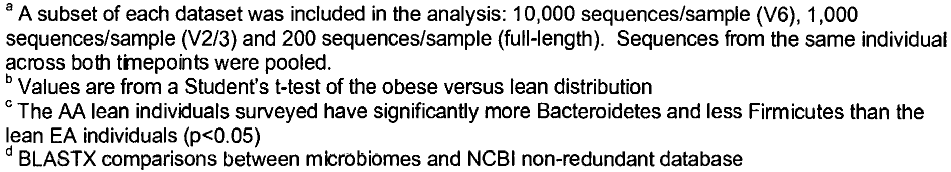

### Example 2: Distribution of phylotypes in individuals

All hosts were searched for bacterial phylotypes present at high abundance using a sampling model based on a combination of standard Poisson and binomial sampling statistics.

### Phylotype sampling model

A sampling model was developed that allows placement of bounds on the maximum abundance of any phylotype found across all samples. The principle here is that if a given phylotype made up not less than some proportion p of the microbiome of all humans, it is then possible to calculate (i) the number of samples of a given size expected to lack that phylotype due to sampling error, and (ii) the probability that an actual proportion p-hat as low as the minimum abundance would be observed in any sample.

The probability P of failing to observe a given microbe at proportion p in a sample of size n is given by Poisson statistics as simply *e^{-pn}*. For equal sample sizes, the probability of observing the phylotype in at least *k* samples using binomial sampling with Pr(success) = (1-P) can therefore be calculated. Then, the inverse binomial can be used to ask what value of P, and therefore of *p*, gives a specified probability (say, 5%) of observing a given phylotype in as few samples as actually observed for the most abundant phylotype. This calculation yields an upper bound for p (i.e. the value of p at which we can reject the idea that we would have seen the phylotype in as few samples as actually observed at the 95% confidence level).

For unequal sizes, there is no analytical solution to the equivalent of the binomial in which Pr(success) differs for each trial. Therefore, numerical optimization must be used to solve for *p*. Because the function relating *p* and the probability of observing the phylotype in at least a given number of samples is monotonic, a bisection search (bounded by *p*=0 and *p*=1) can be used to find the appropriate value of *p* for a desired confidence level. In practice, *P* was calculated for each sample, a vector of random numbers between 0 and 1 was chosen, and the number of times the random number at a given position was less than *P* was counted. Repeating this procedure for a fixed number of iterations (100,000 for the reported values) gives sufficiently smooth values to approximate the monotonic function and to allow the bisection search to converge on the same value of *p* to three significant figures across repeated trials.

In the case where a phylotype was found in all samples, a similar procedure could be used to identify the maximum value of p consistent with the observed minimum abundance of the phylotype whose minimum abundance across all samples is highest. In this case, instead of calculating the fraction of samples in which the phylotype was absent, (i) binomial sampling could be used to randomly sample the number of observed counts of a phylotype given the parametric value of *p* and the sample size of each sample, (ii) the minimum abundance across all samples could be measured, and (iii) this minimum abundance compared to the minimum abundance actually observed. Again, an analytical solution using extreme-value statistics is possible if sample sizes are equal, but the solution must be obtained by numerical methods (in this case, the same type of bisection search used above). The sampling model was implemented in Python using PyCogent.

### Results

Using this model the full-length 16S rRNA dataset described in Example 1 was first analyzed. The most abundant 'species'-level phylotype in each sample made up 11 % of that sample on average (range: 4.2%-22.0%), and the most abundant phylotype found across the combined dataset was found in 25 of the 27 fecal microbiotas (taxonomy assignment = Bacteria; Firmicutes; Clostridia; Clostridiales; Ruminococcus). These data are consistent with no phylotype being present at more than 1.3% abundance in all samples.

The deeper pyrosequencing data confirmed this result. In the V6 dataset, using even sampling of 10,000 sequences/sample, the most abundant phylotype in each sample made up 12% of that sample on average (range: 5.0%-36.6%). The overall most abundant phylotype was found in all 33 samples (Bacteria; Firmicutes; Clostridia; Clostridiales; Eubacterium rectale). However, in some samples, this phylotype was present in frequencies as low as 0.01 %.

The sampling model allows one to ask what level of abundance in every individual the most abundant phylotype could have before its absence from, or limited representation in some samples becomes surprising. For example, with 1,000 sequences/samples, it would be very surprising if a species at 50% abundance across all samples in any out of 30 samples was missed, but it would not be surprising if a species at 0.00001 % abundance were missed.

The sampling model (using 1000 random sequences per sample) indicated that this minimum observed abundance was consistent with a 'true abundance' of no more than 0.66%. In the V2/3 dataset, the most abundant phylotype in each sample made up 14.6% of that sample on average (range: 3.8%-47.1%). The overall most abundant phylotype was present in 270 of 274 samples at this depth of coverage (Bacteria;Bacteroidetes;Bacteroidales; Bacteroidaceae). The sampling model indicated that this frequency was consistent with a true abundance of no more than 0.53%. These results were confirmed, with excellent agreement, by the V6 data: at 1,000 sequences/sample, the maximum abundance OTU is found in 32 of 33 samples, consistent with an abundance of no more than 0.66%. However, at a coverage depth of 10,000 sequences/sample, this OTU is found in all 33 samples but at a minimum observed abundance of 0.02%, consistent with a true abundance of no more than 0.1%. Using all the V6 data without controlling for sampling effort, the minimum observed abundance is consistent with a true abundance of no more than 0.07% (the estimate of the true abundance falls with increased sample size because it is less likely that the low frequency would be observed due to sampling error when more total sequences contribute to the result). Thus, we conclude, with 95% confidence, based on the even sampling used for the other analyses in this study (i.e., 1,000 sequences/sample from V2/3, 10,000 sequences/sample for V6) that the maximum abundance of any OTU across all samples cannot exceed the V2/3 result of 0.53%, although the true maximum abundance might be as much as an order of magnitude lower than this based on the greater depth of coverage in the V6 samples.

In summary, the analysis showed that no phylotype is present at more than ~0.5% abundance in all of the samples in this study, and that although individual microbiotas are dominated by a few abundant phylotypes, these groups vary dramatically in their proportional representation in the sampled gut communities. Also, no phylotypes were detectable in all individuals sampled within this range of coverage (**FIG. 7**).

### Example 3: Taxonomic assignments of metagenomic reads

The International Human Microbiome Project has emphasized the importance of sequencing the genomes of a panel of reference microbial strains. Therefore, shotgun pyrosequencing was used to sample the fecal microbiomes of 18 individuals representing 6 of the families described in Example 1.

### Pyrosequencing of total community DNA

Shotgun sequencing runs were performed on the 454 FLX pyrosequencer from total community DNA of 3 lean European American MZ twin-pairs and their mothers plus 3 obese European American MZ twin pairs and their mothers, yielding 8,294,835 reads and 14,730 16S rRNA fragments. Two samples were also analyzed on a single run employing 454/Roche GS FLX Titanium extra long read sequencing technology (**Tables 8** and **9**). Sequencing reads with degenerate bases ("Ns") were removed along with all duplicate sequences, as sequences of identical length and content are a common artifact of the pyrosequencing methodology. Finally, human sequences were removed by identifying sequences homologous to the H. *sapiens* reference genome (BLASTN e-value<10-5, %identity>75, and score>50).

| **Table 8: Microbiome sequencing statistics** | | | | | | | | | |
|---|---|---|---|---|---|---|---|---|---|
| Subject ID^{a} | Data ID | Twin/ Mom | Family | BMI | Platform | Total nt | Number Reads | Filtered Reads^{b} | 16S rRNA gene fragments ^{c} |
| F1T1Le1 | TS1 | Twin | 1 | Lean | FLX | 60,016,519 | 254,044 | 217,386 | 439 |
| F1T2Le1 | TS2 | Twin | 1 | Lean | FLX | 90,271,969 | 514,022 | 443,640 | 512 |
| F1MOv1 | TS3 | Mom | 1 | Overweight | FLX | 113,506,401 | 571,301 | 510,972 | 723 |
| F2T1Le1 | TS4 | Twin | 2 | Lean | FLX | 107,008,761 | 472,154 | 414,754 | 626 |
| F2T2Le1 | TS5 | Twin | 2 | Lean | FLX | 112,835,879 | 553,142 | 490,776 | 928 |
| F2MOb1 | TS6 | Mom | 2 | Obese | FLX | 135,976,476 | 623,027 | 535,763 | 1,039 |
| F3T1Le1 | TS7 | Twin | 3 | Lean | FLX | 146,946,832 | 607,386 | 555,853 | 1,188 |
| F3T2Le1 | TS8 | Twin | 3 | Lean | FLX | 113,177,766 | 468,769 | 414,497 | 976 |
| F3MOv1 | TS9 | Mom | 3 | Overweight | FLX | 137,564,473 | 552,870 | 499,499 | 934 |
| F7T1Ob1 | TS19 | Twin | 7 | Obese | FLX | 95,538,760 | 583,989 | 498,880 | 569 |
| F7T2Ob1 | TS20 | Twin | 7 | Obese | FLX | 108,342,331 | 550,695 | 495,040 | 829 |
| F7MOb1 | TS21 | Mom | 7 | Obese | FLX | 95,960,723 | 451,177 | 413,772 | 774 |
| F10T1Ob1 | TS28 | Twin | 10 | Obese | Titanium | 138,364,927 | 399,717 | 302,780 | 652 |
| F10T2Ob1 | TS29 | Twin | 10 | Obese | Titanium | 239,971,702 | 672,196 | 502,399 | 1,190 |
| F10MOv1 | TS30 | Mom | 10 | Overweight | FLX | 105,932,316 | 564,184 | 495,865 | 791 |
| F15T1Ob1 | TS49 | Twin | 15 | Obese | FLX | 104,449,087 | 596,149 | 519,072 | 769 |
| F15T2Ob1 | TS50 | Twin | 15 | Obese | FLX | 129,037,456 | 642,191 | 549,700 | 1,209 |
| F15MOb1 | TS51 | Mom | 15 | Obese | FLX | 101,531,105 | 557,165 | 434,187 | 582 |
| | | | | | **SUM** | 2,136,433,483 | 9,634,178 | 8,294,835 | 14,730 |

| | | | | | | | | | |
|---|---|---|---|---|---|---|---|---|---|
| ^{a} ID nomenclature: Family Number, Twin number or mom, and BMI category (Le = lean, Ov = overweight, Ob = Obese; e.g. F1T1Le Stands for family 1, twin 1, lean) ^{b} Sequences used after removing low quality, duplicate, and human sequences ^{c} 16S rRNA gene fragments identified in microbiome sequencing reads | | | | | | | | | |

| **Table 9: Microbiome BLAST statistics^{a}** | | | | | | | | | | | | |
|---|---|---|---|---|---|---|---|---|---|---|---|---|
| Subject ID^{a} | Data ID | Raw Reads | Reads Used | % Sequences Used | Nucleotides Used | Mean Read-length | % Hsa | % RDP | % KEGG | % STRING | % NR | % Gut |
| F1T1Le1 | TS1 | 254,044 | 217,386 | 85.6 | 51,708,794 | 237.9 | 0.42 | 0.21 | 29.1 | 34.5 | 54.9 | 57.9 |
| F1T2Le1 | TS2 | 514,022 | 443,640 | 86.3 | 78,853,892 | 177.7 | 0.08 | 0.12 | 20.3 | 28.7 | 46.9 | 51.7 |
| F1MOv1 | TS3 | 571,301 | 510,972 | 89.4 | 102,717,417 | 201.0 | 0.16 | 0.15 | 23.8 | 33.6 | 56.5 | 61.2 |
| F2T1Le1 | TS4 | 472,154 | 414,754 | 87.8 | 95,003,113 | 229.1 | 0.14 | 0.15 | 26.2 | 44.5 | 72.3 | 74.9 |
| F2T2Le1 | TS5 | 553,142 | 490,776 | 88.7 | 100,599,979 | 205.0 | 0.22 | 0.19 | 23.0 | 27.8 | 54.1 | 62.1 |
| F2MOb1 | TS6 | 623,027 | 535,763 | 86.0 | 118,207,161 | 220.6 | 0.62 | 0.20 | 26.9 | 37.2 | 58.9 | 62.1 |
| F3T1Le1 | TS7 | 607,386 | 555,853 | 91.5 | 134,889,015 | 242.7 | 0.13 | 0.22 | 26.9 | 34.0 | 58.4 | 61.7 |
| F3T2Le1 | TS8 | 468,769 | 414,497 | 88.4 | 100,520,072 | 242.5 | 0.20 | 0.24 | 28.5 | 35.7 | 61.1 | 64.4 |
| F3MOv1 | TS9 | 552,870 | 499,499 | 90.3 | 124,768,172 | 249.8 | 0.14 | 0.19 | 26.8 | 36.6 | 63.2 | 66.3 |
| F7T1Ob1 | TS19 | 583,989 | 498,880 | 85.4 | 82,117,565 | 164.6 | 0.06 | 0.12 | 19.1 | 30.6 | 52.9 | 57.1 |
| F7T2Ob1 | TS20 | 550,695 | 495,040 | 89.9 | 98,053,098 | 198.1 | 0.32 | 0.17 | 22.3 | 29.3 | 47.2 | 49.9 |
| F7MOb1 | TS21 | 451,177 | 413,772 | 91.7 | 88,786,017 | 214.6 | 0.09 | 0.19 | 25.5 | 37.6 | 62.8 | 66.3 |
| F10T1Ob1 | TS28 | 399,717 | 302,780 | 75.7 | 101,434,082 | 335.0 | 0.06 | 0.36 | 24.5 | 28.4 | 53.2 | 55.5 |
| F10T2Ob1 | TS29 | 672,196 | 502,399 | 74.7 | 173,386,030 | 345.1 | 0.11 | 0.29 | 27.5 | 34.8 | 63.2 | 63.9 |
| F10MOv1 | TS30 | 564,184 | 495,865 | 87.9 | 94,405,318 | 190.4 | 0.21 | 0.16 | 22.4 | 32.0 | 54.7 | 60.7 |
| F15T1Ob1 | TS49 | 596,149 | 519,072 | 87.1 | 91,987,878 | 177.2 | 0.29 | 0.15 | 18.6 | 23.0 | 43.7 | 46.4 |
| F15T2Ob1 | TS50 | 642,191 | 549,700 | 85.6 | 111,999,603 | 203.7 | 0.24 | 0.22 | 24.6 | 29.4 | 51.9 | 57.9 |
| F15MOb1 | TS51 | 557,165 | 434,187 | 77.9 | 81,330,211 | 187.3 | 0.40 | 0.14 | 21.0 | 26.3 | 44.2 | 43.9 |
| **Average** | | **535,232** | **460,824** | **86.1** | **101,709,301** | **223.5** | **0.22** | **0.19** | **24.3** | **32.5** | **55.6** | **59.1** |
| **Sum** | | 9,634,178 | 8,294,835 | ------ | 1,830,767,417 | ----- | ----- | ----- | ----- | ----- | ----- | ----- |

| | | | | | | | | | | | | |
|---|---|---|---|---|---|---|---|---|---|---|---|---|
| ^{a}Key: %sequences used=percentage of sequences remaining after removing low quality, duplicate, and human sequences; Hsa=reads matching the H.sapiens genome; %RDP=percentage of reads matching the RDP 16S rRNA database; %KEGG, %STRING, %NR=percentage of reads that were assignable to entries in these various databases; %Gut=percentage of reads assigned to the database of 42 reference genomes | | | | | | | | | | | | |

### Database searches and metabolic reconstructions

The distributions of taxa, genes, orthologs, metabolic pathways, and high-level gene categories were tallied based on the corresponding annotation of the best-BLAST-hit sequence found in each reference database. For KEGG analysis, the closest matching gene with an annotation was used, since many genes in the database remain unannotated, including all KEGG orthologous groups (KOs) assigned to genes with an identical e-value (commands -e 0.00001 -m 9 -b 100 were used to run NCBI BLASTX). Custom Perl scripts were used for all KEGG, STRING, and NCBI NR analyses. Selected genes from recently sequenced reference genomes were manually annotated using NCBI-BLASTP searches against the KEGG, STRING, and NR database. The 42 reference genome database includes predicted proteins from draft or complete assemblies of *Alistipes putredinis, Bacteroides WH2, Bacteroides thetaiotaomicron 3731, Bacteroides thetaiotaomicron 7330, Bacteroides thetaiotaomicron 5482, Bacteroides fragilis, Bacteroides caccae, Bacteroides distasonis, Bacteroides ovatus, Bacteroides stercoris, Bacteroides uniformis, Bacteroides vulgatus, Parabacteroides merdae, Anaerostipes caccae, Anaerotruncus colihominis, Anaerofustis stercorihominis, Bacteroides capillosus, Clostridium bartlettii, Clostridium bolteae, Clostridium eutactus, Clostridium leptum, Clostridium ramosum, Clostridium scindens, Clostridium sp.L2-50, Clostridium spiroforme, Dorea longicatena, Eubacterium dolichum, Eubacterium eligens, Eubacterium rectale, Eubacterium siraeum, Eubacterium ventriosum, Faecalibacterium prausnitzii M212, Peptostreptococcus micros, Ruminococcus gnavus, Ruminococcus obeum, Ruminococcus torques, Collinsella aerofaciens, Bifidobacterium adolescentis, Bifidobacterium longum, Escherichia coli K12, Methanobrevibacter smithii, and Methanobrevibacter stadtmanae* (see http://genome.wustl.edu/pub/ and NCBI GenBank). Draft assemblies of *Clostridium sp. SS2-1* and *Clostridium symbiosum* were also used for functional clustering and diversity analyses (http://genome.wustl.edu/pub/). Coverage plots (percent identity plots) were generated using nucmer and mummerplot (part of the MUMmer v3.19 package), and default parameters.

Annotations were validated with simulated datasets (**FIG. 8**). To do so, the frequency of annotated genes from the KEGG database (v44) was first tallied across the aggregate human gut microbiomes (n=18 datasets). The 1,000 most frequent microbial genes were then used to generate 'simulated reads' between 50 and 500 nt long. The simulated reads were subsequently annotated (BLASTX against the KEGG database), with self-hits excluded. This analysis revealed a low rate of false positives (i.e. high precision), but using very short sequences (e.g. 50-100 nt) increased the rate of false negatives (lower sensitivity) (**FIG. 8**). Given the increased read-length relative 454 GS20 pyrosequencing data, simulated reads with an average length comparable to our data (200-250 nt), demonstrated robust assignments with an e-value<10⁻⁵, %identity>50, and/or bit-score>50. Using all three cutoffs, sequences 200 nt in length returned 81.5% of the correct assignments, with a precision of 0.93 and sensitivity of 0.88, similar to what was observed by re-annotating the original full-length gene sequences after ignoring self-hits. The KEGG cutoff criteria were also applied to BLASTX analysis results for STRING-based predictions, given the similar size of the databases.

ABI 3730xl capillary sequencing reads from 9 previously published adult human gut microbiomes were obtained from the NCBI TraceArchive. The full dataset from each sample was annotated by BLASTX comparisons against the KEGG and STRING database (see above; BLASTX e-value<10⁻⁵, %identity>50, and score>50). To allow quantitative comparisons between these datasets and pyrosequencing data, all forward sequencing reads was first extracted and then one 'simulated pyrosequencer read' from each longer capillary read was generated. Nucleotides spanning positions 100 to 322 were used from all capillary reads of suitable length, to avoid low quality regions that commonly occur at the beginning and end of the reads. These simulated reads were then annotated as described above.

16S rRNA gene fragments were identified in each microbiome through BLASTN searches of the RDP database (version 9.33; e-value<10⁻⁵; Bit-score>50; %identity>50; alignment length≥100). Putative 16S rRNA gene fragments were then aligned using the NAST multi-aligner with a minimum template length of 100 bases and minimum %identity of 75%. Taxonomy was assessed after insertion into an ARB neighbor-joining tree.

Microbiomes were clustered based on their profiles after normalizing across all sampled communities (z-score), using the Pearson's correlation distance metric, followed by single-linkage hierarchical clustering in addition to Principal Components Analysis (Cluster3.0). Results were visualized using the Treeview Java applet. Functional diversity (Shannon index and evenness) was calculated using the number of assignements in each microbiome to each of the 254 pathways present in the KEGG database (EstimateS 8.0). The maximum possible index is the natural log of the total number of pathways: In (254) or 5.54. Shannon evenness was calculated by dividing the Shannon index for a given microbiome by the maximum possible index (scale of 0 to 1, with 1 representing a microbiome with all pathways found at an equal abundance). Results were compared to simulated metagenomic reads generated from 36 recently sequenced reference human gut-derived Bacteroidetes and Firmicutes genomes (http://genome.wustl.edu/pub/organism/). Reads were produced by Readsim v0.10, using the following options: -n 10000 -modlr normal -meanlr 223 -stdlr 0.3. The mean and standard deviation for length of the simulated reads was based on the observed read-length distribution of the 18 fecal microbiome datasets (**Table 9**).

### Results

One fundamental parameter that governs the utility of reference genomes is the ability to accurately assign fragmentary reads from metagenomic datasets to these genomes. Therefore, the filtered pyrosequencing reads from the fecal microbiomes of 18 individuals from the 6 different families described in Example 1 (3 lean twin-pairs and their mothers; 3 obese twin pairs and their mothers; Tables 1 and 2) were compared to a custom database of 42 human gut associated bacterial and archaeal genomes (**FIG. 7**) using BLASTX, and validated these assignments independently against NCBI's non-redundant protein database. The relative abundance of sequences from the 18 individual microbiome datasets assigned to each reference genome was highly variable (see **FIG. 9**; R²=0.26±0.02 for all pairwise comparisons of taxonomic profiles), consistent with the considerable heterogeneity in microbial community structure among the fecal microbiomes observed from sequencing 16S rRNA gene amplicons.

The custom database of 42 reference genomes included 23 Firmicutes but only 13 Bacteroidetes. Since the Firmicutes dominate the gut microbiotas of subjects (**FIG. 6**) and the reference genome database, it might be expected that reads assigned to Firmicutes would match the reference genomes more closely than reads assigned to Bacteroidetes. The opposite was true: on average, 46.3±2.6% of the pyrosequencing reads assigned to Bacteroidetes matched the reference genomes at 100% identity, as compared to only 16.7±1.1% of the reads assigned to Firmicutes (p<10⁻⁴, Mann Whitney; **FIGS. 10** and **11**). This observation underscores the high level of phylogenetic and genomic diversity within the gut-associated Firmicutes, indicates that the readily culturable sequenced gut Firmicutes are not closely related to the abundant gut genomes present in the 18 gut microbiomes, and suggests that future reference microbial genome sequencing efforts should be directed towards representatives of this dominant phylum.

The effect of technical advances that produce longer reads on improving these assignments was also tested by sequencing fecal community samples from one twin pair using next-generation Titanium pyrosequencing methods [average read length of 341±134 nt (SD) versus 208±68 for the standard FLX platform]. **FIG. 12** shows that the frequency and quality of sequence assignments is improved as read length increases from 200 to 350 nt.

**FIG. 13** summarizes the relative abundance of the major bacterial phyla present in these 18 microbiomes, as defined by six different approaches (sequencing full-length, V2/3 and V6 amplicons; BLAST comparisons of shotgun pyrosequencer reads with the NCBI non-redundant and the custom 42 gut genome databases, plus analysis of 16S rRNA gene fragments). Pairwise comparisons of relative abundance data from 16S rRNA gene fragments generated from shotgun sequencing reads correlate most closely with V2/3 PCR data (**FIG. 13** and **Table 7**).

### Example 4: In silico functional analysis of gut microbiomes

The filtered sequences obtained in Example 3 from the 18 microbiomes were used to conduct a functional analysis of gut microbiomes.

### CAZyme analysis

Metagenomic sequence reads described in Example 3 were searched against a library of modules derived from all entries in the Carbohydrate-Active enZymes (CAZy) database (www.cazy.org using FASTY, e-value<10⁻⁶). This library consists of ~180,000 previously annotated modules (catalytic modules, carbohydrate binding modules (CBMs) and other non-catalytic modules or domains of unknown function) derived from ~80,000 protein sequences. The number of sequencing reads matching each CAZy family was divided by the number of total sequences assigned to CAZymes and multiplied by 100 to calculate a relative abundance. An R² value was calculated for each pair of CAZy profiles. The distribution of glycoside hydrolase similarity scores was then compared to the distribution of glycosyltransferase similarity scores.

### Statistical analyses

Xipe (version 2.4) was employed for bootstrap analyses of pathway enrichment and depletion, using the parameters sample size=10,000 and confidence level=0.95. Linear regressions were performed in Excel (version 11.0, Microsoft). Mann-Whitney and Student's t-tests were utilized to identify statistically significant differences between two groups (Prism v4.0, GraphPad; Excel version 11.0, Microsoft). The Bonferroni correction was used to correct for multiple hypotheses. The Mantel test was used to compare distance matrices: the matrix of each pairwise comparison of the abundance of each reference genome, and the abundance of each metabolic pathway, were compared (Mantel program in Python using PyCogent; 10,000 replicates). Data are represented as mean±SEM unless otherwise indicated.

Odds ratios were used to identify 'commonly-enriched' genes in the gut microbiome. In short, all gut microbiome sequences were compared against the custom database of 42 gut genomes (BLASTX e-value<10⁻⁵, bitscore>50, and %identity>50). A gene by sample matrix was then screened to identify genes 'commonly-enriched' in either the obese or lean gut microbiome (defined by an odds ratio greater than 2 or less than 0.5 when comparing the pooled obese twin microbiomes to the pooled lean twin microbiomes and when comparing each individual obese twin microbiome to the aggregate lean twin microbiome, or vice versa). The statistical significance of enriched or depleted genes was then calculated using a modified t-test (q-value<0.05; calculated with code kindly supplied by Mihai Pop and J.R. White, University of Maryland). To search for genes that were consistently enriched or depleted in all six MZ twin-pairs, a gene-by-sample matrix was generated based on BLASTX comparisons of each microbiome with our custom 42-genome database, and an odds ratio was calculated by directly comparing the frequency of each gene in each twin versus the respective co-twin. The analysis revealed only 49 genes (odds ratio>2 or <0.5): they represent a variety of taxonomic groups, including Firmicutes, Bacteroidetes, and Actinobacteria and did not show any clear functional trends.

### Results

Sequences matching 156 total CAZyme families were found within at least one human gut microbiome, including 77 glycoside hydrolase, 21 carbohydrate-binding module, 35 glycosyltransferase, 12 polysaccharide lyase, and 11 carbohydrate-esterase families (**Table 10A** and **B**). On average 2.62±0.13% of the gut microbiome could be assigned to CAZymes (a total of 217,615 sequences), a percentage that is greater than the most abundant KEGG pathway in the gut microbiome ('Transporters'; 1.20±0.06%), and indicative of the abundant and diverse set of microbial genes in the distal gut microbiome directed towards accessing a wide range of polysaccharides.

Category-based clustering of the functions from each microbiome was performed using Principal Components Analysis (PCA) and hierarchical clustering. This analysis revealed two distinct clusters of gut microbiomes based on metabolic profile, corresponding to samples with an increased abundance of Firmicutes and Actinobacteria, and samples with a high abundance of Bacteroidetes (**FIG. 14A**). A linear regression of the first principal component (PC1, explaining 20% of the functional variance) and the relative abundance of the Bacteroidetes showed a highly significant correlation (R²=0.96, p<10-12; **FIG. 14B**). Functional profiles stabilized within each individual's microbiome after ~20,000 sequences had been accumulated (**FIG. 15**). Family members had more similar functional profiles than unrelated individuals (**FIG**. **14C**), suggesting that shared bacterial community structure (who's there based on 16S rRNA analyses) also translates into shared community-wide relative abundance of metabolic pathways. Accordingly, a direct comparison of functional and taxonomic similarity disclosed a significant association: individuals that share similar taxonomic profiles also share similar metabolic profiles (p<0.001; Mantel test).

| **Table 10A: Relative abundance of CAZymes across 9 gut microbiomes (% of sequence assignments across all identified CAZymes)^{a}** | | | | | | | | | |
|---|---|---|---|---|---|---|---|---|---|
| **Subject ID^{b}** | **F1T1Le** | **F1T2Le** | **F1MOv** | **F2T1Le** | **F2T2Le** | **F2MOb** | **F3T1Le** | **F3T2Le** | **F3MOv** |
| **Glycoside hydrolases** | **70.56** | **73.96** | **72.14** | **72.40** | **68.38** | **67.37** | **68.69** | **67.84** | **69.92** |
| GH13 | 8.96 | 6.31 | 6.37 | 3.97 | 10.78 | 8.04 | 8.63 | 9.97 | 8.02 |
| GH2 | 7.40 | 7.10 | 7.01 | 6.51 | 5.13 | 5.49 | 5.81 | 6.02 | 5.94 |
| GH43 | 3.48 | 5.78 | 5.63 | 6.61 | 4.39 | 4.69 | 5.05 | 4.14 | 5.75 |
| GH92 | 3.44 | 6.25 | 5.00 | 7.70 | 3.25 | 5.47 | 3.28 | 2.65 | 4.50 |
| GH3 | 5.72 | 5.37 | 4.31 | 4.47 | 3.20 | 3.94 | 4.03 | 4.70 | 4.09 |
| GH97 | 1.97 | 5.45 | 4.01 | 4.67 | 1.18 | 3.38 | 3.51 | 2.23 | 3.91 |
| GH31 | 2.98 | 2.48 | 2.53 | 2.41 | 3.84 | 2.11 | 2.16 | 3.04 | 2.13 |
| GH20 | 2.40 | 2.30 | 2.35 | 3.34 | 1.93 | 2.93 | 1.99 | 1.92 | 2.19 |
| GH29 | 1.99 | 1.51 | 2.12 | 2.54 | 2.94 | 2.52 | 2.53 | 2.19 | 1.83 |
| GH77 | 2.13 | 1.39 | 1.43 | 0.86 | 2.18 | 2.18 | 2.18 | 2.45 | 1.99 |
| GH28 | 1.58 | 2.44 | 3.71 | 3.07 | 1.46 | 2.24 | 2.25 | 1.79 | 2.00 |
| GH51 | 1.18 | 1.51 | 1.38 | 1.44 | 2.12 | 1.58 | 1.73 | 1.68 | 1.31 |
| GH36 | 1.62 | 1.12 | 1.19 | 0.99 | 1.80 | 1.23 | 1.64 | 2.02 | 1.37 |
| GH1 | 1.51 | 0.87 | 1.02 | 0.34 | 2.90 | 1.08 | 1.50 | 1.50 | 1.67 |
| GH5 | 1.95 | 2.41 | 1.75 | 1.53 | 1.07 | 0.98 | 2.62 | 1.45 | 1.95 |
| GH42 | 0.91 | 0.49 | 0.83 | 0.90 | 2.43 | 0.62 | 1.09 | 1.10 | 1.03 |
| GH105 | 1.56 | 1.65 | 2.07 | 2.07 | 1.01 | 1.38 | 1.46 | 1.27 | 1.83 |
| GHY95 | 1.56 | 1.18 | 1.36 | 1.24 | 0.91 | 1.21 | 1.22 | 1.04 | 0.99 |
| GH32 | 0.91 | 0.61 | 0.70 | 0.75 | 2.12 | 1.18 | 1.05 | 0.91 | 0.84 |
| GH78 | 1.91 | 1.09 | 1.22 | 1.61 | 0.60 | 0.70 | 1.05 | 0.89 | 1.25 |
| **Glycosyltransferases** | **20.25** | **17.20** | **17.49** | **16.26** | **23.34** | **21.64** | **22.09** | **22.78** | **19.66** |
| GT2 | 5.66 | 6.26 | 6.31 | 5.58 | 7.68 | 7.91 | 7.14 | 7.48 | 7.39 |
| GT4 | 3.55 | 3.76 | 3.96 | 4.44 | 4.93 | 4.43 | 4.64 | 4.60 | 4.20 |
| GT35 | 4.75 | 2.47 | 2.07 | 1.62 | 4.75 | 2.85 | 3.58 | 3.91 | 2.90 |
| GT28 | 1.51 | 0.85 | 0.89 | 0.53 | 1.51 | 1.00 | 1.34 | 1.48 | 1.00 |
| GT5 | 1.74 | 0.77 | 0.79 | 0.33 | 1.72 | 0.81 | 1.38 | 1.62 | 1.15 |
| GT51 | 0.77 | 0.78 | 0.75 | 0.74 | 0.99 | 1.08 | 0.92 | 1.17 | 0.80 |
| **Carbohydrate binding molecules** | **1.76** | **2.40** | **2.15** | **2.02** | **2.05** | **2.22** | **2.38** | **2.25** | **2.11** |
| **Carbohydrate esterases** | **5.89** | **4.70** | **5.45** | **5.53** | **5.00** | **5.81** | **5.64** | **5.36** | **6.04** |
| CE4 | 1.53 | 1.01 | 1.03 | 0.78 | 1.41 | 1.04 | 1.16 | 1.27 | 1.20 |
| **Polysaccharide lyases** | **1.55** | **1.74** | **2.77** | **3.79** | **1.22** | **2.95** | **1.20** | **1.78** | **2.27** |

| | | | | | | | | | |
|---|---|---|---|---|---|---|---|---|---|
| ^{a} Groups found at an average relative abundance 1% are shown ^{b} ID nomenclature: Family number, Twin number or mother and BMI category (Le=lean, Ov=overweight, Ob=obese e.g. F1 T1 Le stands for family 1 twin 1 lean) | | | | | | | | | |

| **Table 10B: Relative abundance of CAZymes across 9 gut microbiomes (% of sequence assignments across all identified CAZymes)^{a}** | | | | | | | | | |
|---|---|---|---|---|---|---|---|---|---|
| **Subject ID^{b}** | **F4T1Ob** | **F4T2Ob** | **F4MOb** | **F5T1Ob** | **F5T2Ob** | **F5MOv** | **F6T1Ob** | **F6T2Ob** | **F6MOb** |
| **Glycoside hydrolases** | **73.46** | **70.45** | **71.57** | **64.19** | **69.11** | **69.96** | **68.15** | **69.61** | **71.50** |
| GH13 | 4.68 | 8.36 | 6.37 | 11.17 | 11.80 | 7.05 | 12.34 | 16.84 | 11.19 |
| GH2 | 6.43 | 6.53 | 6.53 | 5.52 | 5.40 | 5.93 | 5.69 | 5.64 | 6.21 |
| GH43 | 5.80 | 6.49 | 5.00 | 4.34 | 6.57 | 5.04 | 5.05 | 5.59 | 4.56 |
| GH92 | 7.66 | 4.36 | 6.72 | 1.71 | 1.73 | 5.70 | 1.93 | 0.60 | 3.59 |
| GH3 | 3.46 | 3.77 | 4.27 | 3.89 | 5.07 | 3.75 | 3.75 | 4.29 | 3.41 |
| GH97 | 4.06 | 3.95 | 3.62 | 0.96 | 1.25 | 3.96 | 1.22 | 0.28 | 1.87 |
| GH31 | 2.67 | 2.06 | 2.49 | 2.86 | 3.37 | 2.52 | 2.81 | 3.99 | 2.79 |
| GH20 | 3.33 | 2.45 | 3.32 | 1.09 | 1.17 | 3.12 | 1.66 | 0.92 | 3.18 |
| GH29 | 3.93 | 1.53 | 3.31 | 1.80 | 1.47 | 2.59 | 1.51 | 0.93 | 1.81 |
| GH77 | 1.32 | 1.95 | 1.49 | 2.87 | 2.95 | 1.62 | 2.64 | 3.47 | 2.04 |
| GH28 | 2.63 | 1.99 | 2.49 | 1.64 | 1.01 | 2.31 | 1.44 | 0.54 | 1.11 |
| GH51 | 1.73 | 2.29 | 1.51 | 1.80 | 2.74 | 1.40 | 1.71 | 2.34 | 1.60 |
| GH36 | 1.24 | 1.79 | 1.39 | 1.52 | 1.92 | 1.28 | 2.20 | 2.63 | 2.37 |
| GH1 | 0.72 | 0.79 | 0.71 | 2.01 | 2.50 | 1.35 | 3.74 | 2.29 | 2.25 |
| GH5 | 1.37 | 2.56 | 1.30 | 1.29 | 1.37 | 0.90 | 0.84 | 1.22 | 0.95 |
| GH42 | 0.94 | 0.44 | 0.98 | 1.80 | 2.82 | 0.93 | 2.26 | 3.87 | 2.06 |
| GH105 | 1.77 | 0.83 | 1.63 | 0.95 | 0.50 | 1.65 | 0.98 | 0.39 | 0.83 |
| GHY95 | 1.33 | 1.90 | 1.12 | 0.68 | 0.75 | 1.35 | 1.01 | 0.48 | 1.44 |
| GH32 | 0.99 | 1.15 | 0.82 | 1.15 | 1.52 | 0.99 | 1.47 | 2.04 | 1.00 |
| GH78 | 1.43 | 1.45 | 0.98 | 1.03 | 1.39 | 0.80 | 0.90 | 0.58 | 1.21 |
| **Glycosyltransferases** | **16.68** | **20.34** | **18.24** | **26.36** | **23.15** | **19.53** | **23.54** | **23.99** | **21.50** |
| GT2 | 6.19 | 6.80 | 6.97 | 9.41 | 9.80 | 6.74 | 7.98 | 7.14 | 6.78 |
| GT4 | 4.17 | 3.99 | 4.08 | 5.62 | 4.43 | 4.50 | 4.42 | 4.18 | 4.80 |
| GT35 | 1.81 | 2.76 | 2.13 | 4.50 | 3.78 | 2.59 | 4.42 | 5.25 | 3.66 |
| GT28 | 0.58 | 0.94 | 0.83 | 1.31 | 1.00 | 1.01 | 1.48 | 2.12 | 1.33 |
| GT5 | 0.46 | 0.83 | 0.65 | 1.54 | 1.24 | 0.96 | 1.74 | 1.90 | 0.96 |
| GT51 | 0.68 | 1.06 | 0.72 | 1.82 | 1.27 | 0.88 | 1.06 | 1.63 | 1.02 |
| **Carbohydrate binding molecules** | **1.90** | **2.06** | **2.15** | **2.66** | **2.88** | **2.08** | **2.22** | **2.28** | **1.98** |
| **Carbohydrate esterases** | **5.19** | **5.19** | **5.02** | **5.24** | **3.94** | **6.01** | **4.68** | **3.84** | **4.15** |
| CE4 | 0.73 | 0.84 | 0.92 | 1.35 | 0.96 | 1.04 | 1.31 | 1.51 | 0.91 |
| **Polysaccharide lyases** | **2.78** | **1.95** | **3.02** | **1.55** | **0.93** | **2.43** | **1.43** | **0.28** | **0.87** |

| | | | | | | | | | |
|---|---|---|---|---|---|---|---|---|---|
| ^{a} Groups found at an average relative abundance 1% are shown ^{b} ID nomenclature: Family number, Twin number or mother and BMI category (Le=lean, Ov=overweight, Ob=obese e.g. F1T1Le stands for family 1 twin 1 lean) | | | | | | | | | |

### Example 5: Different functions for Bacteroides and Firmicutes

Functional clustering of phylum-wide sequence bins representing reads from the Firmicutes or the Bacteroidetes showed discrete clustering by phylum (**FIG. 16A**). A direct comparison of the Firmicutes and Bacteroidetes sequence bins to simulated reads generated from 36 reference Bacteroides and Firmicute genomes represented in the 42 member custom database described in Example 3, revealed that the metabolic profile of each microbiome was similar to the 'average' metabolic profile of each phylum (**FIG. 17**). Bootstrap analyses of the relative abundance of metabolic pathways in the Firmicutes and Bacteroidetes, disclosed 26 pathways with a significantly different relative abundance (**FIG. 16A**). The Bacteroidetes were enriched for a number of carbohydrate metabolism pathways, while the Firmicutes were enriched for transport systems. The finding is consistent with information gleaned from a number of sequenced Bacteroidetes genomes that demonstrate expansive families of genes involved in carbohydrate metabolism, as well as the CAZyme analysis in Example 3, which revealed a significantly higher relative abundance of glycoside hydrolases, carbohydrate-binding modules, glycosyltransferases, polysaccharide lyases, and carbohydrate esterases in the Bacteroidetes sequence bins (**FIG. 16B**).

### Example 6: Identifying a core human gut microbiome

One of the major goals of the international human microbiome project is to determine whether there is an identifiable 'core microbiome' of shared organisms, genes, or functional capabilities found in a given body habitat of all or the vast majority of humans. Although all of the 18 gut microbiomes surveyed showed a high level of beta-diversity with respect to the relative abundance of bacterial phyla (**FIG. 18A**), analysis of the relative abundance of broad functional categories of genes (COG) and metabolic pathways (KEGG) revealed a generally consistent pattern regardless of the sample surveyed (**FIG. 18B** **and Table 11**): the pattern is also consistent with results obtained from a meta-analysis of previously published gut microbiome datasets from 9 adult individuals (**FIG. 19**). This consistency was not simply due to the broad level of these annotations, as a similar analysis of Bacteroidetes and Firmicutes reference genomes revealed substantial variation in the relative abundance of each category (**FIG. 20**). Furthermore, pair-wise comparisons of metabolic profiles revealed an average R² of 0.97±0.0023 (**FIG. 14A**), indicating a high level of functional similarity between adult human gut microbiomes.

| **Table 11: Relative abundance of metabolic pathways in the gut microbiome (% of KEGG assignments)^{a}** | |
|---|---|
| KEGG Metabolic Pathway | Mean±sem across all 18 microbiomes |
| Transporters | 4.93 ± 0.21 |
| Other replication, recombination and repair proteins | 3.35 ± 0.04 |
| ABC transporters | 3.24 ± 0.13 |
| General function prediction only | 2.60 ± 0.06 |
| Purine metabolism | 2.29 ± 0.02 |
| Other enzymes | 2.16 ± 0.03 |
| Aminoacyl-tRNA biosynthesis | 2.14 ± 0.05 |
| Glutamate metabolism | 1.98 ± 0.03 |
| Starch and sucrose metabolism | 1.92 ± 0.03 |
| Pyruvate metabolism | 1.73 ± 0.02 |
| Pyrimidine metabolism | 1.70 ± 0.02 |
| Peptidases | 1.69 ± 0.05 |
| Alanine and aspartate metabolism | 1.58 ± 0.02 |
| Glycine, serine and threonine metabolism | 1.53 ± 0.02 |
| Other translation proteins | 1.37 ± 0.02 |
| Galactose metabolism | 1.37 ± 0.03 |
| Glycolysis / Gluconeogenesis | 1.35 ± 0.02 |
| Other ion-coupled transporters | 1.34 ± 0.06 |
| Fructose and mannose metabolism | 1.31 ± 0.03 |
| Two-component system | 1.31 ± 0.03 |
| Ribosome | 1.27 ± 0.03 |
| Replication complex | 1.18 ± 0.02 |
| Phenylalanine; tyrosine and tryptophan biosynthesis | 1.17 ± 0.02 |
| Valine, leucine and isoleucine biosynthesis | 1.15 ± 0.02 |
| Carbon fixation | 1.15 ± 0.01 |
| Nitrogen metabolism | 1.13 ± 0.02 |
| Glycerolipid metabolism | 1.07 ± 0.02 |
| Oxidative phosphorylation | 1.07 ± 0.03 |
| Butanoate metabolism | 1.05 ± 0.02 |
| Chaperones and folding catalysts | .99 ± 0.01 |
| Pentose phosphate pathway | .95 ± 0.01 |
| Tyrosine metabolism | .95 ± 0.02 |
| Histidine metabolism | .92 ± 0.02 |
| Cell division | .91 ± 0.01 |
| Aminosugars metabolism | .89 ± 0.03 |
| Arginine and proline metabolism | .85 ± 0.01 |
| Citrate cycle (TCA cycle) | .84 ± 0.02 |
| Methlionine metabolism | .83 ± 0.02 |
| Lysine biosynthesis | .82 ± 0.01 |
| RNA polymerase | .81 ± 0.02 |
| Reductive carboxylate cycle (CO2 fixation) | .80 ± 0.03 |
| Propanoate metabolism | .80 ± 0.01 |
| Peptidoglycan biosynthesis | .79 ± 0.01 |
| N-Glycan degradation | .78 ± 0.05 |
| Urea cycle and metabolism of amino groups | .78 ± 0.01 |
| Translation factors | .78 ± 0.02 |
| Selenoamino acid metabolism | .77 ± 0.02 |
| Glyoxylate and dicarboxylate metabolism | .73 ± 0.01 |
| DNA polymerase | .72 ± 0.01 |
| Pentose and glucuronate interconversions | .70 ± 0.02 |
| Cysteine metabolism | .68 ± 0.02 |
| Pantothenate and CoA biosynthesis | .67 ± 0.01 |
| Nucleotide sugars metabolism | .67 ± 0.02 |
| Glycosaminoglycan degradation | .66 ± 0.04 |
| Function unknown | .66 ± 0.01 |
| One carbon pool by folate | .65 ± 0.01 |
| Sphingolipid metabolism | .64 ± 0.03 |
| Protein export | .62 ± 0.01 |

| | |
|---|---|
| ^{a}Pathways with an average relative abundance of > 0.6% are shown | |

Overall functional diversity was compared using the Shannon index, a measurement that combines diversity (the number of different types of metabolic pathways) and evenness (the relative abundance of each pathway). The human gut microbiomes surveyed had a stable and high Shannon index value (4.63±0.01), close to the maximum possible level of functional diversity (5.54; See Example 4). Despite the presence of a small number of abundant metabolic pathways (listed in **Table 11**), the overall functional profile of each gut microbiome is quite even (Shannon evenness of 0.84±0.001 on a scale of 0 to 1), demonstrating that most metabolic pathways are found at a similar level of abundance. Interestingly, the level of functional diversity in each microbiome was significantly linked to the relative abundance of the Bacteroidetes (R²=0.81, p<10⁻⁶); microbiomes enriched for Firmicutes/Actinobacteria had a decreased level of functional diversity. This observation is consistent with an analysis of simulated metagenomic reads generated from each of 36 Bacteroidetes and Firmicutes genomes (**FIG. 21**): on average, the Bacteroidetes genomes have a significantly higher level of both functional diversity and evenness (Mann-Whitney, p<0.01).

At a finer level, 26-53% of 'enzyme'-level functional groups were shared across all 18 microbiomes, while 8-22% of the groups were unique to a single microbiome (**FIGS. 22A-C**). The 'core' functional groups present in all microbiomes were also highly abundant, representing 93-98% of the sequences found in the gut (fecal) microbiome. Given the higher relative abundance of these 'core' groups, >95% were found after 26.11±2.02 Mb of sequence was collected from a given microbiome, whereas the 'variable' groups continue to increase substantially with each additional Mb sequence. Of course, any estimate of the total size of the core microbiome will be dependent upon sequencing effort, especially for functional groups found at a low abundance. On average, this survey achieved greater than 450,000 sequences per fecal sample, which, assuming an even distribution, would allow us to sample groups found at a relative abundance of 10⁻⁴. In order to estimate the total size of the core microbiome based on the 18 sampled individuals, each microbiome was randomly subsampled in 1,000 sequence intervals (**FIG. 22D**). Based on this analysis, the core microbiome is approaching a total of 2,142 total orthologous groups (one site binding hyperbola curve fit to the resulting rarefaction curve, R²=0.9966), indicating that 93% of functional groups (defined by STRING) found within the core microbiome, were already identified. Of these core groups, 64% (KEGG) and 56% (STRING) were also found in 9 previously published but much lower coverage datasets generated by capillary sequencing of adult fecal DNA (average of 78,413±2,044 bidirectional reads/sample).

Metabolic reconstructions of the 'core' microbiome revealed significant enrichment for a number of expected functional categories, including those involved in transcription, translation, and amino acid metabolism (**FIG**. 23). Metabolic profile-based clustering indicated that the representation of 'core' functional groups was highly consistent across samples (**FIG. 24**), and includes a number of pathways likely important for life in the gut, such as those for carbohydrate and amino acid metabolism (e.g. fructose/mannose metabolism, aminosugars metabolism, and N-Glycan degradation). Variably represented pathways and categories include cell motility (only a subset of Firmicutes produce flagella), secretion systems, and membrane transport such as phosphotransferase systems involved in the import of nutrients, including sugars (**FIGS. 23** and **24**).

CAZyme profiles of glycoside hydrolases and glycosyltransferases were compared by calculating the R² value between each pair of microbiomes (see **Table 10** for families with a relative abundance >1%). This analysis revealed that all individuals have a similar profile of glycosyltransferases (mean R²=0.96±0.003), while the profiles of glycoside hydrolases were significantly more variable, even between family members (mean R²=0.80±0.01; p<10-30, paired Student's t-test). This suggests that the number and spectrum of glycoside hydrolases is probably affected by external factors such as diet more than the glycosyltransferases.

### Example 7: Obesity associated pathways

To identify metabolic pathways associated with obesity, only non-core associated (variable) functional groups were included in a comparison of the gut microbiomes of lean and obese twin pairs. A bootstrap analysis was used to identify metabolic pathways that were enriched or depleted in the variable obese gut microbiome. For example, similar to a mouse model of diet-induced obesity, the obese human gut microbiome was enriched for phosphotransferase systems involved in microbial processing of carbohydrates (**Table 12**). To identify specific genes that were significantly associated with obesity, all gut microbiome sequences were compared against the custom database of 42 gut genomes described in example 3. A gene-by-sample matrix was then screened to identify genes 'commonly-enriched' in either the obese or lean gut microbiome (defined by an odds ratio >2 or <0.5 when comparing all obese twin microbiomes to the aggregate lean twin microbiome or vice versa). The analysis yielded 383 genes that were significantly different between the obese and lean gut microbiome (q-value < 0.05; 273 enriched and 110 depleted in the obese microbiome; see **Tables 13** and **14**). By contrast, only 49 genes were consistently enriched or depleted between all twin-pairs.

These obesity-associated genes were representative of the taxonomic differences described above: 75% of the obesity-enriched genes were from Actinobacteria (vs. 0% of lean-enriched genes; the other 25% are from Firmicutes) while 42% of the lean-enriched genes were from Bacteroidetes (vs. 0% of the obesity-enriched genes). Their functional annotation indicated that many are involved in carbohydrate, lipid, and amino acid metabolism (**Tables 13-14**). Together, they comprise an initial set of microbial biomarkers of the obese gut microbiome.

| **Table 12: Pathways enriched or depleted in obese gut microbiomes^{a}** | |
|---|---|
| **Enriched** | Fatty acid biosynthesis |
| | Nicotinate and nicotinamide metabolism |
| | Other ion-coupled transporters |
| | Pentose and glucuronate interconversions |
| | Phosphotransferase system (PTS) |
| | Protein folding and associated processing |
| | Signal transduction mechanisms |
| | Transcription factors |
| **Depleted** | Bacterial chemotaxis |
| | Bacterial motility proteins |
| | Benzoate degradation via CoA ligation |
| | Butanoate metabolism |
| | Citrate cycle (TCA cycle) |
| | Glycosaminoglycan degradation |
| | Other enzymes |
| | Oxidative phosphorylation |
| | Pyruvate/Oxoglutarate oxidoreductases |
| | Starch and sucrose metabolism |
| | Tryptophan metabolism |

**Table 13: Bacterial genes enriched in the gut microbiomes of obese MZ twins**

| **SEQ. ID No:** | **Genome and NCBI proteinID** | **Annotation** | **COG** | **COG Categories** | **KEGG orthologous groups** |
|---|---|---|---|---|---|
| 1 | Bifidobacterium_adolescentis_154486403 | tRNA-ribosyltransferase | COG0343 | J | K00773 |
| 2 | Bifidobacterium_longum_23465114 | Transcriptional regulators | COG 1609 | K | |
| 3 | Bifidobacterium_longum_23466186 | ABC-type sugar transport system, periplasmic component | COG1653 | G | |
| 4 | Bifidobacterium_adolescentis_154488903 | Superfamily I DNA and RNA helicases | COG3973 | R | |
| 5 | Bifidobacterium_adolescentis_154486727 | DNA polymerase IV | COG0389 | L | K02346 |
| 6 | Bifidobacterium_adolescentis_154488882 | peptide/nickel transport system ATP-binding protein | COG1123 | R | K02031/2 |
| 7 | Bifidobacterium_adolescentis_154488633 | Trk-type K+ transport systems | COG0168 | P | |
| 8 | Bifidobacterium_adolescentis_154488131 | Asp-tRNAAsn/Glu-tRNAGln amidotransferase B subunit | COG0064 | J | K02434 |
| 9 | Bifidobacterium_adolescentis_154487571 | Threonine dehydratase | COG1171 | E | K01754 |
| 10 | Bifidobacterium_adolescentis_154486641 | Glucose-6-phosphate isomerase | COG0166 | G | K01810 |
| 11 | Bifidobacterium_adolescentis_154488790 | ATP-dependent helicase Lhr and Lhr-helicase | COG1201 | R | K03724 |
| 12 | Bifidobacterium_adolescentis_11 9025482 | Predicted ATPase involved in cell division | COG2884 | D | K09812 |
| 13 | Bifidobacterium_adolescentis_154486531 | Predicted phosphohydrolases | COG1409 | R | |
| 14 | Bifidobacterium_adolescentis_154486606 | tRNA-(guanine-N1)-methyltransferase | COG0336 | J | K00554 |
| 15 | Bifidobacterium_adolescentis_154486895 | IMP dehydrogenase/GMP reductase | COG0516/7 | FR | K00088 |
| 16 | Bifidobacterium_adolescentis_154486720 | Aspartate/tyrosine/aromatic aminotransferase | COG0436 | E | K00812 |
| 17 | Bifidobacterium_adolescentis_119026599 | Cation transport ATPase | COG0474 | P | K01529 |
| 18 | Bifidobacterium_adolescentis_154486334 | hypothetical protein | | | |
| 19 | Bifidobacterium_adolescentis_119025743 | NAD/NADP transhydrogenase alpha subunit | COG3288 | C | K00324 |
| 20 | Bifidobacterium_longum_23336617 | UspA and related nucleotide-binding proteins | COG0589 | T | |
| 21 | Bifidobacterium_adolescentis_154486937 | ABC-type suqar transport system | COG1653 | G | K02027 |
| 22 | Bifidobacterium_longum_23465912 | hypothetical protein | | | |
| 23 | Bifidobacterium_longum_23335963 | K+ transporter | COG3158 | P | K03549 |
| 24 | Bifidobacterium_adolescentis_119025729 | ABC-type transport system, Fe-S cluster assembly | COG0719 | O | |
| 25 | Bifidobacterium_adolescentis_154487396 | Glutamine synthetase adenylyltransferase | COG1391 | OT | K00982 |
| 26 | Bifidobacterium_adolescentis_154488156 | hypothetical protein | | | |
| 27 | Bifidobacterium_adolescentis_154486668 | Acetyl/propionyl-CoA carboxylase | COG4770 | I | K01946 |
| 28 | Nuclease Bifidobacterium_adolescentis_154487299 | subunit of the excinuclease complex | COG0322 | L | K03703 |
| 29 | Bifidobacterium_longum_23465540 | Acetate kinase | COG0282 | C | K00925 |
| 30 | Clostridium_bartlettii_164687465 | putative conjugative transposon protein | NOG13238 | | |
| 31 | Bifidobacterium_longum_23465037 | Dipeptidase | COG4690 | E | K08659 |
| 32 | Bifidobacterium_adolescentis_154488210 | Predicted hydrolase of the metallo-beta-lactamase superfamily | COG0595 | R | K07021 |
| 33 | Bifidobacterium_adolescentis_154487598 | tRNA/rRNA methyltransferase protein | | | K00599 |
| 34 | Bifidobacterium_adolescentis_119025149 | hypothetical protein | | | |
| 35 | Bifidobacterium_adolescentis_154487052 | hypothetical protein | NOG07592 | | |
| 36 | Bifidobacterium_adolescentis_154486554 | PTS system, enzyme I | | | K00935 |
| 37 | Bifidobacterium_longum_23335005 | Selenocysteine lyase | COG0520 | E | K01763 |
| 38 | Bifidobacterium_longum_23465294 | Branched-chain amino acid permeases | COG1114 | E | K03311 |
| 39 | Bifidobacterium_adolescentis_119025432 | Acyl-CoA thioesterase | COG 1946 | I | K01076 |
| 40 | Bifidobacterium adolescentis 154486528 | Aspartate-semialdehyde dehydrogenase | COG0136 | E | K00133 |
| 41 | Bifidobacterium_adolescentis_154487076 | Predicted ATPase with chaperone activity | COG0606 | O | K07391 |
| 42 | Bifidobacterium_longum_23466221 | Alcohol dehydrogenase, class IV | COG 1454 | C | K00048 |
| 43 | Bifidobacterium_adolescentis_119025541 | Phosphoribosylformylglycinamidine synthase | COG0046/7 | F | K01952 |
| 44 | Bifidobacterium_adolescentis_119026031 | Geranylgeranyl pyrophosphate synthase | COG0142 | H | |
| 45 | Bifidobacterium_longum_23465502 | Signal transduction histidine kinase | COG4585 | T | |
| 46 | Bifidobacterium_adolescentis_154486631 | metal-binding, possibly nucleic acid-binding protein | COG1399 | R | |
| 47 | Bifidobacterium_adolescentis_154488013 | Sugar (pentulose and hexulose) kinases | COG1070 | G | K00853 |
| 48 | Bifidobacterium_adolescentis_119025777 | Aspartate carbamoyltransferase | COG0540 | F | K00609 |
| 49 | Bifidobacterium_adolescentis_119025510 | Superfamily II DNA helicase | COG0514 | L | K03654 |
| 50 | Bifidobacterium_adolescentis_longum_119026360 | Protease II | COG1770 | E | K01354 |
| 51 | Bifidobacterium_adolescentis 119025672 | Signal transduction histidine kinase | COG3920 | T | |
| 52 | Bifidobacterium_adolescentis_154487392 | Orotidine-5'-phosphate decarboxylase | COG0284 | F | K01591 |
| 53 | Bifidobacterium_adolescentis_154487114 | of the major facilitator superfamily | COG0477 | GEPR | |
| 54 | Bifidobacterium_adolescentis_119025804 | Predicted Fe-S-cluster redox enzyme | COG0820 | R | K06941 |
| 55 | Bifidobacterium_longum_23465197 | of the major facilitator superfamily | COG0477 | GEPR | |
| 56 | Bifidobacterium_adolescentis_154487064 | Superfamily II RNA helicase | COG4581 | L | K01529 |
| 57 | Bifidobacterium_longum_23465727 | ABC-type dipeptide transport system | COG0747 | E | K02035 |
| 58 | Bifidobacterium_adolescentis_154486507 | hypothetical protein | | | |
| 59 | Bifidobacterium_longum_23465472 | Predicted transcriptional regulator | COG2865 | K | |
| 60 | Bifidobacterium_adolescentis_154486695 | ABC-type phosphate transport system | COG0226 | P | K02040 |
| 61 | Bifidobacterium_longum_23466332 | Dihydroxyacid dehydratase/phosphogluconate dehydratase | COG0129 | EG | K01687 |
| 62 | Bifidobacterium_adolescentis_54489143 | Predicted phosphatase/phosphohexomutase | COG0637 | R | |
| 63 | Bifidobacterium_adolescentis_154486988 | Phosphoribosylaminoim idazole carboxylase | COG0026 | F | K01589 |
| 64 | Bifidobacterium_adolescentis_154486732 | glycoside hydrolase family 77 | COG1640 | G | K00705 |
| 65 | Bifidobacterium_adolescentis_154487590 | Uncharacterized conserved protein | COG3247 | S | |
| 66 | Bifidobacterium_adolescentis_154486669 | Acetyl-CoA carboxylase I | COG4799 | I | K01966 |
| 67 | Bifidobacterium_adolescentis_154488016 | Homoserine kinase | COG0083 | E | K00872 |
| 68 | Bifidobacterium_adolescentis_119026221 | glycoside hydrolase family 43 | | | |
| 69 | Bifidobacterium_adolescentis_119025727 | CTP synthase (UTP-ammonia lyase) | COG0504 | F | K01937 |
| 70 | Bifidobacterium_adolescentis_154486325 | Uncharacterized protein conserved in bacteria | COG3583 | S | |
| 71 | Bifidobacterium_adolescentis_19025371 | Transcription elongation factor | COG0195 | K | K02600 |
| 72 | Bifidobacterium_adolescentis 154486867 | Sugar (pentulose and hexulose) kinases | COG1070 | G | K00854 |
| 73 | Bifidobacterium_adolescentis_154487511 | putative cell division protein | | | |
| 74 | Bifidobacterium_adolescentis_154487124 | hypothetical protein | | | |
| 75 | Bifidobacterium_adolescentis_119025212 | hypothetical protein | | | |
| 76 | Bifidobacterium_adolescentis_154487481 | hypothetical protein | | | |
| 77 | Bifidobacterium_adolescentis_154488824 | putative two-component sensor kinase | | | |
| 78 | Bifidobacterium_adolescentis_154488224 | serine_threonine protein kinase | | | |
| 79 | Bifidobacterium_adolescentis_154487149 | carbohydrate esterase family 1 | | | |
| 80 | Bifidobacterium_adolescentis_154488135 | rRNA methylases | COG0566 | J | K00599 |
| 81 | Bifidobacterium_adolescentis_154489172 | glycoside hydrolase family 77 | COG1640 | G | K00705 |
| 82 | Bifidobacterium_adolescentis_154487327 | Superfamily II RNA helicase | COG4581 | L | K03727 |
| 83 | Bifidobacterium_adolescentis_119025670 | Transcription elongation factor | COG0782 | K | K03624 |
| 84 | Bifidobacterium_adolescentis_154486326 | Dimethyladenosine transferase | COG0030 | J | K02528 |
| 85 | Bifidobacterium_longum_23465077 | qlycosyl-transferase family 51 | COG0744 | M | K03693 |
| 86 | Bifidobacterium_longum_23464647 | hypothetical protein | NOG25707 | | |
| 87 | Bifidobacterium_adolescentis_154486363 | hypothetical protein | | | |
| 88 | Bifidobacterium_adolescentis_154486438 | Permeases of the major facilitator superfamily | COG0477 | GEPR | |
| 89 | Bifidobacterium_longum_23335686 | antimicrobial peptide transport system | COG0577 | V | K02004 |
| 90 | Bifidobacterium_adolescentis_154486327 | 4-diphosphocytidyl-2C-methyl-D-erythritol 2-phosphate synthase | COG1947 | I | K00919 |
| 91 | Bifidobacterium_adolescentis_154488959 | twitching motility protein PilT | | | K02669 |
| 92 | Bifidobacterium_adolescentis_154486273 | Leucyl-tRNA synthetase | COG0495 | J | K01869 |
| 93 | Bifidobacterium_adolescentis_154486329 | tRNA nucleotidyltransferase/poly(A) polymerase | COG0617 | J | K00970 |
| 94 | Bifidobacterium_adolescentis_154487191 | putative phage protein | | | |
| 95 | Bifidobacterium_adolescentis_154486270 | DNA polymerase III, delta subunit | COG 1466 | L | K02340 |
| 96 | Bifidobacterium_adolescentis_154486380 | hypothetical protein | | | |
| 97 | Anaerostipes_caccae_167747544 | Non-ribosomal peptide synthetase modules and related proteins | COG1020 | Q | |
| 98 | Bifidobacterium_adolescentis_154486501 | Predicted unusual protein kinase | COG0661 | R | |
| 99 | Bifidobacterium_adolescentis_154486855 | Lacl-family transcriptional regulator | | | |
| 100 | Bifidobacterium_adolescentis_154486358 | Hemolysins and related proteins | COG1253 | R | K03699 |
| 101 | Bifidobacterium_adolescentis_154486649 | Acetylomithine deacetylase/Succinyl-diaminopimelate desuccinylase | COG0624 | E | K01439 |
| 102 | Bifidobacterium_adolescentis_119025555 | Orotidine-5'-phosphate decarboxylase | COG0284 | F | K01591 |
| 103 | Bifidobacterium_longum_23465600 | Gamma-glutamyl phosphate reductase | COG0014 | E | K00147 |
| 104 | Bifidobacterium_adolescentis_154486786 | FAD synthase/riboflavin kinase/FMN adenylyltransferase | COG0196 | H | K00861/0953 |
| 105 | Bifidobacterium_adolescentis_154488712 | Ribonuclease D | COG0349 | J | K03684 |
| 106 | Bifidobacterium_adolescentis_154488649 | N-acetylglutamate synthase (N-acetylornithine am inotransferase) | COG1364 | E | K00620/0642 |
| 107 | Bifidobacterium_adolescentis_154489082 | Ribonucleoside-triphosphate reductase | COG1328 | F | K00527 |
| 108 | Bifidobacterium_adolescentis_154487141 | transcriptional regulator, AraC family | | | |
| 109 | Bifidobacterium_longum_23335562 | Acetyltransferase (isoleucine patch superfamily) | COG0110 | R | K00680 |
| 110 | Bifidobacterium_adolescentis_119025600 | ABC-type amino acid transport system, permease component | COG0765 | E | |
| 111 | Bifidobacterium_adolescentis_154486349 | Recombinational DNA repair ATPase (RecF pathway) | COG1195 | L | K03629 |
| 112 | Bifidobacterium_adolescentis_154487341 | Succinvl-CoA synthetase | COG0045 | C | K01903 |
| 113 | Bifidobacterium_adolescentis_154486419 | Adenylosuccinate synthase | COG0104 | F | K01939 |
| 114 | Bifidobacterium_adolescentis_154486323 | transcriptional regulator, AraC family | | | |
| 115 | Bifidobacterium_adolescentis_119025197 | 3-isopropylmalate dehydratase large subunit | COG0065 | E | K01702/3 |
| 116 | Bifidobacterium_adolescentis_154489094 | Predicted dehydrogenases and related proteins | COG0673 | R | |
| 117 | Bifidobacterium_longum_23336262 | O-acetylhomoserine sulfhydrylase | COG2873 | E | K01740 |
| 118 | Bifidobacterium_longum_23465907 | ABC-type dipeptide/oligopeptide/nickel transport systems | COG0601 | EP | K02033 |
| 119 | Bifidobacterium_adolescentis_154487000 | Threonine aldolase | COG2008 | E | K01620 |
| 120 | Bifidobacterium_adolescentis_154487167 | Sortase and related acyltransferases | COG1247 | M | K03823 |
| 121 | Bifidobacterium_longum_23465198 | Thioredoxin reductase | COG0492/05 26 | OC | K00384 |
| 122 | Bifidobacterium_adolescentis_154488926 | Arabinose efflux permease | COG2814 | G | |
| 123 | Bifidobacterium_longum_23465931 | ABC-type antimicrobial peptide transport system, ATPase component | COG1136 | V | K02003/4 |
| 124 | Bifidobacterium_adolescentis_154486352 | Type IIA topoisomerase (DNA gyrase/topo II, topoisomerase IV) | COG0188 | L | K01863/2469 |
| 125 | Bifidobacterium_adolescentis_119026009 | Pyruvate-formate lyase-activating enzyme | COG1180 | O | K04069 |
| 126 | Bifidobacterium_adolescentis_154487279 | Methionine synthase II (cobalamin-independent) | COG0620 | E | K00549 |
| 127 | Bifidobacterium_adolescentis_119025238 | Acetolactate synthase | COG0440 | E | K01653 |
| 128 | Bifidobacterium_adolescentis_119025129 | Signal recoqnition particle GTPase | COG0552 | U | K03110 |
| 129 | Bifidobacterium_adolescentis_154488132 | Asp-tRNAAsn/Glu-tRNAGln amidotransferase | COG0154 | J | K02433 |
| 130 | Bifidobacterium_adolescentis_154486940 | ABC-type dipeptide transport system | COG0747 | E | K02035 |
| 131 | Bifidobacterium_adolescentis_154488789 | Type IIA topoisomerase (DNA gyrase/topo II, topoisomerase IV) | COG0188 | L | K01863/2469 |
| 132 | Bifidobacterium_adolescentis_154487377 | Long-chain acyl-CoA synthetases | COG1022 | I | K01897 |
| 133 | Bifidobacterium_adolescentis_154488794 | DNA-directed RNA polymerase, sigma subunit | COG0568 | K | K03086 |
| 134 | Bifidobacterium_adolescentis_154488989 | Superfamily I DNA and RNA helicases | COG0210 | L | K01529 |
| 135 | Bifidobacterium_adolescentis_154486903 | Prolyl-tRNA synthetase | COG0442 | J | K01881 |
| 136 | Bifidobacterium_adolescentis_154488684 | putative helicase | | | |
| 137 | Bifidobacterium_adolescentis_154486399 | Lysophospholipase | COG2267 | I | |
| 138 | Bifidobacterium_adolescentis_119026611 | ABC-type sugar transport systems, ATPase components | COG3839 | G | K05816 |
| 139 | Bifidobacterium_adolescentis_154486670 | Putative fatty acid synthase/reductase | COG0304/03 31/2030/4981 /4982 | IQ | K00059/209 /665/666/680 |
| 140 | Bifidobacterium_adolescentis 154488852 | ABC-type oligopeptide transport system | COG4166 | E | K02035 |
| 141 | Bifidobacterium_adolescentis_154486664 | putative ABC-type sugar transport system | | | |
| 142 | Bifidobacterium_adolescentis_119025257 | Ribonucleases G and E | COG1530 | J | K01128 |
| 143 | Bifidobacterium_adolescentis_154486472 | ABC-type antimicrobial peptide transport system | COG0577 | V | K02004 |
| 144 | Bifidobacterium_adolescentis_154487036 | hypothetical protein | | | |
| 145 | Bifidobacterium_adolescentis_154487636 | glycoside hydrolase family 2 | COG3250 | G | K01190 |
| 146 | Eubacterium_dolichum_160915695 | glycoside hydrolase family 31 | | | |
| 147 | Bifidobacterium_adolescentis_154489092 | Aspartate/tyrosine/aromatic aminotransferase | COG0436 | E | K00812 |
| 148 | Bifidobacterium_adolescentis_119026440 | hypothetical protein | NOG21350 | | |
| 149 | Bifidobacterium_adolescentis_119025397 | Myosin-crossreactive antigen | COG4716 | S | |
| 150 | Bifidobacterium_adolescentis_119026143 | Glutamine amidotransferase | COG0118 | E | K02501 |
| 151 | Bifidobacterium_adolescentis_154487050 | Universal stress protein UspA | COG0589 | T | |
| 152 | Bifidobacterium_adolescentis 154486729 | Phosphoglycerate dehydrogenase | COG0111 | HE | |
| 153 | Bifidobacterium_adolescentis_154488261 | Predicted hydrolases or acyltransferases | COG0596 | R | |
| 154 | Bifidobacterium_adolescentis_1544891 01 | hypothetical protein | | | |
| 155 | Bifidobacterium_adolescentis 154487476 | Phosphotransacetylase | COG0280/08 57 | CR | K00625 |
| 156 | Bifidobacterium_adolescentis_154488788 | Uncharacterized proteins of the AP superfam ily | COG1524 | R | |
| 157 | Ruminococcus_obeum_153809835 | putative ketose-bisphosphate aldolase | | | |
| 158 | Clostridium_leptum_160933115 | hypothetical protein | | | |
| 159 | Bifidobacterium_adolescentis_119026429 | Ribulose-5-phosphate 4-epimerase | COG0235 | G | K03080 |
| 160 | Bifidobacterium_adolescentis_154487579 | glycoside hydrolase family 36 | COG3345 | G | K07407 |
| 161 | Bifidobacterium_longum_23464678 | hypothetical protein | | | |
| 162 | Bifidobacterium_adolescentis_154486391 | Serine/threonine protein phosphatase | COG0631 | T | K01090 |
| 163 | Bifidobacterium_adolescentis_154486962 | ABC-type amino acid transport/signal transduction systems | COG0834 | ET | K02030 |
| 164 | Bifidobacterium_adolescentis_154486954 | DNA primase | COG0358 | L | K02316 |
| 165 | Bifidobacterium_adolescentis_154486993 | Glutamine phosphoribosylpyrophosphate amidotransferase | COG0034 | F | K00764 |
| 166 | Bifidobacterium_adolescentis_154488913 | HrpA-like helicases | COG1643 | L | K03578 |
| 167 | Bifidobacterium_adolescentis_154486787 | Predicted ATP-dependent serine protease | COG 1066 | O | K04485 |
| 168 | Bifidobacterium_adolescentis_154486493 | Ammonia permease | COG0004 | P | K03320 |
| 169 | Bifidobacterium_adolescentis_154487494 | Methenyl tetrahydrofolate cyclohydrolase | COG0190 | H | K00288/1491 |
| 170 | Bifidobacterlum_adolescentis_1119025196 | Transcriptional regulator | COG 1414 | K | |
| 171 | Dorea longicatena_153853202 | hypothetical protein | | | |
| 172 | Bifidobacterium_adolescentis_154487329 | putative transcriptional regulator | | | |
| 173 | Bifidobacterium_adolescentis_154487591 | Lacl-familv transcriptional regulator | | | |
| 174 | Bifidobacterium_adolescentis_154486321 | glycoside hydrolase family 3 | | | |
| 175 | Bifidobacterium_adolescentis_119025741 | GTPase | COG1159 | R | K03595 |
| 176 | Clostridium_scindens_167758922 | dUTPase | COG0756 | F | K01520 |
| 177 | Bifidobacterium_adolescentis_119025587 | Signal transduction histidine kinase | COG0642 | T | |
| 178 | Bifidobacterium_adolescentis_154486470 | Predicted membrane protein | COG4393 | S | |
| 179 | Clostridium_scindens_167760262 | putative sporulation protein | | | |
| 180 | Bacteroides_stercoris_167763769 | hypothetical protein | | | |
| 181 | Anaerostipes_caccae_167746872 | putative ABC transporter | | | |
| 182 | Bifidobacterium_adolescentis_154486920 | ABC-type amino acid transport/signal transduction systems | COG0834 | ET | K02030 |
| 183 | Bifidobacterium_adolescentis_154487063 | Uncharacterized conserved protein | COG2326 | S | |
| 184 | Bifidobacterium_adolescentis_119025989 | glycoside hydrolase family 13 | COG0366 | G | K01187 |
| 185 | Clostridium_bartlettii_164687864 | Lactoylglutathione lyase | COG0346 | E | K01759 |
| 186 | Bifidobacterium_adolescentis_154486443 | ABC-type antimicrobial peptide transport system | COG0577 | V | K02004 |
| 187 | Bifidobacterium_adolescentis_1544882451 | NADH:flavin oxidoreductases/NADPH2 dehydrogenase | COG1902 | C | K00354 |
| 188 | Bifidobacterium_longum_23465963 | atypical histidine kinase sensor of two-component system | NOG21560 | | |
| 189 | Bifidobacterium_adolescentis_1544889491 | hypothetical protein | | | |
| 190 | Bifidobacterium_adolescentis_154486865 | maltose O-acetyltransferase | | | |
| 191 | Clostridium_scindens_167759009 | cytidylate kinase | | | K00945 |
| 192 | Bifidobacterium_adolescentis_154486901 | ATP-dependent exoDNAse | COG0507 | L | |
| 193 | Ruminococcus_torques_153814251 | hypothetical protein | | | |
| 194 | Bifidobacterium_adolescentis_119025327 | Ribosomal protein L13 | COG0102 | J | K02871 |
| 195 | Bifidobacterium_adolescentis_154488916, | ABC-type antimicrobial peptide transport system | COG1136 | V | |
| 196 | Bifidobacterium_adolescentis_119025389 | putative histidine kinase sensor of two component system | | | |
| 197 | Ruminococcus_gnavus_154504598 | Translation elongation factor P (EF-P)/initiation factor 5A (elF-5A) | COG0231 | J | K02356 |
| 198 | Bifidobacterium_adolescentis_119026648 | ribonuclease P | NOG21633 | | K03536 |
| 199 | Clostridium_scindens_167760715 | hypothetical protein | | | |
| 200 | Bifidobacterium_adolescentis_119026098 | Uncharacterized conserved protein | COG2606 | S | |
| 201 | Clostridium_scindens_167761320 | ABC-type antimicrobial peptide transport system | COG1136 | V | K02003 |
| 202 | Bacteroides_stercoris_167762249 | hypothetical protein | | | |
| 203 | Anaerostipes_caccae_167746530 | putative ion channel | | | |
| 204 | Bifidobacterium_adolescentis_119025057 | Serine/threonine protein kinase | COG0515 | RTKL | |
| 205 | Clostridium_bartlettii_164686672 | Molybdopterin biosynthesis enzymes | COG0521 | H | K03638 |
| 206 | Ruminococcus_obeum_153811887 | hypothetical protein | | | |
| 207 | Clostridium_spiroforme_169349879 | protein-Np-phosphohistidine-sugar phosphotransferase | | | K00890 |
| 208 | Clostridium_ramosum_167756439 | type I restriction enzyme, S subunit | | | K01154 |
| 209 | Bifidobacterium_adolescentis_119025640 | alcohol dehydrogenase of unknown specificity | COG4221 | R | |
| 210 | Eubacterium_ventriosum_154483925 | Uncharacterized conserved protein | COG2501 | S | |
| 211 | Bifidobacterium_adolescentis_154487477 | Phosphoketolase | COG3957 | G | K01621/32/36 |
| 212 | Bifidobacterium_adolescentis_154489149 | Putative molecular chaperone | COG0443 | O | K01529/4043 /8070 |
| 213 | Bifidobacterium_adolescentis_119025585 | hypothetical protein | | | |
| 214 | Clostridium_scindens_167759334 | antimicrobial peptide transport system | COG1136 | V | K02003 |
| 215 | Anaerostipes_caccae_167748732 | Serine-pyruvate aminotransferase/archaeal aspartate aminotransferase | COG0075 | E | K03430 |
| 216 | Ruminococcus_gnavus_154505702 | Putative phage replication protein RstA | COG2946 | L | K07467 |
| 217 | Bifidobacterium_adolescentis_154486389 | Cell division protein FtsI | COG0768 | M | |
| 218 | Bifidobacterium_adolescentis_154488668 | ABC-type cobalt transport system | COG1122 | P | K02006 |
| 219 | Bifidobacterium_adolescentis_154486277 | Fructose-2,6-bisphosphatase/phosphoglycerate mutase | COG0406 | G | K01834 |
| 220 | Clostridium_scindens_167758556 | hypothetical protein | | | |
| 221 | Dorea_longicatena_153855715 | putative acetyltransferase | | | |
| 222 | Eubacterium_dolichum_160915136 | ABC-type antimicrobial peptide transport system | COG1136 | V | K02003 |
| 223 | Bifidobacterium_adolescentis_119026205 | Isoleucyl-tRNA synthetase | COG0060 | J | K01870 |
| 224 | Ruminococcus_obeum_153810514 | glycoside hydrolase family 23 | COG0741/91 | M | |
| 225 | Eubacterium_eligens_Contig2011.538 | putative phosphohydrolase | | | |
| 226 | Bifidobacterium_adolescentis_154487387 | Transcriptional regulator | COG0583 | K | |
| 227 | Ruminococcus_obeum_153812199 | putative flavodoxin | | | |
| 228 | Bifidobacterium_adolescentis_154486996 | Phosphoribosylformylglycinamidine synthase | COG0046/7 | F | K01952 |
| 229 | Dorea_longicatena_153854194 | Ornithine/acetylornithine aminotransferase | COG4992 | E | K00818 |
| 230 | Ruminococcus_gnavus_154505209 | Predicted GTPases | COG1160 | R | |
| 231 | Dorea_longicatena_153853531 | Predicted transcriptional regulators | COG 1695 | K | |
| 232 | Ruminococcus_torques_153814203 | Acetyltransferases | COG0456 | R | K03826 |
| 233 | Clostridium_scindens_167761371 | putative ABC-type transport system | | | |
| 234 | Bifidobacterium_longum_38906105 | F0F1-type ATP synthase | COG0055 | C | K02112 |
| 235 | Collinsella_aerofaciens_139439837 | hypothetical protein | | | |
| 236 | Clostridium_leptum_160933570 | ABC-type antimicrobial peptide transport system | COG0577/11 36 | V | K02003 |
| 237 | Eubacterium_rectale_2731 | putative sensor histidine kinase | | | |
| 238 | Bifidobacterium_adolescentis_154489126 | ABC-type multidrug transport system | COG 1132 | V | K06147 |
| 239 | Ruminococcus_obeum_153812105 | putative conjugative transposon protein | NOG05968 | | |
| 240 | Dorea_longicatena_153853999 | hypothetical protein | | | |
| 241 | Clostridium bolteae 160937390 | hypothetical protein | | | |
| 242 | Ruminococcus_torques_153814809 | cytidylate kinase | | | K00945 |
| 243 | Ruminococcus_obeum_153810530 | hypothetical protein | | | |
| 244 | Clostridium_scindens_167758273 | putative alanine racemase | | | |
| 245 | Clostridium_scindens_167760222 | putative ABC transporter | | | |
| 246 | Dorea_longicatena_153854759 | Sporulation protein | COG2088 | M | K06412 |
| 247 | Bifidobacterium_adolescentis_119025414 | glycosyl-transferase family 4 | | | |
| 248 | Ruminococcus_obeum_153813075 | hypothetical protein | | | |
| 249 | Eubacterium_ventriosum_154482695 | Queuine/archaeosine tRNA-ribosyltransferase | COG0343 | J | K00773 |
| 250 | Ruminococcus_obeum_153811892 | hypothetical protein | | | |
| 251 | Ruminococcus_obeum_153810246 | Type IV secretory pathway, VirB4 components | COG3451 | U | |
| 252 | Dorea_longicatena_153854838 | Ribosomal protein S16 | COG0228 | J | K02959 |
| 253 | Dorea_longicatena_153855241 | putative DNA gyrase, subunit A | | | |
| 254 | Collinsella_aerofaciens_139438412 | putative transcriptional regulator | | | |
| 255 | Clostridium_leptum_160934853 | putative ribosomal-protein-alanine acetyltransferase | | | |
| 256 | Eubacterium_rectale_3602 | Type IV secretory pathway, VirD4 components | COG3505 | U | |
| 257 | Bifidobacterium_adolescentis_154486460 | ABC-type multidrug transport system | COG1132 | V | K06147 |
| 258 | Anaerostipes_caccae_167746203 | exonuclease SbcC | | | K03546 |
| 259 | Ruminococcus_obeum_153813732 | hypothetical protein | | | |
| 260 | Eubacterium_ventriosum_154484729 | protein-Np-phosphohistidine-sugar phosphotransferase | | | K00890 |
| 261 | Eubacterium_rectale_3363 | putative ABC transporter | | | |
| 262 | Ruminococcus_obeum_153809913 | hypothetical protein | | | |
| 263 | Anaerostipes_caccae_167748861 | putative arylsulfate sulfotransferase | | | |
| 264 | Eubacterium_eligens_Contig2011.154 | Uncharacterized conserved protein | COG4283 | S | |
| 265 | Clostridium_scindens_167759418 | putative competence protein ComEA | | | |
| 266 | Eubacterium_rectale_3439 | putative RNA-directed DNA polymerase | | | |
| 267 | Clostridium_bolteae_160940954 | SAM-dependent methyltransferases | COG0500 | QR | K00599 |
| 268 | Ruminococcus_obeum_153811726 | putative DNA topoisomerase | | | |
| 269 | Ruminococcus_obeum_153813044 | putative transposase | | | |
| 270 | Eubacterium_rectale_2410 | type I restriction enzyme, R subunit | | | K01152/3 |
| 271 | Clostridium_bolteae_160941795 | putative recombination protein | | | |
| 272 | Bifidobacterium_adolescentis_154486724 | putative esterase | | | |
| 273 | Collinsella_aerofaciens_139438485 | putative amidohydrolase | | | |

**Table 14: Bacterial genes enriched in gut microbiomes of lean MZ twins**

| **SEQ. No.** | **Genome and NCBI proteinID** | **Annotation** | **COG** | **COG Categories** | **KEGG orthologous groups** |
|---|---|---|---|---|---|
| 274 | Bacteroides_capillosus_154500567 | putative amidohydrolase | | | |
| 275 | Clostridium_leptum_160934848 | putative acetyltransferase | | | |
| 276 | Ruminococcus_obeum_153810033 | phosphocarrier protein HPr | | | K02784 |
| 277 | Eubacterium_siraeum_167749283 | putative ABC transporter related protein | | | |
| 278 | Bacteroides_capillosus_154497054 | Polyribonucleotide nucleotidyltransferase | COG1185 | J | K00962 |
| 279 | Eubacterium_siraeum_167749675 | Isoleucyl-tRNA synthetase | COG0060 | J | K01870 |
| 280 | Eubacterium_rectale_3617 | hypothetical protein | | | |
| 281 | Bacteroides_capillosus_154498345 | putative sporulation protein | | | |
| 282 | Parabacteroides_merdae_154490921 | hypothetical protein | | | |
| 283 | Bacteroides_capillosus_154500960 | putative chromosome segregation protein | | | |
| 284 | Ruminococcus_torques_153814925 | putative sporulation protein | | | |
| 285 | Clostridium_scindens_167758815 | glycosyl-transferase family 4 | | | |
| 286 | Clostridium_sp._L2_50_160893842 | Protease subunit of ATP-dependent Clp proteases | COG0740 | OU | K01358 |
| 287 | B_theta_WH2_000545 | putative type I restriction enzyme EcoAl specificity protein | | | |
| 288 | Bacteroides_capillosus_154500843 | trk system potassium uptake protein TrkA | | | K03499 |
| 289 | Clostridium_bolteae_160936948 | putative two-component transcriptional regulator | | | |
| 290 | Bacteroides_capillosus_154498005 | ATP-dependent serine protease/cysteine S-methyltransferase | COG 1066 | O | K00567 |
| 291 | Parabacteroides_merdae_154492394 | hypothetical protein | | | |
| 292 | Bacteroides_capillosus_154498009 | Fructose/tagatose bisphosphate aldolase | COG0191 | G | K01622 |
| 293 | B_theta_3731_000845 | hypothetical protein | | | |
| 294 | Anaerotruncus_colihominis_167769594 | Predicted ATPase (AAA+ superfamily) | COG1373 | R | |
| 295 | Bacteroides_capillosus_154500228 | putative translation protein | | | |
| 296 | Anaerofustis_stercorihominis_169334667 | putative DNA recombinase | | | |
| 297 | B_theta_3731_003400 | hypothetical protein | | | |
| 298 | Parabacteroides_distasonis_150008749 | hypothetical protein | | | |
| 299 | Bacteroides_fragilis_19068109 | mobilization protein BmgA | NOG11714 | | |
| 300 | Eubacterium_dolichum_160914154 | glycoside hydrolase family 20 | COG3525 | G | K01207 |
| 301 | Bacteroides_capillosus_154497125 | RNA methyltransferase, TrmH family | | | K03218 |
| 302 | Clostridium_sp._L2_50_160894658 | NTP pyrophosphohydrolases | COG0494/33 23 | LRS | K03574 |
| 303 | Parabacteroides_merdae_154494925 | Glyceraldehyde-3-phosphate dehydrogenase | COG0057 | G | K00134 |
| 304 | Bacteroides_capillosus_154496139 | Type IIA topoisomerase (DNA gyrase/topo II, topoisomerase IV) | COG0188 | L | K01863/2469 |
| 305 | Clostridium_ramosum_167755346 | MoxR-like ATPase | | | K03924 |
| 306 | Bacteroides_uniformis_160888848 | hypothetical protein | | | |
| 307 | Ruminococcus_qnavus_154504651 | Putative translation initiation inhibitor | COG0251 | J | K07567 |
| 308 | Bacteroides_uniformis_160890270 | putative phage protein | | | |
| 309 | Bacteroides_capillosus_154500164 | putative DNA recombinase | | | |
| 310 | B_theta_WH2_000807 | sulfotransferase/FAD synthetase | COG0175 | EH | K00957 |
| 311 | Bacteroides_uniformis_160892052 | carbohydrate esterase family 4 and 12 | | | |
| 312 | Clostridium_sp._L2_50_160893671 | hypothetical protein | | | |
| 313 | Bacteroides_capillosus_154500952 | hypothetical protein | | | K09710 |
| 314 | Clostridium_scindens_167759293 | putative ribonucleoside-triphosphate reductase activating protein | | | |
| 315 | Bacteroides_capillosus_154498134 | Predicted GTPases | COG1160 | R | K03977 |
| 316 | Bacteroides_capillosus_154500412 | ribosomal protein | | | |
| 317 | Bacteroides_fragilis_60683403 | Imidazolonepropionase and related amidohydrolases | COG 1228 | Q | K01468 |
| 318 | Peptostreptococcus_micros_160946111 | hypothetical protein | NOG 15344 | | |
| 319 | B theta_7330_001524 | putative transposase | | | |
| 320 | Bacteroides_capillosus_154500229 | putative peptidase | | | |
| 321 | Bacteroides_vulgatus_150006208 | Integrase | COG0582 | L | |
| 322 | Bacteroides_capillosus_154501540 | hypothetical protein | | | |
| 323 | Bacteroides_stercoris_167762500 | Site-specific recombinase XerD | COG4974 | L | |
| 324 | Bacteroides_fragilis_60679880 | glycoside hydrolase family 38 | COG0383 | G | K01191 |
| 325 | Bacteroides_capillosus_154497979 | putative replication protein | | | |
| 326 | Bacteroides capillosus 154500160 | putative helioase | | | |
| 327 | Bacteroides_stercoris_167752230 | Retron-type reverse transcriptase | COG3344 | L | |
| 328 | B theta_WH2_003792 | hypothetical protein | NOG14996 | | |
| 329 | Bacteroides_capillosus_154497731 | hypothetical protein | | | |
| 330 | Parabacteroides_merdae_ 154494117 | UDP-N-acetyl-D-mannosaminuronate dehydrogenase | COG0677 | M | K02472 |
| 331 | Bacteroides_caccae_153807847 | 2-succinyl-6-hydroxy-2,4-cyclohexadiene-1-carboxylate synthase | COG1165 | H | K02551 |
| 332 | Anaerotruncus_colihominis_167771309 | N-acetylglutamate synthase (N-acetylornithine aminotransferase) | COG1364 | E | K00618 |
| 333 | B_theta_WH2_003808 | putative outer membrane protein | | | |
| 334 | Eubacterium_dolichum_160914195 | putative copper-translocating P-type ATPase | | | K01529 |
| 335 | Bacteroides_fragilis_53715551 | Predicted ATPase | COG1373 | R | |
| 336 | Clostridium_bolteae_160937654 | putative phage protein | | | |
| 337 | Bacteroides_fragilis_53712550 | Alkyl hydroperoxide reductase | COG3634 | O | K03387 |
| 338 | Parabacteroides_merdae_154492101 | hypothetical protein | | | |
| 339 | Clostridium_bolteae_160936352 | Uncharacterized conserved protein | COG2606 | S | |
| 340 | Bacteroides_uniformis_160889340 | TraM | | | |
| 341 | B_theta_7330_002089 | Adenine-specific DNA methylase | COG0827/46 46 | KL | |
| 342 | B_theta_WH2_003982 | putative outer membrane protein | | | |
| 343 | Bacteroides_capillosus_154496743 | hypothetical protein | | | |
| 344 | Clostridium_bolteae_160941240 | putative citrate lyase | | | |
| 345 | Bacteroides_capillosus_154496327 | putative v-type ATPase | | | |
| 346 | Bacteroides_capillosus_154496839 | putative cobalamin biosynthesis protein | | | |
| 347 | Bacteroides_fragilis_60683742 | Small-conductance mechanosensitive channel | COG0668 | M | |
| 348 | Eubacterium_siraeum_167749611 | putative transcriptional regulator | | | |
| 349 | Parabacteroides_distasonis_150007998 | Cobyric acid synthase | COG1492 | H | K02232 |
| 350 | Parabacteroides distasonis_150008480 | putative pyruvate formate-lyase 3 activating enzyme | | | |
| 351 | Bacteroides_capillosus_154496329 | Na+-transporting two-sector ATPase/ATP synthase | | | K01549/50 |
| 352 | Bacteroides_capillosus_154496850 | hypothetical protein | | | |
| 353 | Bacteroides_capillosus_154496749 | putative spore maturation protein | | | |
| 354 | Bacteroides_capillosus_154496148 | putative spore protease | | | |
| 355 | Clostridium_bolteae_160937655 | DNA polymerase | | | K00961 |
| 356 | Bacteroides fragilis 60683107 | Putative copper/silver efflux pump | COG3696 | P | K07239/7787 |
| 357 | Bacteroides_capillosus_154496295 | putative short-chain dehydrogenase/reductase | | | |
| 358 | Anaerotruncus_colihominis_167771023 | stage V sporulation protein AC | | | K06405 |
| 359 | B_theta_WH2_004992 | ABC-type multidrug transport system | COG0842 | V | K09686 |
| 360 | Bacteroides_capillosus_154500409 | Transcription antiterminator | COG0250 | K | K02601 |
| 361 | B_theta_3731_003445 | putative tyrosine type site-specific recombinase | NOG36763 | | |
| 362 | B_theta_WH2_003671 | putative 3-oxoacyl-[acyl-carrier-protein] synthase | | | |
| 363 | Parabacteroides_distasonis_150010457 | hypothetical protein | | | |
| 364 | Bacteroides_fragilis_60681723 | putative hydrolase lipoprotein | NOG09493 | | |
| 365 | Clostridium_scindens_167758928 | putative transcriptional regulator | | | |
| 366 | Bacteroides_capillosus_154498046 | Exonuclease VII small subunit | COG 1722 | L | K03602 |
| 367 | Ruminococcus_gnavus_154504691 | putative phage protein | | | |
| 368 | Anaerotruncus_colihominis_167772969 | hypothetical protein | | | |
| 369 | Bacteroides_caccae_153808785 | Predicted nucleoside-diphosphate sugar epimerases | COG1086 | MG | |
| 370 | Alistipes_putredinis_167751920 | phosphoglycolate phosphatase | | | K01091 |
| 371 | Anaerotruncus_colihominis_167772790 | hypothetical protein | | | |
| 372 | Parabacteroides_merdae_154494124 | putative transcriptional regulator | | | |
| 373 | Bacteroides_caccae_153809523 | glycoside hydrolase family 29 | COG3669 | G | K01206 |
| 374 | Bacteroides_fragilis_46242778 | TraO conjugation protein | | | |
| 375 | Bacteroides_capillosus_154499075 | putative site-specific recombinase | | | |
| 376 | Anaerotruncus_colihominis_163816273 | putative DNA helicase | | | |
| 377 | Bacteroides_capillosus_154495881 | Pentose-5-phosphate-3-epimerase | COG0036 | G | K01783 |
| 378 | Bacteroides_uniformis_160887913 | hypothetical protein | | | |
| 379 | Dorea_longicatena_153853397 | putative phage protein | | | |
| 380 | Bacteroides_vulgatus_150003721 | putative outer membrane protein | | | |
| 381 | B_theta_WH2_002145 | putative outer membrane protein | | | |
| 382 | Bacteroides_capillosus_154500525 | hypothetical protein | | | Lean- |
| 383 | Alistipes_putredinis_167752229 | putative DNA primase | NOG22337 | | |

### Example 8: BMI categorization by ethnicity in participants in Missouri Adolescent Female Twin Study

BMI category by ethnicity for the entire MOAFTS wave 5 cohort, based on 3326 twins with complete data on height and weight is summarized in **Table 15**. Dizygotic (DZ) twins had a significantly higher mean BMI than monozygotic (MZ) twins [25.8±6.5 vs. 24.8±5.9, p<0.001, mean±sd], and a higher prevalence of overweight (22.8 vs 20.9%) and obese (20.7 vs 16.1 %; X²= 31.6, p<0.001). This may reflect a higher dizygotic twinning rate among obese women (MZ twinning occurs randomly39). BMI was more highly correlated in MZ twins than in DZ twins, both in EA pairs (rMZ=0.80, rDZ=0.48) and in AA pairs (rMZ=0.73, rDZ=0.26), and this remained true when analysis was restricted to pairs concordant for obesity (EA: rMZ=0.61, rDZ=0.27; AA rMZ=0.62, rDZ=-0.11) or concordant for leanness (EA: rMZ=0.43, rDZ=0.14; AA: rMZ=0.55, rDZ=0.39). After age-adjustment, quantitative genetic modeling yielded an estimated additive genetic variance for BMI of 68% (95% Confidence Interval [Cl]: 57-79%), shared environmental variance of 14% (95% Cl: 2-24%), and non-shared environmental variance of 14% (95%Cl: 17-21%). Data from the Behavioral Risk Factor Surveillance System for Missouri women of comparable age in 2006 yield higher rates of overweight and obesity in EA women (23.8% overweight and 25% obese) compared to rates observed in MOAFTS (19.6% overweight EA, 14.8% obese EA).

| **Table 15: BMI category in the Missouri Adolescent Female Twin Study^{a}** | | | | | | |
|---|---|---|---|---|---|---|
| | **Underweight (n=138)** | **Lean (n=1893)** | **Overweight (n=711)** | **Obese I (n=309)** | **Obese II (n=174)** | **Obese III (n=113)** |
| EA (n=2860) | 4.79 | 60.87 | 19.58 | 8.08 | 4.27 | 2.41 |
| AA (n=478) | 0.21 | 31.80 | 31.59 | 16.32 | 10.88 | 9.21 |

| | | | | | | |
|---|---|---|---|---|---|---|
| ^{a}All numbers are percentages. Underwight: ,18.5 kg/m²; Lean 18.5-24.9kg/m² 25-29.9 kg/m²; Obese I: 30-34..9 kg/m²; Obese II: 35-39.9 kg/m²; Obese III: ≥ 40 kg/m² | | | | | | |

Lean and obese women selected for inclusion in the biospecimen collection project were representative of the entire cohort of lean and obese MOAFTS twins in terms of parity (nulliparous/parous), educational attainment (more than high school education/high school education or less) and marital status (married or living with someone as married/not married; p>0.05 for all comparisons). Obese EA women providing biospecimens had a mean BMI at wave 5 of 36.9±4.7 compared with a mean among EA lean women of 21.4±1.5 (mean±sd). EA twins were selected as being stably lean across all waves of data collection (i.e., baseline at median age 15, one-year follow-up, 5-year follow-up and 7-year follow-up), with a self-reported BMI of 18.5-24.9 kg/m².

### Example 9: Comparison of amplification methods in taxonomic assignments

A frequently reported result from any 16S rRNA gene sequence-based survey is the relative abundance of bacterial phyla. Given the broad nature of these phyla and the fact that a relatively few phyla dominate the human distal gut microbiota, it might be expected that the relative abundance of each phylum be consistent regardless of the amplification and sequencing methods used. However, differences were observed between methods in this study (**FIGS. 13A-E**). Relative to the sampled gut microbiomes (defined by pyrosequencing of total community DNA), the full-length, V2/3, and V6 16S rRNA gene datasets were all significantly depleted for Bacteroidetes (paired Student's t-test, p<0.001), and significantly enriched for Firmicutes (p<0.01). One possible explanation for these differences is that the Bacteroidetes reference genomes are more closely related to those in the microbiomes than the Firmicutes reference genomes, thereby inflating estimates of the relative abundance of this phylum (**FIG. 10**). To address this potential confounding factor, 16S rRNA gene fragments from all 18 microbiome datasets were identified and classified them taxonomically. The results of this analysis confirmed that the three PCR-based methods underestimate the relative abundance of the Bacteroidetes (**FIG. 13F**). Moreover, results obtained from shotgun sequencing 16S rRNA gene fragments and PCR amplification of the V2/3 region showed the strongest correlation (**FIG. 13G**).

## Claims

1. An array comprising a substrate, the substrate having disposed thereon
(a) at least each of the nucleic acid sequences listed in Table 13, or nucleic acid sequences capable of specifically hybridizing to each of the nucleic acid sequences listed in Table 13; or
(b) at least each of the nucleic acid sequences listed in Table 14, or nucleic acid sequences capable of specifically hybridizing to each of the nucleic acid sequences listed in Table 14.

2. The array of claim 1, wherein the nucleic acid sequences are located at a spatially defined address of the array.

3. The array of claim 2, wherein the array has no more than 500 spatially defined addresses.

4. The array of claim 2, wherein the array has at least 500 spatially defined addresses.

5. The array of any one of claims 1-4, wherein the array consists of SEQ ID NOs:1-273 disposed on a substrate.

6. The array of any one of claims 1 to 4, wherein the array consists of SEQ ID NOs:274-383 disposed on a substrate.

## Patentansprüche

1. Array, umfassend ein Substrat, auf welchem
(a) zumindest jede der in Tabelle 13 aufgeführten Nukleinsäuresequenzen, oder Nukleinsäuresequenzen, welche spezifisch an jede der in Tabelle 13 aufgeführten Nukleinsäuresequenzen hybridisieren können, oder
(b) zumindest jede der in Tabelle 14 aufgeführten Nukleinsäuresequenzen, oder Nukleinsäuresequenzen, welche spezifisch an jede der in Tabelle 14 aufgeführten Nukleinsäuresequenzen hybridisieren können,
angeordnet ist.

2. Array nach Anspruch 1, worin sich die Nukleinsäuresequenzen unter einer räumlich definierten Adresse auf dem Array befinden.

3. Array nach Anspruch 2, worin das Array nicht mehr als 500 räumlich definierte Adressen umfasst.

4. Array nach Anspruch 2, worin das Array mindestens 500 räumlich definierte Adressen umfasst.

5. Array nach einem der Ansprüche 1 bis 4, worin das Array aus auf einem Substrat angeordneten SEQ ID NOs: 1 - 273 besteht.

6. Array nach einem der Ansprüche 1 bis 4, worin das Array aus auf einem Substrat angeordneten SEQ ID NOs: 274 - 383 besteht.

## Revendications

1. Puce comprenant un substrat, sur lequel substrat, est disposée,
(a) au moins chacune des séquences d'acide nucléique énoncées dans le tableau 13, ou des séquences d'acide nucléique capables de s'hybrider spécifiquement à chacune des séquences d'acide nucléique énoncées dans le tableau 13 ; ou
(b) au moins chacune des séquences d'acide nucléique énoncées dans le tableau 14, ou des séquences d'acide nucléique capables de s'hybrider spécifiquement à chacune des séquences d'acide nucléique énoncées dans le tableau 14.

2. Puce selon la revendication 1, dans laquelle les séquences d'acide nucléique sont situées à une adresse définie spatialement de la puce.

3. Puce selon la revendication 2, dans laquelle la puce ne comporte pas plus de 500 adresses définies spatialement.

4. Puce selon la revendication 2, dans laquelle la puce comporte au moins 500 adresses définies spatialement.

5. Puce selon l'une quelconque des revendications 1 à 4, dans laquelle la puce est constituée des SÉQ ID NO : 1 à 273 disposées sur un substrat.

6. Puce selon l'une quelconque des revendications 1 à 4, dans laquelle la puce est constituée des SÉQ ID NO : 274 à 383 disposées sur un substrat.
